(19) Europäisches Patentamt
European Patent Office
Office européen des brevets

(11) EP 3 290 044 B1

(12) EUROPÄISCHE PATENTSCHRIFT

(45) Veröffentlichungstag und Bekanntmachung des Hinweises auf die Patenterteilung:
**23.09.2020 Patentblatt 2020/39**

(51) Int Cl.:
***A61K 36/489*** *(2006.01)* ***A61P 31/00*** *(2006.01)*
***A61P 31/04*** *(2006.01)*

(21) Anmeldenummer: **17166298.4**

(22) Anmeldetag: **12.04.2017**

(54) **ANTIBAKTERIELLE ZUSAMMENSETZUNG, UMFASSEND EINEN PFLANZENEXTRAKT, VERFAHREN ZUR GEWINNUNG DES EXTRAKTS, PHARMAZEUTISCHE ZUSAMMENSETZUNG UND DEREN VERWENDUNG**

ANTIBACTERIAL COMPOSITION, COMPRISING A PLANT EXTRACT, METHOD FOR OBTAINING THE EXTRACT, PHARMACEUTICAL COMPOSITION AND USE

COMPOSITION ANTI-BACTÉRIENNE COMPRENANT UN EXTRAIT DE PLANTE, PROCÉDÉ DE PRODUCTION DE L'EXTRAIT, COMPOSITION PHARMACEUTIQUE ET SON UTILISATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **30.08.2016 DE 102016116126**

(43) Veröffentlichungstag der Anmeldung:
**07.03.2018 Patentblatt 2018/10**

(73) Patentinhaber: **SjF Hanse Scientific UG**
**20095 Hamburg (DE)**

(72) Erfinder:
- **Smirnov, Juri**
  **22559 Hamburg (DE)**
- **Fenner, Thomas**
  **DE-20095 Hamburg (DE)**

(74) Vertreter: **RGTH**
**Patentanwälte PartGmbB**
**Neuer Wall 10**
**20354 Hamburg (DE)**

(56) Entgegenhaltungen:
**EP-A2- 2 295 031 DE-A1- 3 144 137**
**GB-A- 1 054 124**

- **DATABASE WPI Week 201406 Thomson Scientific, London, GB; AN 2013-S29978 XP002770416, & KR 2013 0109084 A (UNIV SUNCHON NAT IND ACAD COOP CORPS) 7. Oktober 2013 (2013-10-07)**
- **DATABASE WPI Week 201381 Thomson Scientific, London, GB; AN 2013-M78559 XP002770417, & KR 2013 0078052 A (UNIV SUNCHON NAT IND ACAD COOP CORPS) 10. Juli 2013 (2013-07-10)**
- **DATABASE WPI Week 201523 Thomson Scientific, London, GB; AN 2015-18351T XP002770418, & CN 104 288 286 A (TIBET TIBETAN MEDICINE & PHARMACOLOGY IS) 21. Januar 2015 (2015-01-21)**
- **DATABASE TCM [Online] SIPO; 25. Juni 2014 (2014-06-25), Chen Shilin et al.: "A processing method of Fructus Sophorae Japonicae (processed with Mel) used for treating blood in stool due to heat in the large intestine, hemorrhoid welling and hemorrhage, and headache due to liver-heat.", XP002773135, Database accession no. CN-201210556677-A & CN 103 877 147 A (INST MEDICINAL PLANT DEV CAMS; UNIV HONG KONG BAPTIST) 25. Juni 2014 (2014-06-25)**
- **KITE G C ET AL: "Flavonol tetraglycosides from fruits of Styphnolobium japonicum (Leguminosae) and the authentication of Fructus Sophorae and Flos Sophorae", PHYTOCHEMISTRY, PERGAMON PRESS, GB, Bd. 70, Nr. 6, 1. April 2009 (2009-04-01), Seiten 785-794, XP026145423, ISSN: 0031-9422, DOI: 10.1016/J.PHYTOCHEM.2009.04.003 [gefunden am 2009-05-15]**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 290 044 B1

- DATABASE WPI Week 198416 Thomson Scientific, London, GB; AN 1984-098314 XP002773134, & JP S59 44313 A (YAKULT HONSHA KK) 12. März 1984 (1984-03-12)
- H-F GAO ET AL: "Hydroxymethyl furfural in chinese herbal medicines: Its formation, presence, metabolism, bioactivities and implications", AFRICAN JOURNAL OF TRADITIONAL, COMPLEMENTARY AND ALTERNATIVE MEDICINES, Bd. 12, Nr. 2, 13. April 2015 (2015-04-13) , Seite 43, XP55401546, DOI: 10.4314/ajtcam.v12i2.9
- KIMURA M ET AL: "INTERACTION IN THE ANTI BACTERIAL ACTIVITY OF FLAVONOIDS FROM SOPHORA-JAPONICA TO PROPIONIBACTERIUM", YAKUGAKU ZASSHI, Bd. 104, Nr. 4, 1984, Seiten 340-346, XP009195323, ISSN: 0031-6903
- YANG WOO-YOUNG ET AL: "Streptococcus mutanssortase A inhibitory metabolites from the flowers ofSophora japonica", BIOORGANIC & MEDICINAL CHEMISTRY LETTERS, Bd. 25, Nr. 7, 27. Februar 2015 (2015-02-27), Seiten 1394-1397, XP029148599, ISSN: 0960-894X, DOI: 10.1016/J.BMCL.2015.02.051
- HE XIRUI ET AL: "Local and traditional uses, phytochemistry, and pharmacology ofSophora japonicaL.: A review", JOURNAL OF ETHNOPHARMACOLOGY, ELSEVIER IRELAND LTD, IE, Bd. 187, 13. April 2016 (2016-04-13), Seiten 160-182, XP029557741, ISSN: 0378-8741, DOI: 10.1016/J.JEP.2016.04.014

## Beschreibung

Die Erfindung betrifft eine antibakterielle Zusammensetzung, umfassend einen Pflanzenextrakt, ein Verfahren zur Gewinnung des Extrakts, eine pharmazeutische Zusammensetzung und deren Verwendung.

### Hintergrund des Standes der Technik

In den letzten Jahren sind multiresistente Erreger zu einem alarmierenden Problem geworden. Vor allem die sog. nosokomialen Infektionen sind auf dem Vormarsch. Unter nosokomialer Infektion wird eine Infektion verstanden, die aufgrund eines Aufenthalts oder einer Behandlung in einem Krankenhaus oder einer Pflegeeinrichtung erworben wurde.

Der hohe Antibiotikaverbrauch in der Behandlung von bakteriellen Infektionskrankheiten hat zu einer deutlichen Zunahme von multiresistenten Problemerregern, wie Methicillin-resistentem Staphylococcus aureus (MRSA), Vancomycin-resistenten Enterokokken (VRE), beta-Lactamase produzierenden Enterobakterien (ESBL) oder multiresistenten gramnegativen Erregern (MRGN) und weiteren geführt. Der vermehrte Einsatz von Antibiotika steht mit der Zunahme von multiresistenten Erregern in direkter Wechselbeziehung. Die multiresistenten Keime haben sich im Lauf der Zeit an die Antibiotika angepasst und reagieren nur noch schlecht oder gar nicht mehr auf die derzeit bekannten Präparate. Zunehmend verkompliziert sich demzufolge auch die antibiotische Therapie von Infektionen, wobei häufig mehr als ein Antibiotikum appliziert werden muss.

Laut der Weltgesundheitsorganisation WHO (World Health Organisation) infizieren sich jährlich etwa 400.000 Menschen mit resistenten Bakterienstämmen, und bis zu 25.000 sterben an den Folgen dieser Infektionen in Europa. Das entspricht einer Infektionsrate von 5% bis 12% in den Krankenhäusern der europäischen Union. Insbesondere sind auch viele der Todesfälle auf Methicillin-resistente Staphylococcus aureus (MRSA)-Stämme zurückzuführen. Der Grund, warum diese resistenten Erreger immer häufiger auftreten, wird zum Beispiel auf die zu häufige und unsachgemäße Verabreichung von Antibiotika in der Humanmedizin, der Landwirtschaft und Tierzucht zurückgeführt. Gleichzeitig ist aber die Entwicklung und Erforschung neuer antibakterieller Substanzen seit den 90er Jahren des letzten Jahrhunderts auch aus Kostengründen bei vermeintlich ausreichender Empfindlichkeit der Erreger immer weiter zurückgenommen worden.

Es besteht demnach ein Bedarf, weitere antibakteriell wirkende Mittel für unterschiedliche therapeutische Zwecke zu finden, deren Wirksamkeit zu erforschen und als applizierbare Medikamente bereitzustellen.

So sind pflanzliche antibakterielle Wirkstoffe von zunehmendem Interesse. Zahlreiche Wirkstoffe wurden in der Literatur beschrieben, von denen einige nachfolgend angegeben sind:
Beispielsweise offenbart das koreanische Patent KR 1020020008349 von KIM, Jong Deok eine funktionelle Zusammensetzung von natürlichen Produkten mit einem Anti-Aging-Effekt und einer inhibierenden Wirkung auf das Wachstum von schädlichen Bakterien. Unter einer großen Reihe von Pflanzen werden beispielsweise die Paeonia Japonica und die Sophora Japonica beschrieben.

In dem Artikel "The Mechanism of Antimicrobial Activity of Sophoraflavanone B against Methicillin-Resistant Staphylococcus aureus" von Joung Dae-Ki, Shin Dong-Won et al., Foodborne Pathogens and Disease, März 2014, 11(3): 234 ff. wird eine Reihe von pflanzlichen Extrakten mit antibakterieller Wirkung beschrieben. Insbesondere wird Sophoraflavanon B eingesetzt, das beispielsweise aus den Wurzeln von Desmodium caudatum isoliert wird und als die lichtabsorbierende Komponente in der Sophora Japonica bekannt ist.

Das Patent Nr. 9810003/14 Russische Föderation von Tschitjakow Aleksej Genadjiwitsch offenbart eine Salbe, die verschiedene pflanzliche Komponenten enthält; unter anderem wird von den zahlreichen möglichen Zusätzen auch die Sophora Japonica erwähnt.

In dem Artikel "Interaction in the antibacterial activity of flavonoids from Sophora japonica L. to Propioni bacterium" von Yakugaku Zasshi, PubMed 1984 Apr;104(4): 340ff wird die Sensitivität von Propioni-Bakterien gegenüber den Flavonoiden der Sophora Japonica erwähnt. Derartige Bakterien sind in medizinischer Hinsicht jedoch nur von geringer Bedeutung.

Weiterhin beschreibt die KR 2013 0109084 A antibakterielle pharmazeutische Zusammensetzungen, enthaltend Sophora japonica-Extrakt. Beispielsweise wird ein Ethanol-Extrakt von Sophora japonica offenbart, wobei zusätzlich eine Bestrahlung mit Licht einer Stärke von 2000 bis 18000 Lux für 1 bis 24 Stunden bevorzugt durchgeführt wird.

Dies KR 2013 0078052 A bezieht sich auf antibakterielle Zusammensetzungen, umfassend Extrakte, beispielsweise von Sophora japonica. Als Extraktionsmittel werden u.a. Ethanol und Ethylacetat erwähnt.

Die CN 104 288 286 A betrifft eine flüssige Zusammensetzung mit antibakterieller Wirkung, enthaltend Sophora japonica-Extrakt.

Die CN 103 877 147 A beschreibt ein Verarbeitungsverfahren für die Früchte von Sophora Japonica mit Honigwein, wobei der Gehalt an Sophoricosid und Rutin erhöht wird. Die antibakteriellen und antioxidativen Eigenschaften werden erwähnt.

Kite G. C. et al.: "Flavonol tetragycosides from fruits of Styphnolobium japonicum (Leguminosae) and the authentication of Fructus Sophorae and Flos Sophorae", Phytochemistry, Pergamon Press, GB, Bd. 70, Nr. 6, 1. April 2009, S. 785-794 beschäftigt sich mit den Flavonoiden der Sophora Japonica, die im Einzelnen isoliert und charakterisiert werden, da diese als wahrscheinliche Quelle pharmakologischer Aktivität gelten.

Die JP S59 44313 A bezieht sich auf eine antimikrobielle Zusammensetzung, die Quercetin, Rutin und/oder Isorhamnetin-3-O-rutinosid enthält, und vorwiegend gegen Dermatopathien, verursacht durch Propionibakterien (P. acne, P. avidum), eingesetzt wird.

Die DE 31 44 137 A1 beschreibt ein Flächendesinfektionsmittel, insbesondere auf Basis von Dialdehyden, wobei durch Zusatz einer stark bakteriostatischen Verbindung, wie einem PHB-Ester, die Wirksamkeit gegen spezielle Keime erhöht wird. Die explizite Verwendung von Sophora japonica wird nicht beschrieben.

Die EP 2 295 031 A2 offenbart die Verwendung von Pterocarpanen als Anti-Cellulite-Wirkstoffe, wobei auch die antibakterielle Wirkung von Maackiain und Medicarpin beschrieben wird (siehe Abschnitt [0025]).

H.-f. Gao et al.: "Hydroxymethyl furfural in Chinese herbal medicines: Its formation, presence, metabolism, bioactivities and implications", African Journal of Traditional, complementary and alternative Medicines; Bd. 12, Nr. 2, 13. April 2015, S. 43 beschreibt Hydroxymethylfurfural (HMF) in der chinesischen Kräutermedizin mit u.a. antimikrobiellen Eigenschaften.

Kimura M. et al.: "Interaction in the Antibacterial Activity of Flavonoids from Sophora-Japonica to Propionibacterium", Yakugaku Zasshi, Bd. 104, Nr. 4, 1984, S. 340-346 offenbart Flavonoide der Sophora japonica mit antibakterieller Wirksamkeit im schwach sauren Milieu gegen Propionibakterien, insbesondere Rutin, Quercetin, Isorhamnetin-3-rutinosid und Kämpferol-3-rutinosid.

Yang Woo-Young et al.: "Streptococcus mutanssortase A inhibitory metabolites from the flowers of Sophora japonica", Bioorganic & Medicinal Chemistry Letters, Bd. 25, Nr. 7, S. 1394-1397 offenbart die Isolierung von Maltolderivaten sowie Flavanolglykosiden aus Sophora japonica sowie deren Verwendung bei humaner Dentalkaries.

Die GB 1 054 124 A beschreibt eine pharmazeutische Zusammensetzung zur Behandlung von Hauterkrankungen, die u.a. Neomycin und antiinflammatorische Glucocorticoide oder Flavonoide aufweist.

He Xirui et al: "Local and traditional uses, phytochemistry, and pharmacology of Sophora japonica L.: A review", Journal or Ethnopharmacology, Elsevier Ireland Ltd., IE, Bd. 187, 13. April 2016, S. 160-182 beschreibt den Extrakt von Sophora japonica sowie 153 chemische Verbindungen, die aus Teilen der Pflanze gewonnen wurden. Einzelne aus der Sophora japonica isolierte Verbindungen sind in Tabelle 2 auf S. 175ff. aufgelistet. Die antibakterielle Wirksamkeit eines Ethanol-Extrakts von Blumenknospen der Saphora japonica wird in Kapitel 5.2. auf S. 165 ff. gegen Staphylococcus aureus, Propionibacterium avidum und Propionibacterium acnes in schwach sauren Bedingungen beschrieben. Ferner wird zur Gewinnung von Rutin aus Flos Sophorae Immaturus im Brückenabsatz auf S. 163/164 erwähnt, dass eine Alkalilösung und Säureabscheidung durchgeführt wird, wobei auf Tabelle 4, S. 180, verwiesen wird, wo alkalische Extraktionen und Fällungen mit Natriumborat angegeben sind. Es wird demnach keine Säure-Aktivierung des Extrakts oder von dessen Inhaltsstoffen wie in der vorliegenden Erfindung beschrieben.

Die bisher bekannten Untersuchungen und Studien untersuchen eine große Reihe an Pflanzen und führen breite Screening-Studien durch, um möglichst alle für die Verwendung geeigneten Pflanzen zu erfassen. Eingehende Untersuchungen einer einzelnen Pflanze, wie beispielsweise die tiefergehende Untersuchung der chemischen Zusammensetzung und Charakterisierung, werden dort nicht durchgeführt. Ebenso fehlen die Angaben zur unmittelbaren Wirksamkeit der Extrakte auf einzelne bakterielle Erreger. Die Literatur erschöpft sich in Übersichtsdarstellungen, die wenige Informationen über einzelne Pflanzen enthalten. Es fehlen weiterführende detailliertere Untersuchungen hierzu.

Der vorliegenden Erfindung liegt die Aufgabe zugrunde, ein antibakteriell wirkendes Mittel auf pflanzlicher Basis für unterschiedliche therapeutische Zwecke bereitzustellen, das über ein geeignetes Wirkungsspektrum verfügt, um Bakterien zu hemmen oder abzutöten, und insbesondere auch bei einzelnen resistenten Krankheitserregern eine bakterizide Wirkung entfalten kann. Weiterhin soll das Mittel in einfacher Weise gewonnen und anwendbar sein.

## Detaillierte Beschreibung der Erfindung

Die vorliegende Erfindung bezieht sich auf eine antibakterielle Zusammensetzung, umfassend oder bestehend aus einem Extrakt aus den Früchten von Sophora japonica, derchemisch modifiziert wurde und eine bakterizide Wirkung hat.

Insbesondere bezieht sich die vorliegende Erfindung auf eine antibakterielle Zusammensetzung, umfassend oder bestehend aus einem Extrakt aus den Früchten von Sophora Japonica,
wobei der Extrakt erhältlich ist aus
einem ersten Extraktionsverfahren der Fruchtstücke von Sophora Japonica, wobei als Extraktionsmittel ein Gemisch aus Alkohol und Wasser, bevorzugt Ethanol und Wasser, eingesetzt wird, und
einem zweiten Extraktionsverfahren, das mit Ethylacetat als Extraktionsmittel durchgeführt wird,
wobei der nach der ersten Extraktion gewonnene Extrakt isoliert und dann die zweite Extraktion durchgeführt wird, oder

sich nach der ersten Extraktion, ohne Isolieren des Extrakts, die zweite Extraktion anschließt,
und der erhaltene Extrakt einer chemischen Modifikation in Form eines Aktivierungsverfahrens unterzogen wurden, erhältlich durch
Zugabe von verdünnter oder konzentrierter Salzsäure,
Erwärmen auf eine Temperatur zwischen 40 und 60°C,
anschließendes Zugeben von Wasser,
erneutes und fortgesetztes Erwärmen des erhaltenen Gemisches und
Einstellen eines neutralen pH-Werts
unter Erhalt eines aktivierten Extrakts.

Sophora japonica oder Styphnolobium japonicum, auch als japanischer Schnurbaum bezeichnet und aufgrund seiner säuerlich schmeckenden Samen auch unter der Bezeichnung Sauerschotenbaum bekannt, ist eine Pflanzenart in der Unterfamilie der Schmetterlingsblütler (Faboideae). Sophora japonica ist ein mittelgroßer, sommergrüner Baum, der eine Höhe bis zu 30 Metern erreichen kann. Die Blütezeit reicht von August bis September. Die Blüten stehen in endständigen langen Rispen und sind cremeweiß. Die Pflanze trägt von August bis Oktober Hülsenfrüchte, die jeweils ein bis sechs Kerne oder Samen enthalten. Die Kerne sind gelblich-grün und werden getrocknet schwarz-braun. Alle Pflanzenteile, insbesondere die Fruchtschale werden als giftig beschrieben.

Das natürliche Verbreitungsgebiet von Sophora japonica ist eigentlich Asien, in Europa werden jedoch vermehrt Anpflanzungen aufgrund der dekorativen Blüten und als Bienennährpflanze in Parks und in Alleen vorgenommen.

Die Blüten finden Anwendung in der chinesischen Küche, beispielsweise mit Eiern und Mehl als Omelette zubereitet. Die frischen und getrockneten Blüten werden in der traditionellen chinesischen Medizin verwendet und werden auch für Tee und in verschiedenen Gerichten eingesetzt. Die Blüten sollen blutdrucksenkende und entzündungshemmende Wirkung haben (https://de.wikipedia.org/wiki/ Japanischer_Schnurbaum - cite_note-PracticalPlantsDb-8).

Im Rahmen der vorliegenden Erfindung wird nunmehr eine neue antibakteriell wirksame Zusammensetzung, basierend auf dem Extrakt aus den Früchten der Sophora japonica bereitgestellt. Der Extrakt zeigt eine hohe Wirksamkeit und Effizienz gegen grampositive und gramnegative Erreger und insbesondere auch gegen multiresistente Krankheitserreger. Die Krankheitserreger sind beispielsweise klassifizierte (ATCC) Bakterienstämme, wie Staphylococcus aureus, Staphylococcus epidermidis, Streptococcus pyogenes, Enterococcus faecalis, Klebsiella pneumoniae, Enterobacter spp., Pseudomonas aeruginosa oder Escherichia coli, oder multiresistente Bakterienstämme, wie MRSA, Vancomycinresistente Enterococcus faecalis (VRE).

Eine besonders gute Wirksamkeit des Extrakts wird bei grampositiven Krankheitserregern beobachtet, besonders aber auch bei MRSA. Die durchgeführten Testungen sind im experimentellen Abschnitt im Einzelnen dargestellt.

Der Begriff "Extrakt" umfasst gemäß der vorliegenden Erfindung die gewonnene Mischung von Pflanzeninhaltsstoffen aus der Sophora japonica. Der Extrakt umfasst viele verschiedene biologisch aktive Substanzen, von denen einige identifiziert und charakterisiert wurden.

Der Extrakt wird zusätzlich einem Aktivierungsverfahren unterzogen, wodurch eine aktivierte Form des Extrakts erhalten wird, die in Wasser löslich ist und eine noch bessere antibakterielle Wirksamkeit bereitstellt. Der nicht in Wasser lösliche Extrakt wird durch das Aktivierungsverfahren daher in eine lösliche Form überführt, um seine antibakterielle Wirksamkeit besser entfalten zu können. Auch dieser behandelte oder aktivierte Extrakt wird hier einfach als "Extrakt" bezeichnet. Bei dieser Aktivierung durch das hier beschriebene neu entwickelte Verfahren finden mehrere Prozesse statt, die noch detailliert beschrieben werden und zu einer deutlichen Steigerung der antibakteriellen Wirksamkeit führen.

Aus der antibakteriellen Zusammensetzung, umfassend oder bestehend aus dem Extrakt, kann eine pharmazeutische Zusammensetzung hergestellt werden, die in einer Vielzahl von Darreichungsformen zur therapeutischen Behandlung dienen kann.

Die Erfindung betrifft daher auch eine pharmazeutische Zusammensetzung, umfassend die antibakterielle Zusammensetzung mit einem oder mehreren pharmazeutisch akzeptablen Träger(n) und/oder Hilfsstoffen. Die gewonnene Zusammensetzung kann ebenfalls in konzentrierter Form als Desinfektionsmittel eingesetzt werden.

Gegenstand der vorliegenden Erfindung ist auch die Verwendung der pharmazeutischen Zusammensetzung im therapeutischen Bereich, bevorzugt zur topischen und/oder systemischen Behandlung, insbesondere in der Behandlung von Haut- oder Schleimhauterkrankungen, bevorzugt Infektionen oder Entzündungen der Haut oder Schleimhaut, oder in der Behandlung oder Vorbeugung von Verletzungen der Haut oder Schleimhaut, insbesondere bei (Brand-)Wunden.

Die Erfindung bezieht sich auch auf die Verwendung der antibakteriellen Zusammensetzung oder der pharmazeutischen Zusammensetzung als Desinfektionsmittel, insbesondere gegen beispielsweise Staphylococcus aureus, Staphylococcus epidermidis, Streptococcus pyogenes, Enterococcus faecalis, Klebsiella pneumoniae, Enterobacter spp., Pseudomonas aeruginosa oder Escherichia coli, oder multiresistente Bakterienstämme, wie MRSA, Vancomycinresistente Enterococcus faecalis (VRE).

Nachfolgend soll zunächst die Gewinnung des Extrakts aus Sophora japonica beschrieben werden:

**Verfahren zur Gewinnung des Extrakts:**

Die Erfindung betrifft ein Verfahren zur Gewinnung des beschriebenen Extrakts, das einer chemischen Modifikation in Form eines Aktivierungsverfahrens unterzogen wurde, umfassend die folgenden Schritte:

- Bereitstellen von trockenen Früchten der Sophora japonica;
- Zerkleinern der getrockneten Früchte zu Fruchtstücken;
- Extrahieren der erhaltenen Fruchtstücke mit einer fest-flüssig Extraktion, bevorzugt in einem Soxhlet-Extraktor bei einer Temperatur oberhalb von 80°C, wobei ein Gemisch aus Alkohol und Wasser, bevorzugt Ethanol und Wasser, als Extraktionsmittel verwendet wird;
- gegebenenfalls mehrfaches Durchführen der Extraktion, um die Ausbeute zu erhöhen; und
- Verdampfen des Extraktionsmittels unter Erhalt des Extrakts;
- Bereitstellen einer wässerigen Lösung/Dispersion des erhaltenen Extrakts;
- Durchführen einer flüssig-flüssig Extraktion mit der wässerigen Lösung/Dispersion des Extrakts, wobei Ethylacetat als Extraktionsmittel verwendet wird;
- gegebenenfalls mehrfaches Durchführen der Extraktion, um die Ausbeute zu erhöhen; und
- Verdampfen des Extraktionsmittels unter Erhalt eines konzentrierten Extrakts;
- Durchführen eines Aktivierungsverfahrens des konzentrierten Extrakts mit den folgenden Schritten:
- Zugeben von konzentrierter oder verdünnter Salzsäure (HCl) zum Extrakt;
- Erwärmen des erhaltenen Gemisches unter Rühren;
- Zugeben von Wasser zu dem Gemisch;
- erneutes oder fortgesetztes Erwärmen des erhaltenen Gemisches;
- optional Zugeben eines Salzes oder hydrogenen Salzes aus einer schwachen Säure und einem Alkali- oder Erdalkalimetall zu dem Gemisch und erneutes oder fortgesetztes Erwärmen des erhaltenen Gemisches; und
- Einstellen eines neutralen pH-Wertes unter Erhalt eines aktivierten Extrakts.

Die Erfindung bezieht sich demnach auf ein Verfahren zur Gewinnung des Extrakts, das einer chemischen Modifikation in Form eines Aktivierungsverfahrens unterzogen wurde, umfassend die folgenden Schritte:

- Bereitstellen von trockenen Früchten der Sophora japonica;
- Zerkleinern der getrockneten Früchte zu Fruchtstücken;
- Extrahieren der erhaltenen Fruchtstücke mit einer fest-flüssig Extraktion, bevorzugt in einem Soxhlet-Extraktor bei einer Temperatur oberhalb von 80°C, wobei ein Gemisch aus Alkohol und Wasser, bevorzugt Ethanol und Wasser, als Extraktionsmittel verwendet wird;
- gegebenenfalls mehrfaches Durchführen der Extraktion, um die Ausbeute zu erhöhen;
- Verdampfen des Extraktionsmittels unter Erhalt des Extrakts.

An das obige Verfahren schließen sich die nachfolgenden Schritte an:

- Bereitstellen einer wässerigen Lösung/Dispersion des erhaltenen Extrakts;
- Durchführen einer flüssig-flüssig Extraktion mit der wässerigen Lösung/Dispersion des Extrakts, wobei Ethylacetat als Extraktionsmittel verwendet wird;
- gegebenenfalls mehrfaches Durchführen der Extraktion, um die Ausbeute zu erhöhen; und
- Verdampfen des Extraktionsmittels unter Erhalt eines konzentrierten Extrakts.

Bevorzugt schließt sich dieses Verfahren zur Wirksamkeitssteigerung direkt an obiges Verfahren zur Gewinnung des Extrakts ohne weitere Zwischenschritte direkt an.

Der erhaltene konzentrierte Extrakt wird dann in einem weiteren Verfahren zur chemischen Aktivierung und Verbesserung der Löslichkeitseigenschaften des gewonnenen Extrakts behandelt. Dieses zusätzliche Verfahren wird hier als "Aktivierungsverfahren" bezeichnet. Das erfindungsgemäße Verfahren weist daher ein zusätzliches Aktivierungsverfahren auf, das sich das obige Verfahren anschließt und die folgenden Schritte umfasst:

- Zugeben von konzentrierter oder verdünnter Salzsäure (HCl) zum erhaltenen konzentrierten Extrakt;
- Erwärmen des erhaltenen Gemisches unter Rühren;
- Zugeben von Wasser zu dem Gemisch;
- erneutes oder fortgesetztes Erwärmen des erhaltenen Gemisches;
- optional Zugeben eines Salzes oder hydrogenen Salzes aus einer schwachen Säure und einem Alkali- oder Erdalkalimetall zu dem Gemisch und erneutes oder fortgesetztes Erwärmen des erhaltenen Gemisches; und

- Einstellen eines neutralen pH-Wertes unter Erhalt eines aktivierten Extrakts.

Nachfolgend sollen die jeweiligen Verfahrensschritte für die Gewinnung des Extrakts beispielhaft im Einzelnen erläutert werden, ohne die Erfindung hierauf zu beschränken:

Gewinnung des unbehandelten Extrakts:

Zunächst werden die Früchte der Sophora japonica (Styphnolobium japonicum) bereitgestellt, beispielsweise durch Ernten und anschließendes Trocknen. Die Früchte können auch käuflich erworben werden. Dann werden vorteilhafterweise die Kerne aus der Frucht verarbeitet, jedoch ist es auch sinnvoll, die Früchte zu nehmen, da hierdurch zusätzliche Schritte zur Entfernung und Sammlung der Kerne aus den Früchten entfallen können.

Vorzugsweise findet die Ernte statt, wenn die Früchte ausreichend reif sind, beispielsweise im September/Oktober. Das Trocknen kann in beliebiger Weise erfolgen, beispielsweise durch Liegenlassen an Luft für mehrere Tage, wie etwa 10 bis 14 Tage.

Die bereitgestellten getrockneten Früchte werden zerkleinert, beispielsweise in Stücke, die einige mm groß sind. Bevorzugt sind Größen von etwa 2 bis 3 mm. Diese Größe hat sich als besonders vorteilhaft für das Verfahren ergeben.

Dann werden die Fruchtstücke in einem (ersten) Extraktionsverfahren extrahiert. Das Extraktionsverfahren kann beispielsweise mithilfe eines Soxhlet-Extraktors durchgeführt werden. Dazu wird zunächst der Extraktionsaufsatz des Extraktors mit den zerkleinerten Früchten befüllt. Als Extraktionsmittel wird ein Gemisch aus Alkohol und Wasser, bevorzugt Ethanol und Wasser, eingesetzt. Vorzugsweise wird ein Gemisch aus reinem Ethanol (z.B. 96%) und destilliertem Wasser verwendet. Das Extraktionsmittel wird gemäß dieser bevorzugten Ausführungsform in den Lösungsmittelkolben des Soxhlet-Extraktors gefüllt.

Besonders gute Ergebnisse werden erzielt, wenn die Menge an Ethanol im Extraktionsmittel etwa 30 bis 50%, besonders bevorzugt 35 bis 45%, insbesondere 40%, des Volumens der Fruchtstücke im Extraktionsaufsatz beträgt. Die Heizbadtemperatur wird so eingestellt, dass das Extraktionsgemisch zu sieden beginnt. Vorzugsweise werden mehrere Durchläufe oder Zyklen durchgeführt, beispielsweise können drei oder mehr Zyklen erfolgen. Beispielsweise können vier Zyklen ausreichen, um die maximale Ausbeute des Extrakts zu erreichen. Diese variiert von Charge zu Charge und liegt beispielsweise zwischen 25% und 40%, bezogen auf die Masse der extrahierten getrockneten Früchte.

Nach den durchgeführten Extraktionszyklen (erste oder fest-flüssig Extraktion) kann das Lösungsmittel abgedampft und ein Gemisch aus einem kristallinen Feststoff und einer öligen Masse erhalten werden. Dieses Gemisch wird im Folgenden als "Extrakt" oder "unbehandelter Extrakt" bezeichnet.

Gewinnung des konzentrierten Extrakts:

Gemäß der vorliegenden Erfindung werden die bakteriziden Substanzen durch einen weiteren Extraktionsschritt mit wesentlich höherer Reinheit gewonnen. Dazu wird eine zweite Extraktion (flüssig-flüssig Extraktion) mit Ethylacetat als Extraktionsmittel durchgeführt. Zu diesem Zweck kann der nach der ersten Extraktion gewonnene Extrakt isoliert und dann die zweite Extraktion durchgeführt werden, oder unmittelbar nach der ersten Extraktion, ohne Isolieren des Extrakts, kann sich die zweite Extraktion anschließen.

Wenn der Extrakt nicht isoliert wird, wird aus dem noch vorliegenden Extraktionsmittelgemisch aus Wasser und Alkohol, bevorzugt Wasser und Ethanol, zunächst der Alkohol abgedampft, so dass eine wässerige Lösung/Dispersion erhalten wird. Der Begriff "wässerige Lösung/Dispersion" bedeutet, dass je nach Menge an verwendetem Wasser und Konzentration des Extrakts, entweder eine Lösung oder Dispersion vorliegt.

Es ist bevorzugt, den Wasseranteil dieser Lösung/Dispersion beispielsweise um 50% zu reduzieren. Beispielsweise können so Konzentrationen von 0,3g/ml bis 0,5 g/ml in wässeriger Lösung des aus der ersten Extraktion erhaltenen Extrakts eingestellt werden.

Diese wässerige Lösung/Dispersion wird dann einer zweiten (flüssig-flüssig) Extraktion unterzogen. Selbstverständlich kann der Extrakt auch erst isoliert und dann eine geeignete wässerige Lösung/Dispersion hergestellt werden, die dann in einer zweiten Extraktion extrahiert wird.

Die flüssig-flüssig Extraktion wird bevorzugt im sauren pH-Bereich durchgeführt. Dazu kann der wässerigen Lösung/Dispersion beispielsweise Salzsäure (HCl) zugegeben werden. Die Einstellung einer Säurekonzentration von 200 mMol/l bis 800 mMol/l ist bevorzugt, noch bevorzugter sind 400 mMol/l. Die Lösung wird dann bei erhöhter Temperatur, bevorzugt zwischen 30°C und 60°C, bevorzugter bei etwa 40°C, gerührt. Das Rühren erfolgt beispielsweise für etwa 20 Minuten. Es hat sich als bevorzugt erwiesen, wenn zu dieser Lösung/Dispersion ein Salz in Form einer schwachen Base, wie Natriumhydrogencarbonat, zugegeben wird, um die Säure etwas abzuschwächen, beispielsweise bis die Säurekonzentration sich um 50 mMol/l absenkt. Eine geeignete Menge und Konzentration an Säure und Salz kann der Fachmann durch wenige orientierende Versuche bestimmen. Unter "hydrogenem Salz" wird vorliegend ein Salz verstanden, bei dem die Säure-Wasserstoffatome nicht vollständig durch ein Metallatom ersetzt wurden.

Als Extraktionsmittel wird Ethylacetat verwendet. Ethylacetat wird bevorzugt im Verhältnis 1 zu 1 zu der wässerigen Phase zugegeben. Die Extraktion wird vorzugsweise unter Rühren durchgeführt. Es haben sich 30 min. Extraktionszeit als günstig erwiesen. Daraufhin wird die organische Phase abgetrennt. Der Extraktionsschritt kann wiederholt werden, um die Ausbeute zu steigern. Zwei Extraktionsschritte können bereits ausreichend sein. Anschließend wird die wässerige Phase verworfen und das Ethylacetat aus dem Extraktionsgemisch abgedampft. Ausdampfen unter Stickstofffluss ist dabei eine bekannt günstige Technologie, die bevorzugt verwendet werden kann. Es wird ein Feststoff gewonnen, der in der vorliegenden Erfindung auch als "konzentrierter Extrakt" bezeichnet wird. Dieser konzentrierte Extrakt ist in einer Mischung aus Ethanol und Wasser löslich.

Gewinnung des aktivierten Extrakts:

In unerwarteter Weise wurde festgestellt, dass eine chemische Modifikation des Extrakts in Form eines Aktivierungsverfahrens zu einer Wirkungsverstärkung beiträgt. Aus dem erhaltenen Extrakt (nur erste Extraktion) oder konzentrierten Extrakt (erste und zweite Extraktion) konnte durch Zugabe von (verdünnter oder konzentrierter) Salzsäure, Erwärmen zwischen 40 und 60°C, anschließender Zugabe von Wasser und optional einem basischen Salz und erneutem oder fortgesetztem Erwärmen sowie Einstellen eines neutralen pH-Werts, ein vollständig wasserlöslicher Extrakt bzw. konzentrierter Extrakt hergestellt werden. Der Ausdruck "erneutes oder fortgesetztes Erwärmen" bedeutet, dass das Erwärmen unterbrochen werden kann, dies aber nicht in jedem Fall erforderlich ist, so dass das Erwärmen während der Zugabe von Wasser und gegebenenfalls der Zugabe von Salz einfach kontinuierlich fortgeführt wird. Die Temperatur, auf die jeweils erwärmt wird, kann gleich oder verschieden sein.

Es wurde festgestellt, dass diese Behandlung eine Aktivierung und überraschende weitere Steigerung der antibakteriellen Wirksamkeit des Extrakts und daher auch seiner Inhaltsstoffe bewirkt.

Der aktivierte Extrakt wird durch ein zusätzliches Aktivierungsverfahren gewonnen, das die oben angegebenen Schritte aufweist. Bevorzugt schließt sich dieses Aktivierungsverfahren an das obige Verfahren zur Gewinnung des Extrakts und konzentrierten Extrakts ohne weitere Zwischenschritte direkt an.

Nachfolgend soll dieses Aktivierungsverfahren beispielhaft im Einzelnen erläutert werden, ohne die Erfindung hierauf zu beschränken:

In einem ersten Schritt wird Salzsäure (HCl) in konzentrierter oder verdünnter Form zum erhaltenen konzentrierten Extrakt, das als kristalliner Feststoff oder Feststoffgemisch, gegebenenfalls zusammen mit einer öligen Masse vorliegt, zugegeben. Zum Beispiel kann kontrolliert und unter Rühren 25%ige HCl zugegeben werden, bis sich eine deutliche Gelbfärbung ausprägt.

Dann wird das Gemisch erwärmt; eingesetzt wird hierbei eine Temperatur zwischen 40°C und 60°C, bevorzugter sind etwa 50°C. Es wird beispielsweise für 25 Minuten gerührt. Dann wird Wasser, bevorzugt destilliertes Wasser, zum Beispiel in dem dreifachen Volumenanteil der Salzsäure zugegeben und erneut erwärmt. Bevorzugt wird dann auf dieselbe Temperatur wie zuvor für mehrere Minuten erwärmt, beispielsweise für 10 Minuten.

Anschließend wird ein Salz oder Hydrogensalz aus einer schwachen Säure und einem Alkali- oder Erdalkalimetall zu dem Gemisch zugegeben. Bevorzugt wird beispielsweise Natriumhydrogencarbonat zugesetzt, bis der pH-Wert bei 8 ist. Unter Rühren wird dann wieder erwärmt, beispielsweise auf die obige Temperatur für 10 Minuten. Dann wird die Lösung auf einen pH-Wert von 7 abgepuffert. Die Lösung verfärbt sich dunkelgrün und wird klar im Fall des konzentrierten Extrakts, braun im Fall des Extrakts. Es können so Lösungen von c= 0,5 g/ml für den Extrakt und von c= 20 mg/ml für den konzentrierten Extrakt hergestellt werden.

Diese Lösungen werden im Folgenden als der "aktivierte Extrakt" bzw. der "aktivierte konzentrierte Extrakt" bezeichnet.

Die Verfahrensführung wurde speziell im Hinblick auf die Sophora japonica entwickelt und auf diese abgestimmt, so dass eine optimierte Verfahrensweise möglich wird. Das Verfahren zur Herstellung bzw. Gewinnung des Extrakts erlaubt daher eine besonders effiziente und ökonomische Arbeitsweise, wobei es gelingt, die Pflanzeninhaltsstoffe in hoher Ausbeute aus den Früchten zu extrahieren.

Bei dem beschriebenen Aktivierungsverfahren finden mehrere Prozesse statt: So kommt es zu einer Umwandlung einiger Flavonoide in die entsprechenden Hydrochloride sowie zur Bildung von Natriumsalzen der enthaltenen organischen Säuren. Die Flavonoid-Glukoside, wie Rutin, werden hydrolisiert, was die bakterizide Wirkung steigert. Des Weiteren erfolgt eine Umwandlung eines Terpen-Moleküls zu drei neuen isomeren Verbindungen.

Bei der Aktivierung wird der gesamte wasserlösliche und unlösliche Teil des Extrakts insgesamt in eine wasserlösliche Form überführt, und die antibakterielle Potenz wird dadurch erheblich gesteigert. Zudem lässt sich der aktivierte Extrakt aufgrund der Wasserlöslichkeit arzneimitteltechnisch sehr gut weiterverarbeiten.

Als eine schnelle und zuverlässige Methode, um den Gehalt an aktiven Substanzen in einer Charge des Extrakts zu analysieren, hat sich die Dünnschichtchromatographie erwiesen. Einzelheiten hierzu sind im experimentellen Teil angegeben.

Der wie oben beschrieben gewonnene Extrakt kann als Basis für eine Darreichungsform dienen, beispielsweise als Salbengrundlage zur äußeren Anwendung.

Bei der Herstellung von Darreichungsformen versteht es sich von selbst, dass die Produktionsabläufe und -umgebung in Übereinstimmung mit den Standards der Guten Herstellungspraxis (GMP) zur Qualitätssicherung in der Produktion von Arzneimitteln und Wirkstoffen durchgeführt werden.

Der Extrakt enthält neben den antibakteriell wirkenden Inhaltsstoffen auch Kohlenwasserstoff-Polymere, die eine gute Trägersubstanz bilden und ebenfalls in gewissen Verabreichungsformen sinnvoll anzuwenden sind. Es wurden sowohl der Extrakt als auch der aktivierte und konzentrierte Extrakt auf die bakterizide Potenz hin untersucht. Es stellte sich heraus, dass der aktivierte konzentrierte Extrakt eine vergleichbare Potenz wie bereits in der Therapie eingesetzte moderne Antibiotika zeigt.

Tests zur Untersuchung der antibakteriellen Wirksamkeit der aktivierten Extraktlösung gegen Bakterien ergaben eine Wirkungsverstärkung, die sich darin zeigte, dass sich der Breakpoint der aktivierten Extraktlösung um einen Faktor von etwa 8 bis 10 verschiebt. Damit ergaben sich für ausgetestete Staphylococcus aureus Stämme eine hemmende Konzentration von 1 mg/ml des aktivierten Sophora japonica-Extrakts und eine hemmende Konzentration von 20 μg/ml des aktivierten konzentrierten Sophora japonica-Extrakts. Das entspricht einem handelsüblichen auch oral einsetzbaren Antibiotikum, das ebenfalls in den Versuchen parallel mitgetestet wurde.

Die erwünschte Wirkungsverstärkung durch das Aktivierungsverfahren ist daher wohl auf mehrere Effekte zurückzuführen: Nach der Aktivierung, bei der insbesondere eine Hydrochlorid-Bildung stattfindet, sind die zusätzlich gewonnenen Salze sehr gut wasserlöslich, was sich positiv auf die Wirkung im biologischen System auswirkt. Die im Extrakt enthaltenen Fettsäuren bilden Natriumsalze, die ebenfalls wasserlöslich sind, wie z.B. das Natriumoleat. Des Weiteren werden die Rutin-Glykoside hydrolysiert, was den Quercetin-Anteil erhöht, welcher ebenfalls die antibakterielle Wirkung zusätzlich steigert. Schließlich findet eine Umlagerung an einem enthaltenen Terpen-Molekül statt, welche ebenfalls die antibakterielle Wirkung verstärkt.

## Chemische Zusammensetzung des Extrakts

Zum besseren Verständnis der Wirkung und des Aufbaus des Extrakts wurde die chemische Zusammensetzung genauer analysiert. Zur Bestimmung der chemischen Zusammensetzung wurden verschiedene Trennverfahren sowie Analysen der aufgetrennten Fraktionen durchgeführt. Als Trennverfahren wurden Säulenchromatographie (SC), Dünnschichtchromatographie (DC) und Gaschromatographie (GC) verwendet. Als Analyseverfahren wurden Kernspinresonanz (NMR) und Massenspektrometrie (MS) eingesetzt.

Nach der GC-Auftrennung des Gesamtextrakts wurden die Massenspektren der isolierten Substanzen mit dem Massenspektrometer (MS) analysiert. Eine Reihe von Substanzen konnte anhand der charakteristischen Fragmentierungspeaks identifiziert werden. Als bislang noch nicht in der Literatur beschriebene chemische Komponenten der Sophora japonica konnten Parabene (para-Hydroxybenzoesäure-Ester oder PHB-Ester) sowie 4',5,7-Triacetoxyflavon identifiziert werden. Sowohl das NMR- als auch das Massenspektrum belegen das Vorhandensein der Substanzen im Extrakt.

Unter dem Begriff "Parabene" werden para-Hydroxybenzoesäure-Ester (abgekürzt: PHB-Ester) verstanden, in denen der Rest R eine Alkylgruppe bedeutet. Die beiden Begriffe sollen hier synonym verstanden werden.

Des Weiteren konnten unter den zahlreichen vorhandenen Verbindungen das Flavanol-Glukosid, Rutin (auch Rutosid genannt), identifiziert werden. Zur Kontrolle wurde von im Handel erhältlichem Rutin ein Massenspektrum aufgenommen und mit dem aus Sophora japonica erhaltenen Rutin verglichen. Die Spektren waren praktisch identisch, so dass tatsächlich Rutin als weitere Verbindung im erhaltenen Extrakt der Sophora japonica vorliegt.

Als weitere Flavonoide wurden Quercetin und 4',5,7-Triacetoxyflavon isoliert. Als Vertreter der Pterocarpane wurde Maackiain isoliert. Auch eine Hydrazin-Verbindung, nämlich 1,1-Dimethyl-2-pentylhydrazin wurde aus dem Extrakt abgetrennt und identifiziert.

Als weitere Verbindungen konnten aus dem Gesamtextrakt das Triterpen (Squalen) sowie einige organische Säuren, wie die Linolensäure und die Ölsäure isoliert werden. Es hat sich herausgestellt, dass bei der Aktivierung eine Umlagerungsreaktion zum Entstehen des Isopropylmaltol führt.

Des Weiteren wurde festgestellt, dass sich bei der flüssig-flüssig Extraktion aus der sauren Phase Hydroxymethylfurfural (HMF) bildet und somit ebenfalls in dem als konzentrierter Extrakt bezeichneten Stoffgemisch vorliegt.

Auch 4',5,7-Triacetoxyflavon und 3,5,7-Triacetoxy-4'-acetoxyflavon wurden als antibakterielle Substanzen identifiziert.

In der nachfolgenden Tabelle 1 sind einige aus dem Extrakt erhaltene Verbindungen oder Verbindungsklassen mit ihrer chemischen Strukturformel und den chemischen Formelnamen zusammengestellt:

Tabelle 1

| Bezeichnung | Strukturformel |
|---|---|
| Parabene oder para-Hydroxybenzoesäure-Ester (PHB-Ester) R = Alkylgruppe | 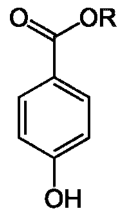 |
| Rutin | 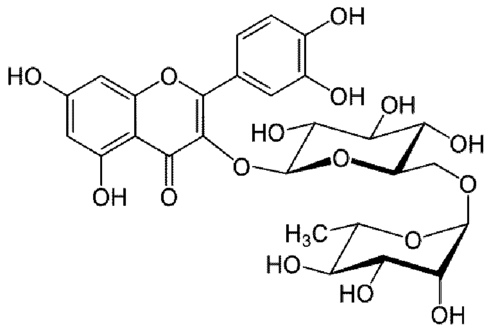 |
| 1,1-Dimethyl-2-pentylhydrazin | 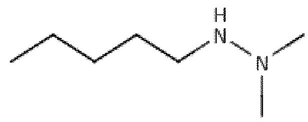 |
| 4',5,7-Triacetoxyflavon | 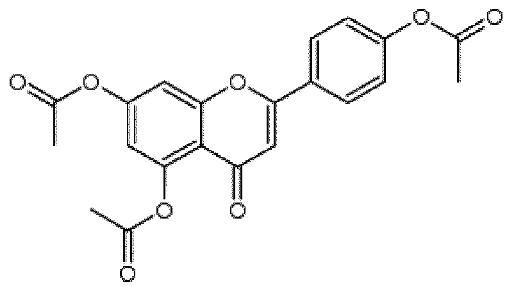 |
| Quercetin | 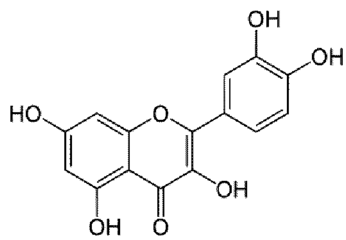 |
| Pterocarpane | 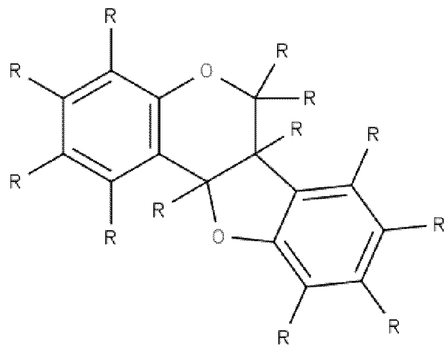 |

(fortgesetzt)

| Bezeichnung | Strukturformel |
|---|---|
| Kaempferol | 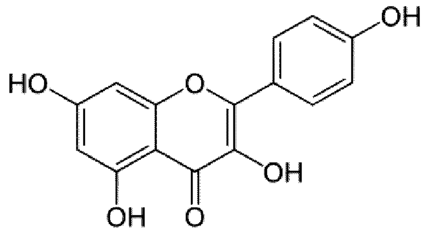 |
| Flavon | 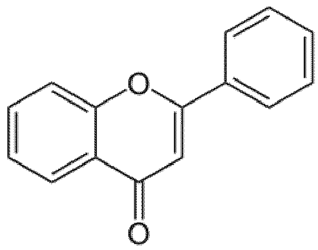 |
| Isoprpylmaltol | 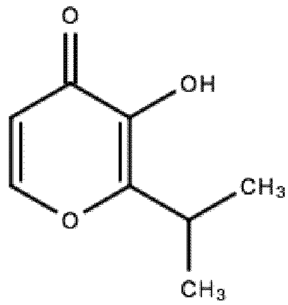 |
| Hydroxymethylfurfural | 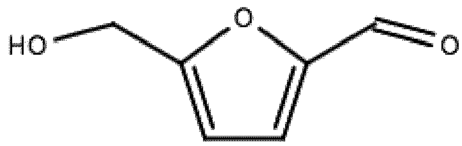 |

In den Abbildungen sind die Spektren von einigen der isolierten Verbindungen abgebildet. Im Einzelnen zeigen:

Abb. 1a das Massenspektrum des para-Hydroxybenzoesäure-Ethylesters (PHB-Ester;
Abb. 1b das $^{1}$H-NMR-Spektrum des para-Hydroxybenzoesäure-Ethylesters (PHB-Ester);
Abb. 2a das Massenspektrum von Rutin aus der Sophora japonica;
Abb. 2b das Massenspektrum von im Handel erhältlichem Rutin als Referenzspektrum;
Abb. 3 das Massenspektrum von 1,1-Dimethyl-2-pentylhydrazin;
Abb. 4 das Massenspektrum von 4',5,7-Triacetoxyflavon;
Abb. 5 das Massenspektrum von Quercetin;
Abb. 6 das Massenspektrum von Maackiain;
Abb. 7 das Massenspektrum von Squalen;
Abb. 8 das Massenspektrum von Isopropylmaltol;
Abb. 9 das Massenspektrum von Hydroxymethylfurfural;

Die Spektren wurden mit den nachfolgend angegebenen Geräten unter den folgenden Bedingungen aufgenommen:
Das NMR Spektrum wurde mit einem NMR-Spektrometer AVANCE der Firma BRUKER bei SF = 400,13 MHz aufgenommen. Deuterochlorophorm wurde als Lösungsmittel verwendet.
Die Massenspektren wurden mit dem "GCMS 5975 C inert XL MSD with triple-Axis detector" der Firma Agilent aufgenommen.
Für die Trennung wurde folgende Säule verwendet: HP1 von Agilent mit 12 m Länge und einem Durchmesser

von 0,2 mm und einer Filmdicke von 0,33 μm (100% Dimethylpolysiloxane-Beschichtung).

Es wurden 0,5 μl Injektionsvolumen bei einer Injektionstemperatur von 250°C eingegeben.

Das Temperaturprogramm war wie folgt strukturiert: 60°C Anfangstemperatur, danach linearer Anstieg von 20°C/Minute bis auf 280°C - 19 Minuten halten und ergab 30 Minuten Gesamtlaufzeit.

Für die Massenspektrometrie wurde Elektrische Ionisation (EI) bei -70V verwendet.

Der Begriff "Abundance" auf den Massenspektren bedeutet die absolute Intensität. Die Auswertung der Spektren führte zu den nachfolgenden Ergebnissen:

Abb. 1a Massenspektrum des para-Hydroxybenzoesäure-Ethylesters (PHB-Ester):

$C_9H_{10}O_3$ (166,17);

Massenspektrometrie ($EI^+$): Hauptpeak bei m/z = 121).

Abb. 1b $^1$H-NMR-Spektrum des para-Hydroxybenzoesäure-Ethylesters (PHB-Ester):
$^1$H-NMR ($CDCl_3$, 400,13 MHz): δ 8,03 (s, 1H), 7,4 (d, J = 9 Hz, 2H), 6,8 (d, J = 9 Hz, 2H), 3,3 (Quartett, 2H), 1,3 (Triplett, 3H), Lösungsmittel-Signal der nicht deuterierten Rückstände bei δ = 4,8 ppm.

Abb. 2a Massenspektrum von Rutin aus der Sophora japonica:

$C_{27}H_{36}O_{19}$ (664,56);

Massenspektrometrie ($EI^+$): Hauptpeak bei: m/z= 303.

Abb. 2b Massenspektrum von im Handel erhältlichem Rutin als Referenzspektrum:

$C_{27}H_{36}O_{19}$ (664,56);

Massenspektrometrie ($EI^+$): Hauptpeak bei: m/z= 303.

Abb. 3 Massenspektrum von 1,1-Dimethyl-2-pentylhydrazin:

$C_7H_{17}N_2$ (130,14);

Massenspektrometrie ($EI^+$): Hauptpeak bei m/z = 59.

Abb. 4 Massenspektrum von 4',5,7-Triacetoxyflavon:

$C_{21}H_{16}O_8$ (396,08);

Massenspektrometrie ($EI^+$): Hauptpeak bei m/z = 270.

Abb. 5 Massenspektrum von Quercetin:

$C_{15}H_{10}O_7$ (302,04);

Massenspektrometrie (zur Löslichkeitsverbesserung einfach-acetyliertes Molekül): ($EI^+$): Hauptpeak bei m/z = 346 entspricht (m = 43 für Acetylrest) m/z = 303 $[M+H]^+$.

Abb. 6 Massenspektrum von Maackiain:

$C_{16}H_{12}O_5$ (284,07);

Massenspektrometrie ($EI^+$): Hauptpeak bei m/z = 284 $[M+H]^+$.

Abb. 7 Massenspektrum von Squalen:

$C_{30}H_{50}$ (410,71);

Massenspektrometrie ($EI^+$): Hauptpeak bei m/z= 69.

Abb. 8 Massenspektrum von Isopropylmaltol:

$C_8H_{10}O_3$ (154,16);

Massenspektrometrie ($EI^+$): Hauptpeak bei m/z= 154 $[M+H]^+$.

Abb. 9 Massenspektrum von Hydroxymethylfurfural:

$C_6H_6O_3$ (126,11);

Massenspektrometrie ($EI^+$): Hauptpeak bei m/z= 126 $[M+H]^+$.

Die neue antibakteriell wirksame Zusammensetzung, umfassend oder bestehend aus einem Extrakt aus den Früchten der Sophora japonica, die einer chemischen Modifikation in Form eines Aktivierungsverfahrens unterzogen wurden, weist daher eine Vielzahl von biologisch aktiven Substanzen auf, von denen einige eindeutig identifiziert wurden, was in der bisher bekannten Literatur in der Form nicht erfolgte.

## Synergistische Wirkung

Gemäß einem Aspekt der vorliegenden Erfindung wird von einer synergistischen Wirkung des Gesamtextrakts von Sophora japonica ausgegangen. Bei einer Kombination von Komponenten liegt dann eine synergistische Wirkung vor, wenn sich die Kombinationswirkung nicht in der Summe der Einzelwirkungen erschöpft, sondern die Gesamtwirkung in therapeutischer Hinsicht über die Wirkung der einzelnen Komponenten hinausgeht. Dies kann durch den Vergleich und die Gegenüberstellung der Wirkungen der Komponenten erfolgen und belegt werden. Beispielsweise ist die Wirkung dann als synergistisch anzusehen, wenn die Gesamtwirkung von mehreren Komponenten der Wirkung, die mit einer Komponente allein erreicht werden kann, überlegen ist.

In der vorliegenden Erfindung wird das Ausmaß der Hemmwirkung gegenüber einem Bakterienstamm von einer oder mehrerer Komponenten als Kriterium für die Beurteilung herangezogen. Um dies zu untersuchen, wurde eine Vielzahl verschiedener Tests durchgeführt, die im experimentellen Abschnitt im Einzelnen erläutert sind.

Die durchgeführten Tests führten zu folgenden Ergebnissen:

Zunächst wurde gefunden, dass die Wirkung von para-Hydroxybenzoesäure-Ethylester (ein bevorzugter PHB-Ester) allein geringer war als die Wirkung des gesamten Extrakts von Sophora japonica. Ferner wurde festgestellt, dass die Effektivität der Wirkung wahrscheinlich wesentlich von der Zusammenwirkung des PHB-Esters und der Flavonoide sowie weiterer Verbindungen der Sophora japonica bestimmt wird. Wichtige Komponenten der Flavonoid-Zusammensetzung sind Rutin, Kaempferol, Quercetin, Flavon und das 4',5,7-Triacetoxyflavon.

Die Tests belegen, dass die Mischung aus den Flavonoiden und dem PHB-Ester wesentlich potenter das Bakterienwachstum hemmt als der PHB-Ester allein. Die PHB-Ester-Konzentration, die zur Hemmung notwendig ist, beträgt ca. 65 mg/ml, was allein keine ausreichende antibakterielle Potenz darstellen würde. Der PHB-Ester wäre alleine kein geeignetes antibakterielles Mittel. Erst in Kombination mit einem oder mehreren der anderen Bestandteile des Extrakts kann dieser eine vorteilhafte antibakterielle Wirkung entfalten.

Die Kombination des PHB-Esters mit Rutin verringert zudem die zur Hemmung notwendige Konzentration auf 10 mg/ml, was eine signifikante Verstärkung der antibakteriellen Wirkung darstellt. Dies ist in hohem Maße überraschend, da Rutin in der Lösung in einer Konzentration von weniger als 1 mg/ml vorlag. Zudem hat Rutin allein überhaupt keine antibakteriell hemmende Wirkung. Erst in Kombination mit den anderen Komponenten werden die vorteilhaften Wirkungen erzielt.

Aus den Tests lässt sich daher schließen, dass die Wirkung auf einer Synergie der Komponenten basiert, die man in dieser Form nur in einem pflanzlichen Naturstoff findet, der sich in der Natur durch den Synergismus einen Selektionsvorteil durch die antibakterielle Wirksamkeit verschafft. Es wird angenommen, dass gerade diese Multikomponenten-Beschaffenheit des Extrakts es aber auch den Mikroorganismen deutlich erschwert, Resistenzen gegen den Extrakt zu bilden.

Des Weiteren konnte mit den durchgeführten Tests gezeigt werden, dass eine Zugabe von PHB-Ester zu dem Extrakt der Sophora japonica den Breakpoint zu geringeren Konzentrationen verschieben kann. Dies bedeutet, dass geringere Konzentrationen des Extrakts notwendig sind, um eine Hemmung der Bakterienstämme zu erreichen, d.h. die antibakterielle Wirksamkeit steigt deutlich an. Hierbei wirkt der PHB-Ester zusammen mit einer oder mehreren Verbindungen aus der Sophora japonica, wie beispielsweise dem Rutin, 4',5,7-Triacetoxyflavon, Maackiain und den

oben genannten Flavonoiden.

Ausgehend von dieser vorteilhaften Wirkung wurde die PHB-Ester-Konzentration bestimmt, die dem Sophora japonica-Extrakt bevorzugt zugegeben wird, um eine optimale Wirkung zu erzielen. Es hat sich hierbei als besonders vorteilhaft erwiesen, wenn der PHB-Ester, bevorzugt para-Hydroxybenzoesäure-Ethylester oder para-Hydroxybenzoesäure-Propylester, zum Extrakt in einer derartigen Menge zugegeben wird, dass ein Verhältnis von PHB-Ester : Sophora japonica-Extrakt im Bereich von 0,1 bis 5 : 5 bis 15, bezogen auf das Gewicht, vorliegt, bevorzugt 0,5 bis 3 : 8 bis 12, bezogen auf das Gewicht, ganz besonders bevorzugt 1 : 10, bezogen auf das Gewicht. Bei einem Massenverhältnis von PHB-Ester : Extrakt von 1 : 10, liegt der Extrakt in der 10fachen Gewichtsmenge gegenüber der Menge an PHB-Ester vor. Der PHB-Ester ist in diesem Fall der zum Extrakt zusätzlich zugegebene Ester, der im Extrakt bereits vorliegende PHB-Ester wird hierbei nicht berücksichtigt. Insgesamt liegt daher die Gesamtmenge an PHB-Ester (aus der Zugabe und dem Extrakt) höher als angegeben.

Durch Zugabe des PHB-Esters, bevorzugt in den oben angegebenen Bereichen, verschiebt sich daher der Breakpoint - wie mit den durchgeführten Tests belegt - in vorteilhafter Weise. Beispielsweise wird durch eine Zugabe von 0,5 mg/ml PHB-Ester zum Extrakt eine hemmende Konzentration bei 5 mg/ml bei dem hier getesteten Staphylococcus aureus-ATCC-Stamm erhalten. Ohne Zusatz von PHB-Ester, d.h. wenn nur der Extrakt an sich verwendet wird, erhält man einen Breakpoint bei 10 mg/ml. Es wird daher eine deutliche Steigerung der antibakteriellen Wirksamkeit durch Zusatz von PHB-Ester erzielt.

Als Faustregel kann angegeben werden, dass der PHB-Ester, wie beispielsweise para-Hydroxybenzoesäure-Ethylester, bevorzugt in einer Konzentration/Menge von 0,01 g/ml bis 1 g/ml, bevorzugt von 0,05 g/ml bis 0,5 g/ml, ganz besonders bevorzugt von 0,1 g/ml bis 0,2 g/ml, zum Sophora japonica-Extrakt zusätzlich zugegeben wird.

Bemerkenswert in diesem Zusammenhang ist, dass die Konzentration/Menge des PHB-Esters im Extrakt auch nach zusätzlicher Zugabe im Allgemeinen weit geringer ist als die Konzentration/Menge, die benötigt wird, damit der PHB-Ester als Einzelsubstanz eine antibakterielle Wirkung entfaltet; diese liegt bei über 60 mg/ml.

Weiterhin wurden für die Kombination der Substanzen in den durchgeführten Tests die folgenden Ergebnisse erhalten:
Es konnte gezeigt werden, dass die Flavonoid-Mischung aus Quercetin, Kaempferol und Rutin eine wesentlich stärkere Wirkung zeigt als die jeweiligen Einzelsubstanzen.

Die minimalen hemmenden Konzentrationen (MHK) betrugen für die Substanzen:

Tabelle 2:

| | | | | | |
|---|---|---|---|---|---|
| Rutin | 0,04 mg/ml | Kaempferol | 0,04 mg/ml | Quercetin | 0,1 mg/ml |

Des Weiteren wurde gefunden, dass durch die Zugabe von PHB-Ester die Breakpoints bei den Einzelsubstanzen in vorteilhafter Weise zu niedrigeren minimalen, für die Hemmung von Bakterien notwendigen, Konzentrationen verschoben wurden. Die benötigten minimalen Hemmkonzentrationen (MHK) bei den Einzelsubstanzen waren nun wie folgt:

Tabelle 3:

| | | | | | | | |
|---|---|---|---|---|---|---|---|
| Rutin | 0,01 mg/ml | Kaempferol | 0,02 mg/ml | Quercetin | 0,04 mg/ml | PHB | 8 mg/ml |

In einer Reihe von Tests konnte zudem gezeigt werden, dass der Breakpoint bei der getesteten Flavonoid-Mischung durch PHB-Ester ebenfalls in vorteilhafter Weise zu niedrigeren minimalen, für die Hemmung von Bakterien notwendigen, Konzentrationen verschoben werden konnte. Die Konzentration des zugegebenen PHB-Esters betrug 8 mg/ml, d.h. eine Konzentration, die weit von dem Breakpoint des PHB-Esters (über 60 mg/ml) entfernt liegt.

Tabelle 4:

| | | | |
|---|---|---|---|
| Quercetin | 0,3 mg/ml | Quercetin + PHB | 0,16 mg/ml |
| Kaempferol | Keine Hemmung | Kaempferol + PHB | 0,1 mg/ml |
| Flavon | 2 mg/ml | Flavon + PHB | 0,5 mg/ml |

Bei allen Testreihen verstärkte die PHB-Ester-Zugabe daher die antibakterielle Flavonoid-Wirkung um ein Vielfaches.

Eine weitere Synergiewirkung wurde zwischen dem Hydroxymethylfurfural (HMF) und Flavonoiden z.B. dem Quercetin festgestellt. Dabei hat es sich herausgestellt, dass der Breakpoint, der bei HMF allein bei 5 mg/ml liegt, sich durch Zugabe von Quercetin auf etwa 0,5 mg/ml absenken lässt. Von besonderer Bedeutung ist, dass die dabei zugegebene Konzentration von Quercetin unter 0,2 mg/ml lag. Diese eingesetzte Konzentration ist damit unterhalb der Konzentration, bei der Quercetin selbst eine relevante Hemmung entwickeln kann.

Ein ähnlicher Effekt wurde bei der Zugabe von Rutin beobachtet. Dies belegt, dass es eine weitere Synergie-Wirkung zwischen dem HMF und einigen Flavonoiden, insbesondere Quercetin und Rutin gibt.

Die durchgeführten Tests und die erhaltenen Ergebnisse machen deutlich, dass die Wirkung der antibakteriellen Zusammensetzung auf Mikroorganismen wahrscheinlich auf einem auf den Multikomponenten beruhenden Mechanismus basiert. Es wird angenommen, dass dies der Grund ist, warum es für Bakterien deutlich schwieriger ist, Resistenzen gegen den Wirkkomplex zu bilden. Dies ist eine mögliche Erklärung, warum die antibakterielle Zusammensetzung der Erfindung auch bei multiresistenten Keimen seine Wirksamkeit entfaltet und diese effektiv hemmt.

Darreichungsformen

Die antibakterielle Zusammensetzung kann als pharmazeutische Zusammensetzung, zusammen mit einem oder mehreren pharmazeutisch akzeptablen Trägern und/oder Hilfsstoffen in einer geeigneten Darreichungsform verabreicht werden.

Die pharmazeutische Zusammensetzung kann in fester, halbfester, flüssiger oder gasförmiger Darreichungsform bereitgestellt werden, weil die antibakteriell wirksamen Substanzen des Pflanzenextraktes einem Träger in der vorliegenden Form gut beigegeben werden können. Zur oralen Verabreichung kann die pharmazeutische Zusammensetzung beispielsweise in fester Dosierungsformen, wie Kapseln, Tabletten, Pellets, Pillen, Pulvern oder Granulaten oder in flüssiger Dosierungsform, wie Tropfen, Emulsionen, Mikroemulsionen, Lösungen, Suspensionen, Sirupe oder Elixiere formuliert werden.

Die pharmazeutische Zusammensetzung kann beispielsweise zur topischen und/oder systemischen Behandlung eines Patienten eingesetzt werden. Ein Patient kann ein Mensch oder Tier sein, der/das einer entsprechenden äußerlichen Behandlung bedarf. Eine topische Behandlung bedeutet, dass die pharmazeutische Zusammensetzung nur örtlich, z.B. in Form von Salben, Cremes, Sprays, Tinkturen oder Tropfen, aufgetragen oder eingebracht wird. Die topische Behandlung ist in der Regel nebenwirkungsärmer als die systemische, da hohe Wirkstoffkonzentrationen nur in einem umschriebenen Körperareal erreicht werden und nicht in hohen Konzentrationen in den Stoffwechsel gelangen.

Zur topischen lokalen Verabreichung kann die pharmazeutische Zusammensetzung in einem oder mehreren pharmazeutisch akzeptablen Trägermedien formuliert werden. Typische derartige Formulierungen sind Salben, Cremes, Pasten, wie Zahnpasta, Lotionen, Gelees, Lösungen, Suspensionen, Tropfen, Tinkturen, Öl-in-Wasser- oder Wasser-in-Öl-Emulsionen, Sprays, imprägnierte Verbände, Pflaster und dergleichen.

Beispielsweise kann eine antibakteriell wirksame Tinktur zur äußeren Anwendung zur Verfügung gestellt werden.

Die pharmazeutische Zusammensetzung kann beispielsweise direkt auf die Haut (dermal) aufgebracht werden. Diese kann auf bestimmte zu behandelnde Körperstellen lokal begrenzt (topisch) aufgebracht werden, um eine Erkrankung zu behandeln, die nicht den ganzen Körper betrifft, sondern lediglich ein klar umgrenztes erkranktes Areal.

Die systemische Behandlung hat den Vorteil, dass eine optimale Dosierung in verschiedene Kompartimente gelangen kann und z.B. auch eine Gewebedurchdringung erfolgen kann, so dass der oder die antibakterielle(n) Wirkstoff(e) sein/ihr optimales Wirkungsspektrum an unterschiedlichen Stellen, die z.B. von einer Infektion mit Bakterien betroffen sind, entfalten kann/können.

Gemäß einer bevorzugten Ausführungsform der Erfindung wird daher sowohl eine Präparation für eine topische als auch eine systemische Behandlung angestrebt. Die Verabreichungswege umfassen beispielsweise kutan bzw. dermal (z.B. Salbe, Tinktur), transdermal (z.B. transdermales Pflaster, Salbe), (intra-)nasal (z.B. Tropfen, Tinktur) oder oral bzw. peroral (z.B. Pulver, Tropfen, Tabletten).

Die Konzentration in dem Extrakt hängt von einer Vielzahl von Faktoren ab, wie beispielsweise der Art der Extraktgewinnung und welche Komponenten darin enthalten sind. Die nachfolgenden Konzentrations-/Mengen-Angaben sind zur Veranschaulichung gedacht und können dem Fachmann zur Orientierung dienen:

Im Falle des Extrakts nach der fest-flüssig Extraktion: Es liegt beispielsweise eine Konzentration/Menge des Extrakts in der pharmazeutischen Zusammensetzung im Bereich von 0,01 g/ml bis 1 g/ml, bevorzugt von 0,1 g/ml bis 0,2 g/ml, ganz besonders von 0,13 g/ml bis 0,17 g/ml vor.

Im Falle des konzentrierten Extrakts nach der flüssig-flüssig Extraktion: Beispielsweise liegt eine Konzentration/Menge des konzentrierten Extrakts in der pharmazeutischen Zusammensetzung im Bereich von 0,1 mg/ml bis 10 mg/ml, bevorzugt von 1 mg/ml bis 2 mg/ml, ganz besonders von 1,4 mg/ml bis 1,7 mg/ml vor.

Eine bevorzugte Verabreichungsform der Erfindung ist eine Salbe. Diese kann beispielsweise durch Vermischen des, bevorzugt auf einen geeigneten pH-Wert eingestellten, Konzentrats aus (unbehandeltem/konzentriertem/aktiviertem) Extrakt und Wasser, wie oben beschrieben, mit einem geeigneten Trägermedium, hergestellt werden. Gemäß einer bevorzugten Ausführungsform wird Konzentrat: Trägermedium im Verhältnis 1 : 2, bezogen auf das Gewicht, für eine Salbe verwendet. Als Trägermedium kann jedes dem Fachmann bekannte Trägermedium verwendet werden. Für

Beispiele wird auf das Deutsche Arzneibuch (DAB 10) verwiesen. Somit kann eine gut haltbare Salbe für äußerliche, antibakterielle Anwendung und zur Wundheilung bereitgestellt werden.

Topische Anwendungen umfassen auch die intranasale Verabreichung, wobei die Formulierungen ebenfalls kompatible herkömmliche Träger und/oder Hilfsstoffe aufweisen können.

Eine transdermale Verabreichung ist ebenfalls möglich, beispielsweise in Form von einzelnen Pflastern, die angepasst sind, um mit der Epidermis des Empfängers für eine verlängerte Zeitspanne in engem Kontakt zu bleiben.

In den pharmazeutischen Zusammensetzungen wird der Extrakt in der Regel mit einem oder mehreren pharmazeutisch annehmbaren Trägern und/oder einem oder mehreren pharmazeutisch annehmbaren Hilfsstoffen formuliert. Die Träger oder Hilfsstoffe, die verwendet werden, sind selbstverständlich im Sinne einer Kompatibilität mit den anderen Bestandteilen der Zusammensetzung annehmbar bzw. akzeptabel und dürfen für den Patienten nicht schädlich sein. Geeignete Träger und Hilfsstoffe für die einzelnen Darreichungsformen sind in der pharmazeutischen Literatur bekannt.

Die Vorteile der vorliegenden Erfindung sind außerordentlich vielschichtig:

Es wird eine neue antibakteriell wirksame Zusammensetzung mit dem Extrakt aus den Früchten der Sophora japonica bereitgestellt, der einer chemischen Modifikation in Form eines Aktivierungsverfahrens unterzogen wurde. So konnte bei in vitro-Untersuchungen die bakterizide Wirksamkeit des Extraktes aus den Früchten der Sophora japonica gegen verschiedene pathogene oder fakultativ pathogene Bakterienstämme, wie sie bei Wunden auf der Haut anzutreffen sind, gezeigt werden. Anwendungsversuche bestätigen, dass neben der bakteriziden Wirkung auch gegen multiresistente Erreger, insbesondere aus der Gruppe der grampositiven Erreger, (MRSA) eine positive Wundheilung zu beobachten ist. Des Weiteren konnte eine eindeutige Wirkung auch auf andere multiresistente Erreger, wie die Vancomycin-resistenten Enterokokken, nachgewiesen werden. Dies wird auf die wirkungsstarken Komponenten para-Hydroxybenzoesäure-Ester, Rutin, Triacetoxyflavon, Flavon, Quercetin und Maackiain in der antibakteriellen Zusammensetzung zurückgeführt.

Es kann davon ausgegangen werden, dass die Multikomponenten-Zusammensetzung des Extrakts es Mikroorganismen deutlich erschwert, Resistenzen gegen die Zusammensetzung zu bilden.

Der Extrakt lässt sich in sehr effizienter Weise aus den Früchten der Sophora japonica gewinnen, wobei ein Verfahren zur Gewinnung unter optimierten Extraktionsbedingungen bereitgestellt wird. Das Verfahren zur Herstellung bzw. Gewinnung des Extrakts ermöglicht es, die Ausbeute deutlich zu erhöhen.

Die antibakterielle Wirkung konnte um ein Vielfaches erhöht werden, indem eine weitere Extraktion (zweite oder flüssig-flüssig Extraktion) und ein zusätzliches Aktivierungsverfahren durchgeführt wurde. Hierdurch gelang es, den in Wasser unlöslichen Teil des Extrakts, beispielsweise durch Versetzen mit verdünnter oder konzentrierter Salzsäure, in Hydrochlorid(e) umzuwandeln. Die Hydrochlorid-Bildung führte zu einer wesentlich besseren Löslichkeit in Wasser und damit wohl zur verstärkten Aktivität in biologischen Systemen. Eine derartige Aktivierung wurde bislang im Stand der Technik nicht beschrieben.

Ferner konnten Synergie-Effekte verschiedener chemischer Inhaltsstoffe der antibakteriellen Zusammensetzung belegt werden. So hat es sich als besonders vorteilhaft herausgestellt, wenn der antibakteriellen Zusammensetzung ein para-Hydroxybenzoesäure-Ester zugegeben wird. Sämtliche Tests zeigen, dass die PHB-Ester-Zugabe die antibakterielle Wirksamkeit um ein Vielfaches erhöht.

Somit lassen sich sehr effektive antibakteriell wirksame Darreichungsformen je nach der Art der Zusammensetzung herstellen.

Der in vivo-Test der Salbe, die auf der Grundlage des Extrakts der Sophora japonica formuliert wurde, lieferte bereits sehr positive Resultate.

## Experimenteller Abschnitt

### Tests zur Bestimmung der antibakteriellen Wirksamkeit

Es wurden Tests durchgeführt, um die antibakterielle Wirksamkeit des Extrakts von Sophora japonica zu untersuchen. Ziel der Tests war eine Abschätzung der Wirksamkeit des Extrakts mit Hilfe der ermittelten Hemmkonzentrationen anhand von Extinktionsmessungen, die einen Rückschluss über die antibakterielle Aktivität des untersuchten Extrakts gegen einen untersuchten Krankheitserreger ermöglichen. Für die vergleichende Bewertung der antibakteriellen Zusammensetzung in Form des Extrakts bei einer Vielzahl von Erregern werden hier Konzentrationen herangezogen, die nur 50% des maximalen Bakterienwachstums zulassen. Diese minimale Hemmkonzentration wird als Breakpoint bezeichnet. Hierdurch kann auf eine antibakterielle Wirksamkeit der antibakteriellen Zusammensetzung, die in vivo zu erwarten ist, geschlossen werden.

**In vitro-Testmethode**

Die antibakterielle Wirksamkeit des Extrakts wurde in vitro unter Verwendung des Mikrodilutionsverfahrens bestimmt. Dabei wurde eine NaCl-Lösung der Erreger (McFarland 0.5) verwendet. Die Wachstumshemmung wurde durch die Veränderung der optischen Extinktion bei 450 nm nachgewiesen.

Nach dem Lambert-Beerschen-Gesetz ist die Konzentration von streuenden Objekten in einer Lösung proportional zu dem Logarithmus der Extinktionsänderung. Es gilt:

$$\log\left(\frac{I_0}{I_q}\right) = a \cdot c$$

$I_0$... Intensität des transmittierten Lichts (Einheit: $W \cdot m^{-2}$)
$I_q$... Intensität des einfallenden (eingestrahlten) Lichts (Einheit: $W \cdot m^{-2}$)
a.... Proportionalitätskoeffizient (Einheit: ml/g)
c.... Konzentration (Einheit: g/ml)

Zur Versuchsdurchführung wurden 50 μl der zu testenden Hemmsubstanz (Sophora japonica-Extrakt in destilliertem Wasser) mit 50 μl McFarland 0.5-Bakterien-NaCl-Lösung zusammengebracht und 100 μl Hirn-Herz-Aufguss-Bouillon (Brain Heart Bouillon = BHI) als Nährmedium hinzugefügt. Es wurde eine Verdünnungsreihe mit der Testzusammensetzung angesetzt, die Verdünnungsschritte können der nachfolgenden Tabelle 5 entnommen werden.

Tabelle 5: Konzentrationsstufen der Testzusammensetzung für die 12 eingesetzten Verdünnungsstufen

| Verdünnungsstufe | 1 | 2 | 3 | 4 | 5 | 6 | 7 | 8 | 9 | 10 | 11 | 12 |
|---|---|---|---|---|---|---|---|---|---|---|---|---|
| [mg/ml] | 160 | 80 | 40 | 20 | 10 | 5 | 2,5 | 1,3 | 0,6 | 0,3 | 0,15 | 0 |

Die Extinktionsdifferenzen wurden nach 24 Stunden Inkubationszeit bestimmt. Als Messapparat wurde ein Standard ELISA-Scanner eingesetzt. Mithilfe dieses Verfahrens konnte die Konzentration ermittelt werden, die nur 50% des maximalen Bakterienwachstums zulässt. Diese wird als Breakpoint (BP) bezeichnet.

Es wurden folgende Stämme untersucht:

- Staphylococcus aureus ATCC: 29213,
- Streptococcus pyogenes ATCC: 12344,
- Enterococcus faecalis ATCC: 29212,
- Klebsiella pneumoniae ATCC: 12344,
- Staphylococcus epidermidis ATCC: 12228,
- Escherichia coli ATCC: 25922.

Des Weiteren wurden Clindamycin-resistente und -sensitive Staphylokokken-Stämme getestet, sowie Vancomycin-resistente Enterococcus faecium-Stämme.

**Tests zur antibakteriellen Wirkung auf ATCC klassifizierte Stämme**

Zunächst wurde die antibakterielle Zusammensetzung auf Stämmen, die nach dem ATCC-Code klassifiziert sind, getestet. Die Resultate der Untersuchung sind in Abbildung 10a bis 10f zusammengefasst.

Die Konzentrationsstufen der Testzusammensetzung wurden in 12 Stufen angesetzt und sind der obigen Tabelle 5 zu entnehmen.

Die Diagramme der Abbildungen 10a bis 10f zeigen die Logarithmen des Extinktionszuwachses in % bei den zwölf Konzentrationen der hemmenden Zusammensetzung nach der Inkubation. Nach dem Lambert-Beerschen-Gesetz sind diese Größen proportional zum Zuwachs der Bakterienkonzentration. Die Teststämme gemäß den Diagrammen von Abbildung 10a bis 10f waren:

Abb.: 10a Staphylococcus aureus
Abb.: 10b Streptococcus pyogenes
Abb.: 10c Enterococcus faecalis

Abb.: 10d Klebsiella pneumoniae
Abb.: 10e Staphylococcus epidermidis
Abb.: 10f Escherichia coli

Die Diagramme der Abbildungen 10a bis 10f belegen, dass die antibakterielle Zusammensetzung aus dem Extrakt der Sophora japonica eine hemmende Wirkung auf alle in dieser Reihe getesteten Stämme ausübt. In den Diagrammen geben die Balken den Logarithmus des Extinktionszuwachses an, der nach dem Lambert-Beerschen Gesetz proportional zum Bakterienwachstum ist. Es ist deutlich, dass bei den höheren Konzentrationen der Zusammensetzung des Sophora japonica kein Extinktionsanstieg vorliegt, und damit wird belegt, dass kein Bakterienwachstum stattfindet. Bei weiterer Verdünnung sinkt die Konzentration des antibakteriell wirksamen Sophora japonica-Extraktes, und das bakterielle Wachstum beginnt proportional zu steigen.

Für die Analyse der Wirksamkeit wurde anhand der Extinktionsmessungen die Konzentration der Hemmsubstanz bestimmt, bei der nach der Inkubation nur die Hälfte der maximalen Bakterienanzahl beobachtet wurde. Aus den Diagrammen der Abbildung 10 a bis f wurden die Breakpoints bestimmt.

Diese Breakpoints (BP) sind für die untersuchten Stämme in der nachfolgenden Tabelle 6 zusammengefasst.

Tabelle 6: Breakpoints (BP) für die ATCC-klassifizierten Stämme

| Stamm: | Staph. aureus | Streptococcus py. | Enterococcus faec. |
|---|---|---|---|
| BP | 10 mg/ml | 2,5 mg/ml | 5 mg/ml |
| Stamm: | Klebsiella pneu. | Staph. epidermidis | E. coli |
| BP | 5 mg/ml | 10 mg/ml | 10 mg/ml |

**Tests zur antibakteriellen Wirkung auf multiresistente Stämme**

Die wie bereits beschrieben hergestellte Stammlösung wurde an Bakterienstämmen getestet, die Resistenzen gegen Penicilline und Cephalosporine haben. Die Resultate der Untersuchung sind in den Diagrammen der Abbildungen 11a bis 11c zusammengefasst.

Die Konzentrationsstufen der Testzusammensetzung wurden in 12 Stufen angesetzt und sind der Tabelle 5 zu entnehmen.

Die Diagramme der Abbildungen 11a bis 11c zeigen die Logarithmen des Extinktionszuwachses nach der Inkubation für diverse resistente Stämme. Nach dem Lambert-Beerschen-Gesetz sind diese Größen proportional zum Zuwachs der Bakterienkonzentration. Es wurden die folgenden multiresistenten Stämme, wie in den Diagrammen von Abbildung 11a bis 11c angegeben, untersucht:

Abb.: 11a Clindamycin-resistente Staphylokokken
Abb.: 11b Clindamycin-sensitive Staphylokokken
Abb.: 11c Vancomycin-resistenter Enterococcus faecium

Die Diagramme aus den Abbildungen 11a bis c belegen, dass die antibakterielle Zusammensetzung aus dem Extrakt der Sophora japonica eine deutliche Hemmung an multiresistenten Stämmen bewirkt. Vor allem der medizinisch relevante Methicillin-resistente Staphylococcus aureus (MRSA) sowie Vancomycin resistente Enterokokken wurden von der Zusammensetzung gehemmt. Es wurden aber auch zwei Stämme mit einer besonderen Resistenz verglichen: ein Clindamycin-sensitiver Stamm und ein Clindamycin-resistenter Stamm. Beide wurden von der getesteten antibakteriellen Zusammensetzung aus dem Extrakt der Sophora japonica gehemmt.

Aus den Diagrammen der Abbildungen 11a bis 11c wurden ferner die Breakpoints bestimmt. Die ermittelten Breakpoints sind in der nachfolgenden Tabelle 7 zusammengestellt.

Tabelle 7: Breakpoints für Clindamycinresistente Stämme

| Stamm: | Clindamycin res. Staph. aureus | Clindamycin sens. Staph. aureus | VRE |
|---|---|---|---|
| BP | 20 mg/ml | 5 mg/ml | 2,5 mg/ml |

<u>Tests zur antibakteriellen Wirkung nach Durchführung des Aktivierungsverfahrens</u>

Der oben bereits beschriebene wasserunlösliche Niederschlag, der beim unbehandelten Extrakt der Sophora

japonica gewonnen wird, enthält ein oder mehrere Flavonoide. Dieser Niederschlag wurde dem oben beschriebenen Aktivierungsverfahren unterzogen, hierdurch in das/die Hydrochlorid/e umgewandelt und dessen antibakterielle Wirkung getestet. Im Einzelnen wurde hierfür wie folgt vorgegangen:
Der erhaltene unbehandelte Extrakt wurde suspendiert und teilweise in Wasser gelöst, dann filtriert und das gewonnene Filtrat, d.h. der Niederschlag, isoliert und getrocknet. Es wurde 0,5 ml 25%-ige HCI-Lösung pro Gramm Filtrat zugegeben. Nach 1 min. Verrühren färbte sich die Lösung gelb. Die Lösung wurde 20 min. auf 50°C unter Rühren erwärmt. Dann wurden 10 ml $H_2O$ zugegeben und weiter bei 50°C für 10 min. gerührt, dann wurde der pH-Wert mit Natriumhydrogencarbonat auf pH = 7 eingestellt. Die Flüssigkeit wurde verdampft, bis sich dunkelgelbe Kristalle bildeten. Das so gewonnene Hydrochlorid war sehr gut wasserlöslich. Die wässerige Lösung des Hydrochlorids wurde auf ihre antibakterielle Aktivität getestet. Es wurde ein Staphylococcus aureus mit der ATCC Nummer 29213 verwendet.

Die für das Hydrochlorid erhaltenen Ergebnisse sind in Abbildung 12 dargestellt. Die nachfolgende Tabelle 8 gibt die Konzentrationsstufen des in Wasser gelösten Hydrochlorids mit den eingesetzten 6 Verdünnungsstufen an.

Tabelle 8: Konzentrationsstufen des in Wasser gelösten Hydrochlorids für die 6 eingesetzten Verdünnungsstufen

| Verdünnungsstufe | 1 | 2 | 3 | 4 | 5 | 6 |
|---|---|---|---|---|---|---|
| [mg/ml] | 6 | 3 | 1,5 | 0,75 | 0,4 | 0,2 |

Es wurde die antibakterielle Wirkung des Extrakts vor und nach Hydrochlorid-Bildung untersucht. Hierzu wurde die antibakterielle Zusammensetzung in Form des Extrakts von Sophora japonica, wie oben beschrieben, in Wasser gelöst und der in Wasser unlösliche Niederschlag abgetrennt. Weiterhin wurde der Niederschlag zum Hydrochlorid umgewandelt und das Hydrochlorid wieder zum löslichen Anteil des kristallinen Feststoffs in Wasser zugegeben. Es resultierte eine aktivierte Extraktlösung. Die Ergebnisse der Tests sind in den Abbildungen 13a und b dargestellt.

In Abbildung 13a ist die antibakterielle Wirkung des unbehandelten Extrakts dargestellt, und in Abbildung 13b ist die antibakterielle Wirkung des aktivierten Extrakts (Hydrochlorid-Bildung des Niederschlags) gezeigt. Die Breakpoints des aktivierten Extrakts sind um einen Faktor von etwa 8 bis 10 verschoben. Für den Staphylococcus aureus bedeutet dies eine hemmende Konzentration von 1 mg/ml des aktivierten Sophora japonica-Extrakts, d.h. die antibakterielle Wirkung des aktivierten Extrakts wurde deutlich verstärkt und ist vergleichbar mit der Wirkung eines potenten oder sehr potenten Antibiotikums.

**Tests zur Synergiewirkung der einzelnen Komponenten des Extrakts**

Es wurden verschiedene Tests durchgeführt, um zu bestimmen, ob die Komponenten der antibakteriellen Zusammensetzung im Extrakt eine Synergiewirkung entfalten. Bei sämtlichen Tests wurde Staphylococcus aureus mit der ATCC-Nummer 29213 verwendet. Es wurde wie folgt vorgegangen:

para-Hydroxybenzoesäure-Ester (PHB-Ester)

Zunächst wurde die Wirkung des para-Hydroxybenzoesäure-Esters (PHB-Ethylesters) allein auf Staphylococcus aureus (ATCC-Nummer 29213) untersucht. Die Ergebnisse der Wachstumshemmung sind in Abbildung 14 dargestellt.

Die Konzentrationsstufen des PHB-Esters für die 6 eingesetzten Verdünnungsstufen gehen aus der nachfolgenden Tabelle 9 hervor.

Tabelle 9: Konzentrationsstufen des PHB-Esters für die 6 eingesetzten Verdünnungsstufen

| Verdünnungsstufen | 1 | 2 | 3 | 4 | 5 | 6 |
|---|---|---|---|---|---|---|
| [mg/ml] | 128,0 | 64,0 | 32,0 | 16,0 | 8,0 | 4,0 |

PHB-Ester und Rutin

Die hemmende Wirkung des para-Hydroxybenzoesäure-Esters (PHB-Esters) als Reinsubstanz wurde mit der hemmenden Wirkung des Gemischs aus PHB-Ester und Rutin verglichen. Das Rutin allein hat keine hemmende Wirkung. Die Ergebnisse sind in Abbildung 15a dargestellt.

PHB-Ester und Sophora japonica-Extrakt

Weiterhin wurden verschiedene Mengen des PHB-Esters zusätzlich zum Sophora japonica-Extrakt zugegeben und die Wirksamkeit mit dem reinen Extrakt (ohne zusätzliche Zugabe von PHB-Ester) verglichen. Die Wirkung von Extrakt/PHB-Ester mit unterschiedlichen Verdünnungsstufen wurde mit der Wirkung des reinen Extrakts in Abbildung 15b verglichen. Die Ergebnisse von Abbildung 15b zeigen, dass durch die Zugabe des PHB-Esters eine verstärkte hemmende Wirkung des Extrakts erreicht wird.

PHB-Ester und Quercetin

Die Wirkung des Quercetin als Reinsubstanz wurde mit der Wirkung des Gemischs aus PHB-Ester/Quercetin verglichen. Die Ergebnisse sind in den Abbildungen 16a und 16b dargestellt. Das Quercetin wurde als Hydrochlorid-Salz in wässerige Lösung gebracht.

Die nachfolgende Tabelle 10 gibt die Konzentrationsstufen des Quercetin und des PHB-Esters für die 6 eingesetzten Verdünnungsstufen an.

Tabelle 10: Konzentrationsstufen des Quercetin und des PHB-Esters für die 6 eingesetzten Verdünnungsstufen

| Verdünnungsstufen | 1 | 2 | 3 | 4 | 5 | 6 |
|---|---|---|---|---|---|---|
| PHB [mg/ml] | 32,0 | 16,0 | 8,0 | 4,0 | 2,0 | 1,0 |
| Quercetin in Fig. 16a [mg/ml] | 1,2 | 0,6 | 0,3 | 0,15 | 0,08 | 0,04 |
| Quercetin in Fig. 16b [mg/ml] | 0,6 | 0,3 | 0,16 | 0,08 | 0,04 | 0,02 |

PHB-Ester und Kaempferol

Die Wirkung des Kaempferol als Reinsubstanz wurde mit der Wirkung des Gemischs aus PHB-Ester und Kaempferol verglichen. Die Ergebnisse sind in Abbildung 17a und b dargestellt.

Die nachfolgende Tabelle 11 gibt die Konzentrationsstufen des Kaempferol und des PHB-Esters für die 6 eingesetzten Verdünnungsstufen an.

Tabelle 11: Konzentrationsstufen des Kaempferol und des PHB-Esters für die 6 eingesetzten Verdünnungsstufen

| Verdünnungsstufen | 1 | 2 | 3 | 4 | 5 | 6 |
|---|---|---|---|---|---|---|
| PHB [mg/ml] | 32,0 | 16,0 | 8,0 | 4,0 | 2,0 | 1,0 |
| Kaempferol in Abb.: 17a [mg/ml] | 0,2 | 0,1 | 0,05 | 0,03 | 0,015 | 0,007 |
| Kaempferol in Abb.: 17b [mg/ml] | 0,13 | 0,06 | 0,03 | 0,016 | 0,008 | 0,004 |

PHB-Ester und Flavon

Die Wirkung des Flavon als Reinsubstanz wurde mit der Wirkung des Gemischs aus PHB-Ester und Flavon verglichen. Die Ergebnisse sind in Abbildung 18a und Abbildung 18b dargestellt.

Die nachfolgende Tabelle 12 gibt die Konzentrationsstufen des Flavons und des PHB-Esters für die 6 eingesetzten Verdünnungsstufen an.

Tabelle 12: Konzentrationsstufen des Flavon und des PHB-Esters für die 6 eingesetzten Verdünnungsstufen

| Verdünnungsstufen | 1 | 2 | 3 | 4 | 5 | 6 |
|---|---|---|---|---|---|---|
| PHB [mg/ml] | 32,0 | 16,0 | 8,0 | 4,0 | 2,0 | 1,0 |
| Flavon in Abb.: 18a [mg/ml] | 7,5 | 4,0 | 2,0 | 1,0 | 0,5 | 0,25 |
| Flavon in Abb.: 18b [mg/ml] | 4,0 | 2,0 | 1,0 | 0,5 | 0,25 | 0,13 |

PHB-Ester und Flavonoid-Mischung

Die Wirkung der Flavonoid-Mischung aus Rutin, Kaempferol und Quercetin, jeweils als Reinsubstanz enthalten,

wurde mit der Wirkung des Gemischs aus PHB-Ester und Flavonoid-Mischung (Rutin, Kaempferol und Quercetin) verglichen. Die Ergebnisse sind in Abbildung 19a und b dargestellt.

Die nachfolgende Tabelle 13 gibt die Konzentrationsstufen der getesteten Flavonoid-Mischung und des PHB-Esters für die 6 eingesetzten Verdünnungsstufen an.

Tabelle 13: Konzentrationsstufen der getesteten Flavonoid-Mischung und des PHB-Esters für die 6 eingesetzten Verdünnungsstufen

| Verdünnungsstufen | 1 | 2 | 3 | 4 | 5 | 6 |
|---|---|---|---|---|---|---|
| PHB [mg/ml] | 32,0 | 16,0 | 8,0 | 4,0 | 2,0 | 1,0 |
| Rutin in Abb.: 19a [mg/ml] | 0,07 | 0,04 | 0,017 | 0,008 | 0,004 | 0,002 |
| Rutin in Abb.: 19b [mg/ml] | 0,05 | 0,03 | 0,013 | 0,006 | 0,003 | 0,0016 |
| Kaempferol in Abb.: 19a [mg/ml] | 0,08 | 0,04 | 0,02 | 0,01 | 0,005 | 0,003 |
| Kaempferol in Abb.: 19b [mg/ml] | 0,06 | 0,03 | 0,016 | 0,008 | 0,004 | 0,002 |
| Quercetin in Abb.: 19a [mg/ml] | 0,2 | 0,1 | 0,05 | 0,03 | 0,015 | 0,007 |
| Quercetin in Abb.: 19b [mg/ml] | 0,16 | 0,08 | 0,04 | 0,02 | 0,01 | 0,005 |

Hydroxymethylfurfural und Flavonoid-Mischung

Die antibakterielle Wirkung des Hydroxymethylfurfurals (HMF) als Reinsubstanz wurde mit der Wirkung des Gemischs aus HMF und dem Flavonoid (Quercetin) verglichen. Die Ergebnisse sind in Abbildung 20a und b dargestellt und in Tabelle 14 zusammengefasst.

Tabelle 14: Konzentrationsstufen des Hydroxymethylfurfurals (HMF) und des Quercetin für die 6 relevanten Verdünnungsstufen

| Verdünnungsstufen | 1 | 2 | 3 | 4 | 5 |
|---|---|---|---|---|---|
| Quercetin in Abb.: 20b [mg/ml] | 3,8 | 1,9 | 0,9 | 0,4 | 0,2 |
| HMF in Abb.: 20a [mg/ml] | 10,0 | 5,0 | 2,5 | 1,3 | 0,6 |
| HMF in Abb.: 20b [mg/ml] | 5,0 | 2,5 | 1,3 | 0,6 | 0,3 |

Schnelltest für die Gehaltsbestimmung des gewonnenen Extrakts anhand der Dünnschichtchromatographie:

Um den Gehalt an aktiven Substanzen einer Charge des Extrakts schnell und zuverlässig zu analysieren, wurde die Dünnschichtchromatographie verwendet. Hierzu wird beispielsweise wie folgt vorgegangen:
Als mobile Phase werden

1) ein Chloroform-/Methanol-Gemisch (0,95 : 0,05),
2) ein Ethylacetat-/$H_2O$-/Ameisensäure-Gemisch (8 : 1 : 1)

verwendet.

Als stationäre Phase wurde Kieselgur auf einer 1,5 mm dicken Glasplatte eingesetzt.

Es konnten zwei Kriterien identifiziert werden, um die gewünschte Qualität des Extrakts zu bestätigen:

DC:

Laufmittel 1: Ein Punkt bei etwa 65% der Laufstrecke mit etwa dem gleichen Durchmesser wie der aufgetragene

Punkt. Dieser Punkt bestand aus den enthaltenen Parabenen.

Laufmittel 2: Ein Punkt bei etwa 90% der Laufstrecke mit etwa dem gleichen Durchmesser wie der aufgetragene Punkt. Dieser Punkt bestand aus den enthaltenen hochmolekularen Verbindungen.

**In vivo-Testmethode**

Ein Hund mit einer etwa 3 x 2 cm großen bakteriell besiedelten Ulzeration (offene Hautläsion) wurde mit der in dieser Patentschrift beschriebenen Salbe behandelt, die den Extrakt aus Sophora japonica enthielt. Dabei wurde die Salbe 10 Tage lang täglich mit einem sterilen Tupfer auf die Wunde aufgetragen. Die Veränderungen an der Wunde wurden täglich evaluiert. Diese orientierende Behandlung zeigte eine deutlich schnellere Wundheilung als dies unbehandelt bei einer Hauteffloreszenz dieser Größe bei dem Tier hätte erwarten lassen.

Weitere Versuche wurden mittlerweile bei unterschiedlich großen Ulzerationen anderer Hunde unterschiedlicher Rasse versuchsweise getestet. Die Ergebnisse des Vorversuches ließen sich bestätigen.

**Patentansprüche**

1. Antibakterielle Zusammensetzung, umfassend oder bestehend aus einem Extrakt aus den Früchten von Sophora Japonica;
wobei der Extrakt erhältlich ist aus
einem ersten Extraktionsverfahren der Fruchtstücke von Sophora Japonica, wobei als Extraktionsmittel ein Gemisch aus Alkohol und Wasser, bevorzugt Ethanol und Wasser, eingesetzt wird, und
einem zweiten Extraktionsverfahren, das mit Ethylacetat als Extraktionsmittel durchgeführt wird,
wobei der nach der ersten Extraktion gewonnene Extrakt isoliert und dann die zweite Extraktion durchgeführt wird, oder sich nach der ersten Extraktion,
ohne Isolieren des Extrakts, die zweite Extraktion anschließt,
und der erhaltene Extrakt einer chemischen Modifikation in Form eines Aktivierungsverfahrens unterzogen wurde, erhältlich durch Zugeben von verdünnter oder konzentrierter Salzsäure,
Erwärmen auf eine Temperatur zwischen 40 und 60°C,
anschließendes Zugeben von Wasser,
erneutes und fortgesetztes Erwärmen des erhaltenen Gemisches und Einstellen eines neutralen pH-Werts
unter Erhalt eines aktivierten Extrakts.

2. Antibakterielle Zusammensetzung nach Anspruch 1,
**dadurch gekennzeichnet,**
**dass** diese eine deutlich gesteigerte antibakterielle Wirksamkeit gegen Krankheitserreger gegenüber einem nicht-aktivierten Extrakt, insbesondere multiresistente Krankheitserreger, aufweist,
wobei die Krankheitserreger bevorzugt klassifizierte (ATCC) Bakterienstämme sind, wie Staphylococcus aureus, Staphylococcus epidermidis, Streptococcus pyogenes, Enterococcus faecalis, Klebsiella pneumoniae, Enterobacter spp., Pseudomonas aeruginosa oder Escherichia coli, oder multiresistente Bakterienstämme, wie MRSA, Vancomycin-resistente Enterococcus faecalis (VRE) sind.

3. Antibakterielle Zusammensetzung nach Anspruch 1 oder 2,
**dadurch gekennzeichnet,**
**dass** der aktivierte Extrakt in konzentrierter Form vorliegt.

4. Antibakterielle Zusammensetzung nach mindestens einem der vorangehenden Ansprüche 1 bis 3,
**dadurch gekennzeichnet,**
**dass** in dem Extrakt eine zusätzliche Menge an para-Hydroxybenzoesäure-Ester (PHB-Ester), bevorzugt para-Hydroxybenzoesäure-Ethylester oder para-Hydroxybenzoesäure-Propylester, vorhanden ist, wobei die Konzentration des zugesetzten PHB-Esters bevorzugt im Bereich von 0,01 g/ml bis 1 g/ml bevorzugter von 0,05 g/ml bis 0,5 g/ml, ganz besonders bevorzugt von 0,1 g/ml bis 0,2 g/ml liegt.

5. Verfahren zur Gewinnung des Extrakts nach einem der Ansprüche 1 bis 4, der einer chemischen Modifikation in Form eines Aktivierungsverfahrens unterzogen wurde, umfassend die folgenden Schritte:

    - Bereitstellen von trockenen Früchten der Sophora japonica;

- Zerkleinern der getrockneten Früchte zu Fruchtstücken;
- Extrahieren der erhaltenen Fruchtstücke mit einer fest-flüssig Extraktion, bevorzugt in einem Soxhlet-Extraktor bei einer Temperatur oberhalb von 80°C, wobei ein Gemisch aus Alkohol und Wasser, bevorzugt Ethanol und Wasser, als Extraktionsmittel verwendet wird;
- gegebenenfalls mehrfaches Durchführen der Extraktion, um die Ausbeute zu erhöhen;
- Verdampfen des Extraktionsmittels unter Erhalt des Extrakts;
- Bereitstellen einer wässerigen Lösung/Dispersion des erhaltenen Extrakts;
- Durchführen einer flüssig-flüssig Extraktion mit der wässerigen Lösung/Dispersion des Extrakts, wobei Ethylacetat als Extraktionsmittel verwendet wird;
- gegebenenfalls mehrfaches Durchführen der Extraktion, um die Ausbeute zu erhöhen; und
- Verdampfen des Extraktionsmittels unter Erhalt eines konzentrierten Extrakts;
- Durchführen eines Aktivierungsverfahrens des erhaltenen konzentrierten Extrakts mit den folgenden Schritten:
- Zugeben von konzentrierter oder verdünnter Salzsäure (HCl) zum Extrakt;
- Erwärmen des erhaltenen Gemisches unter Rühren auf eine Temperatur zwischen 40 und 60°C;
- Zugeben von Wasser zu dem Gemisch;
- erneutes oder fortgesetztes Erwärmen des erhaltenen Gemisches;
- optional Zugeben eines Salzes oder hydrogenen Salzes aus einer schwachen Säure und einem Alkali- oder Erdalkalimetall zu dem Gemisch und erneutes oder fortgesetztes Erwärmen des erhaltenen Gemisches; und
- Einstellen eines neutralen pH-Wertes unter Erhalt eines aktivierten Extrakts.

**6.** Verfahren nach Anspruch 5,
**dadurch gekennzeichnet, dass**
als Extraktionsmittel ein Gemisch aus Ethanol und Wasser eingesetzt wird, wobei die Menge an Ethanol im Bereich von 30 bis 50%, besonders bevorzugt 35 bis 45%, insbesondere 40%, des Volumens der eingesetzten Fruchtstücke eingestellt wird.

**7.** Pharmazeutische Zusammensetzung, umfassend die antibakterielle Zusammensetzung nach mindestens einem der vorangehenden Ansprüche 1 bis 4, mit einem oder mehreren pharmazeutisch akzeptablen Trägern und/oder Hilfsstoffen.

**8.** Pharmazeutische Zusammensetzung nach Anspruch 7 zur Verwendung im therapeutischen Bereich, bevorzugt zur topischen und/oder systemischen Behandlung, insbesondere in der Behandlung von Haut- oder Schleimhauterkrankungen, bevorzugt Infektionen oder Entzündungen der Haut oder Schleimhaut, oder in der Behandlung oder Vorbeugung von Verletzungen der Haut oder Schleimhaut, insbesondere bei (Brand-)Wunden.

**9.** Antibakterielle Zusammensetzung nach einem der Ansprüche 1 bis 4 oder pharmazeutische Zusammensetzung nach Anspruch 7 zur Verwendung als Desinfektionsmittel, insbesondere gegen Staphylococcus aureus, Streptococcus pyogenes, Enterococcus faecium, Staphylococcus epidermidis, Escherichia coli.

**10.** Pharmazeutische Zusammensetzung nach Anspruch 7 zur Verwendung zur dermalen, transdermalen, (intra-)nasalen oder (per-)oralen Verabreichung.

**11.** Salbe, umfassend oder bestehend aus der antibakteriellen Zusammensetzung nach einem der Ansprüche 1 bis 4, in Wasser und einem Trägermedium, wobei die antibakterielle Zusammensetzung in Wasser: Trägermedium bevorzugt im Verhältnis 1 : 2, bezogen auf das Gewicht, vorliegt.

## Claims

**1.** Antibacterial composition comprising or consisting of an extract from the fruits of Sophora Japonica;
wherein the extract is obtainable from
a first extraction method of the fruit pieces of Sophora Japonica,
wherein a mixture of alcohol and water, preferably ethanol and water, is used as an extraction means, and
a second extraction method which is performed with ethyl acetate as extraction means,
wherein the extract obtained after the first extraction is isolated and then the second extraction is performed, or the second extraction follows the first extraction, without an isolation of the extract,
and the obtained extract is subject to a chemical modification in the form of an activation method, which can be obtained by

adding diluted or concentrated hydrochloric acid,
heating to a temperature of between 40 and 60°C,
subsequently adding water,
again and continuously heating the obtained mixture and
adjusting a neutral pH value
while obtaining an activated extract.

**2.** Antibacterial composition according to claim 1,
**characterized in that**
it has a significantly increased antibacterial activity against pathogens, in particular multi-resistant pathogens, compared to a not activated extract, wherein the pathogens are preferably classified (ATCC) bacterial strains, such as Staphylococcus aureus, Staphylococcus epidermidis, Streptococcus pyogenes, Enterococcus faeconia, Klebsiella pneumoniae, Enterobacter spp., Pseudomonas aeruginosa or Escherichia coli, or multi-resistant bacterial strains such as MRSA, vancomycin-resistant Enterococcus faecalis (VRE).

**3.** Antibacterial composition according to claim 1 or 2,
**characterized in that**
the activated extract is in the form of a concentrated extract.

**4.** Antibacterial composition according to at least one of the preceding claims 1 to 3,
**characterized in that**
in the extract an additional amount of para-hydroxybenzoic acid ester (PHB ester), preferably para-hydroxybenzoic acid ethyl ester or para-hydroxybenzoic acid propyl ester, is present, wherein the concentration of the added PHB ester is preferably in the range from 0.01 g / ml to 1 g / ml, more preferably from 0.05 g / ml to 0.5 g / ml, very particularly preferably from 0.1 g / ml to 0.2 g / ml.

**5.** Method for obtaining the extract according to any one of claims 1 to 4, which has been subject to a chemical modification in the form of an activation method, comprising the following steps:

- providing dry fruits of Sophora japonica;
- crushing the dried fruit into fruit pieces;
- extracting the obtained fruit pieces with a solid-liquid extraction, preferably in a Soxhlet extractor at a temperature of above 80°C, wherein a mixture of alcohol and water, preferably ethanol and water, is used as extraction means;
- if necessary, performing the extraction several times in order to increase the yield;
- evaporating the extraction means while obtaining the extract;
- providing an aqueous solution / dispersion of the obtained extract;
- performing a liquid-liquid extraction with the aqueous solution / dispersion of the extract, wherein ethyl acetate is used as extraction means;
- if necessary, performing the extraction several times in order to increase the yield; and
- evaporating the extraction means while obtaining a concentrated extract;
- performing an activation method of the obtained concentrated extract with the following steps:

    - adding concentrated or diluted hydrochloric acid (HCl) to the extract;
    - heating the obtained mixture while stirring to a temperature of between 40 and 60°C;
    - adding water to the mixture;
    - again or continuously heating the obtained mixture;
    - optionally adding a salt or hydrogen salt of a weak acid and an alkali or alkaline earth metal to the mixture and again or continuously heating the obtained mixture; and
    - setting a neutral pH value while obtaining an activated extract.

**6.** Method according to claim 5,
**characterized in that**
a mixture of ethanol and water is used as extraction means, wherein the amount of ethanol is set in the range from 30 to 50%, particularly preferably 35 to 45%, in particular 40%, of the volume of the fruit pieces used.

**7.** Pharmaceutical composition comprising the antibacterial composition according to at least one of the preceding claims 1 to 4, with one or more pharmaceutically acceptable carriers and / or excipients.

8. Pharmaceutical composition according to claim 7 for use in the therapeutic field, preferably for topical and / or systemic treatment, in particular in the treatment of skin or mucous membrane diseases, preferably infections or inflammations of the skin or mucous membrane, or in the treatment or prevention of injuries to the skin or mucous membrane, in particular with (burn) wounds.

9. Antibacterial composition according to one of claims 1 to 4 or the pharmaceutical composition according to claim 7 for use as a disinfectant, in particular against Staphylococcus aureus, Streptococcus pyogenes, Enterococcus faecium, Staphylococcus epidermidis, Escherichia coli.

10. Pharmaceutical composition according to claim 7 for use for dermal, transdermal, (intra-) nasal or (per-) oral administration.

11. Ointment comprising or consisting of the antibacterial composition according to one of claims 1 to 4, in water and a carrier medium, wherein the antibacterial composition in water: carrier medium preferably is present in a ratio of 1 : 2 by weight.

**Revendications**

1. Composition antibactérienne, comprenant un ou constituée d'un extrait des fruits du Sophora Japonica ;
l'extrait étant susceptible d'être obtenu à partir
d'un premier procédé d'extraction des morceaux de fruits du Sophora Japonica, étant mis en œuvre en tant que produit d'extraction un mélange d'alcool et d'eau, de préférence d'éthanol et d'eau, et
d'un deuxième procédé d'extraction, qui est réalisé avec de l'acétate d'éthyle en tant que produit d'extraction,
l'extrait obtenu après la première extraction étant isolé, suite à quoi étant réalisée la deuxième extraction, ou la deuxième extraction se raccordant à la première extraction, sans isolation de l'extrait,
et l'extrait obtenu étant soumis à une modification chimique, sous la forme d'un procédé d'activation, susceptible d'être obtenu en
ajoutant de l'acide chlorhydrique dilué ou concentré,
faisant chauffer à une température comprise entre 40 et 60°C,
par la suite, en ajoutant de l'eau,
faisant chauffer une nouvelle fois et de manière poursuivie le mélange obtenu et
réglant une valeur pH neutre
pour obtenir un extrait activé.

2. Composition antibactérienne selon la revendication 1,
**caractérisée en ce qu'**
elle fait preuve d'un effet antibactérien nettement augmenté contre les agents pathogènes, notamment les agents pathogènes multi-résistants en comparaison d'un extrait non activé,
les agents pathogènes étant de préférence des souches bactériennes (ATCC) classifiées, comme le Staphylococcus aureus, le Staphylococcus epidermidis, le Streptococcus pyogenes, l'Enterococcus faecalis, le Klebsiella pneumoniae, l'Enterobacter spp., le Pseudomonas aeruginosa ou l'Escherichia coli, ou des souches bactériennes multirésistantes, comme le MRSA, l'Enterococcus faecalis (VRE) résistant à la vancomycine.

3. Composition antibactérienne selon la revendication 1 ou 2,
**caractérisée en ce que**
l'extrait activé se présente sous forme concentrée.

4. Composition antibactérienne selon au moins l'une quelconque des revendications précédentes 1 à 3,
**caractérisée en ce que**
dans l'extrait est présente une quantité additionnelle d'ester d'acide para-hydroxybenzoïque (ester PHB-), de préférence d'éthylester d'acide para-hydroxybenzoïque ou de propylester d'acide para-hydroxybenzoïque, la concentration de l'ester PHB ajouté se situant de préférence dans l'ordre de 0,01 g/ml à 1 g/ml, de manière plus préférentielle, de 0,05 g/ml à 0,5 g/ml, de manière particulièrement préférentielle, de 0,1 g/ml à 0,2 g/ml.

5. Procédé, destiné à obtenir l'extrait selon l'une quelconque des revendications 1 à 4, qui a été soumis à une modification chimique sous la forme d'un procédé d'activation, comprenant les étapes suivantes, consistant à :

- mettre à disposition des fruits secs du Sophora japonica ;
- broyer les fruits séchés en morceaux de fruits ;
- extraire les morceaux de fruits obtenus par extraction solide/liquide, de préférence dans un extracteur Soxhlet, à une température supérieure à 80°C, un mélange d'alcool et d'eau, de préférence d'éthanol et d'eau étant utilisé en tant que produit d'extraction ;
- le cas échéant, procéder plusieurs fois à l'extraction, pour augmenter le rendement ;
- faire évaporer le produit d'extraction, pour obtenir l'extrait ;
- mettre à disposition une solution/dispersion aqueuse de l'extrait obtenu ;
- procéder à une extraction liquide/liquide avec la solution/dispersion aqueuse de l'extrait, l'acétate d'éthyle étant utilisé en tant que produit d'extraction ;
- le cas échéant, procéder plusieurs fois à l'extraction, pour augmenter le rendement ; et
- faire évaporer le produit d'extraction, pour obtenir un extrait concentré ;
- réaliser un procédé d'activation de l'extrait concentré obtenu, présentant les étapes suivantes, consistant à :
- ajouter à l'extrait de l'acide chlorhydrique (HCl) concentré ou dilué ;
- faire chauffer en brassant le mélange obtenu à une température comprise entre 40 et 60°C ;
- ajouter de l'eau au mélange ;
- faire chauffer à nouveau ou de manière poursuivie le mélange obtenu ;
- ajouter en option au mélange un sel ou un sel hydrogène d'un acide faible et un métal alcalin ou alcalino-terreux et faire chauffer à nouveau ou de manière poursuivie le mélange obtenu ; et
- régler une valeur pH neutre, pour obtenir un extrait activé.

**6.** Procédé selon la revendication 5,
**caractérisée en ce qu'**
on met en œuvre en tant que produit d'extraction un mélange d'éthanol et d'eau, la quantité d'éthanol étant réglée dans l'ordre de 30 à 50 %, de manière particulièrement préférentielle, de 35 à 45%, notamment à 40%, du volume des morceaux de fruits mis en œuvre.

**7.** Composition pharmaceutique, comprenant la composition antibactérienne selon au moins l'une quelconque des revendications précédentes 1 à 4, avec un ou plusieurs véhicules et/ou excipients acceptables d'un point de vue pharmaceutique.

**8.** Composition pharmaceutique selon la revendication 7, destinée à être utilisée dans le domaine thérapeutique, de préférence pour le traitement topique et/ou systémique, notamment pour le traitement des affections de la peau ou des muqueuses, de préférence des infections ou des inflammations de la peau ou des muqueuses, ou dans le traitement ou la prévention de lésions de la peau ou des muqueuses, notamment dans le cas de plaies (par brûlure).

**9.** Composition antibactérienne selon l'une quelconque des revendications 1 à 4 ou composition pharmaceutique selon la revendication 7, destinée à être utilisée en tant que produit désinfectant, notamment contre le Staphylococcus aureus, le Streptococcus pyogenes, l'Enterococcus faecium, le Staphylococcus epidermidis, l'Escherichia coli.

**10.** Composition pharmaceutique selon la revendication 7, destinée à être administrée par voie cutanée, transcutanée, (intra)nasale ou (per)orale.

**11.** Pommade, comprenant la ou constituée de la composition antibactérienne selon l'une quelconque des revendications 1 à 4, dans de l'eau et un milieu véhiculeur, la composition antibactérienne se présentant dans l'eau : le milieu véhiculeur de préférence dans la proportion 1 : 2, en rapport au poids.

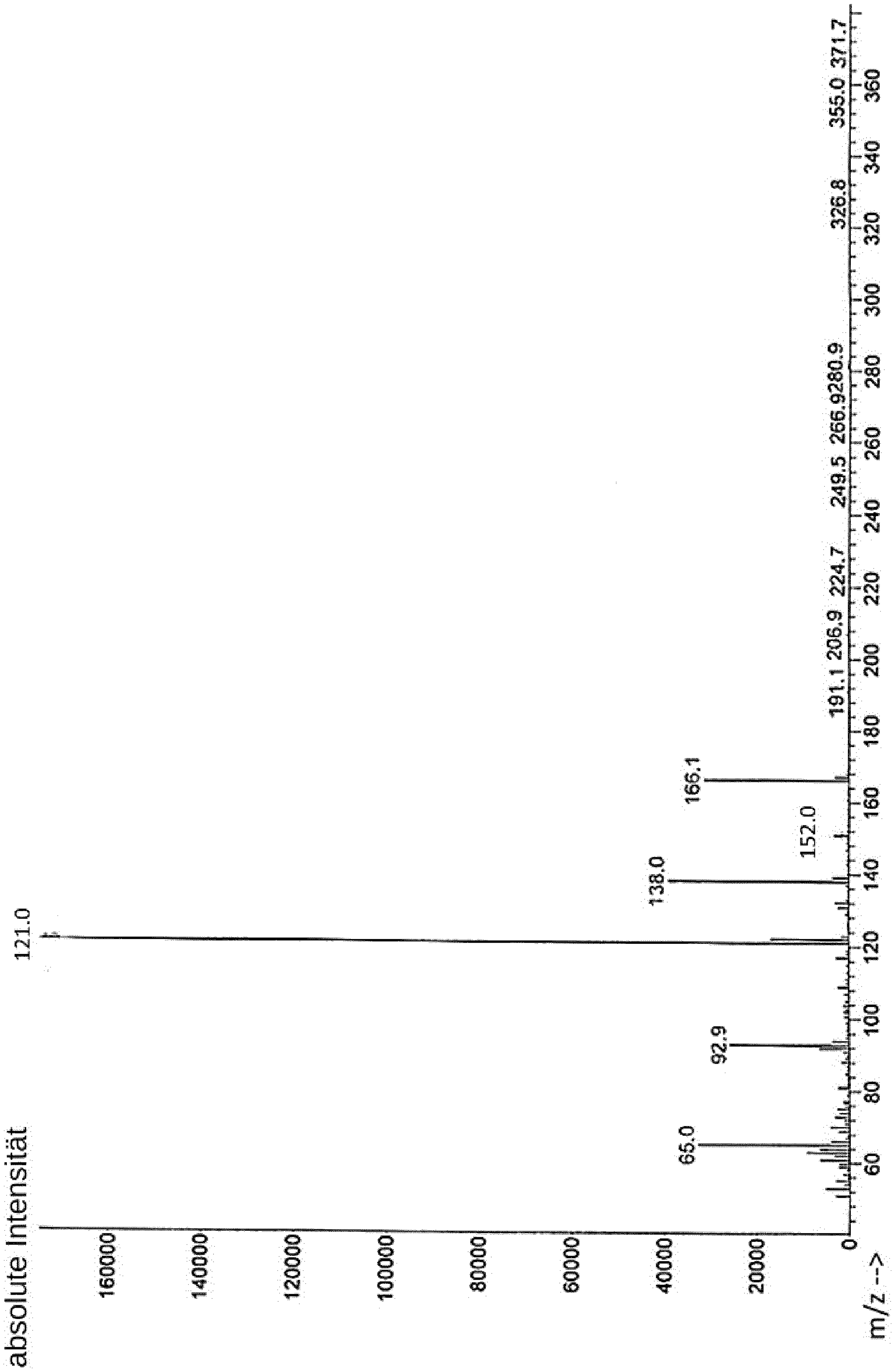
absolute Intensität
160000
140000
120000
100000
80000
60000
40000
20000
0
m/z -->
65.0
92.9
121.0
138.0
152.0
166.1
191.1
206.9
224.7
249.5
266.9280.9
326.8
355.0
371.7
60
80
100
120
140
160
180
200
220
240
260
280
300
320
340
360

Fig. 1a

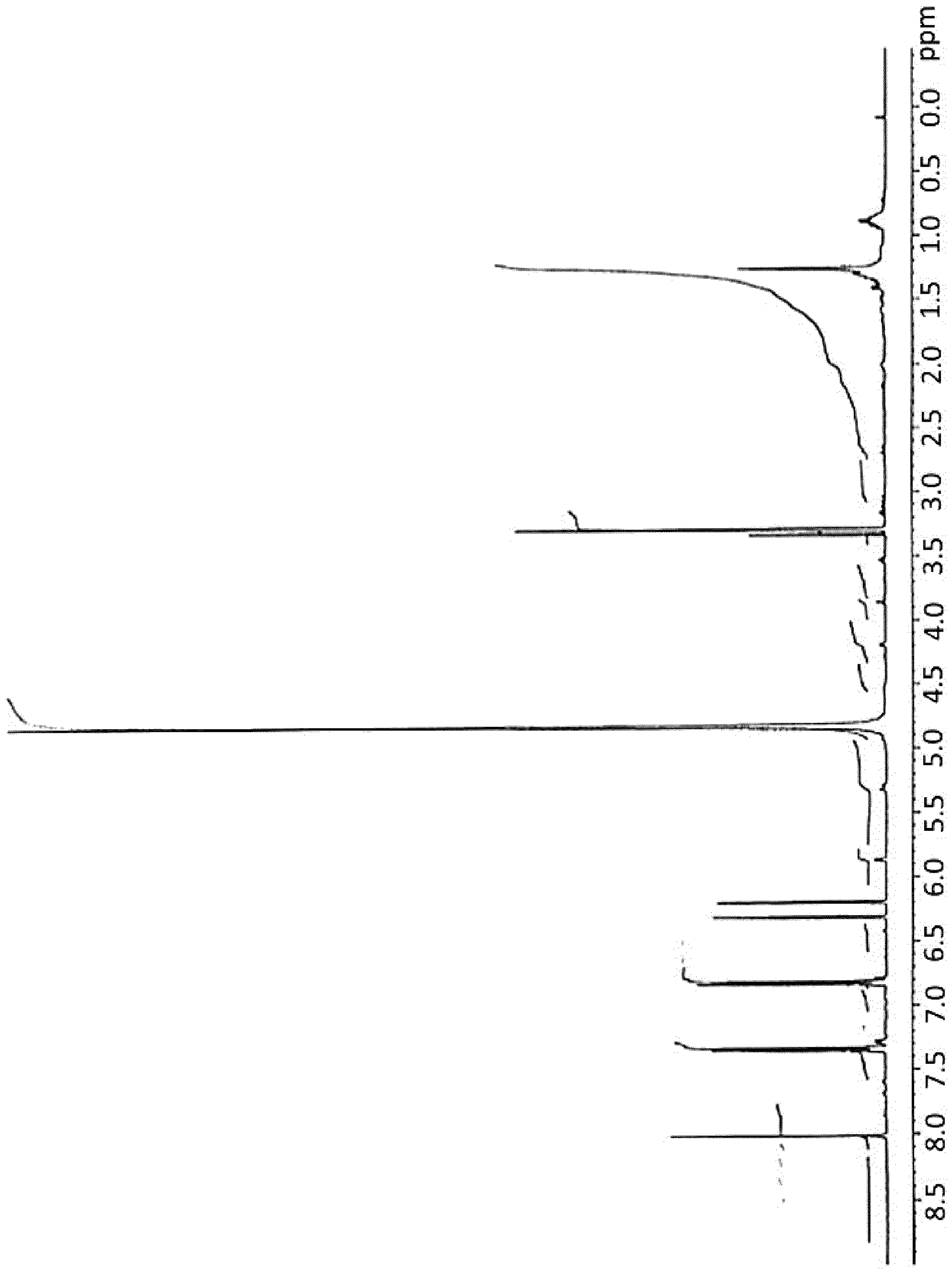


Fig. 1b

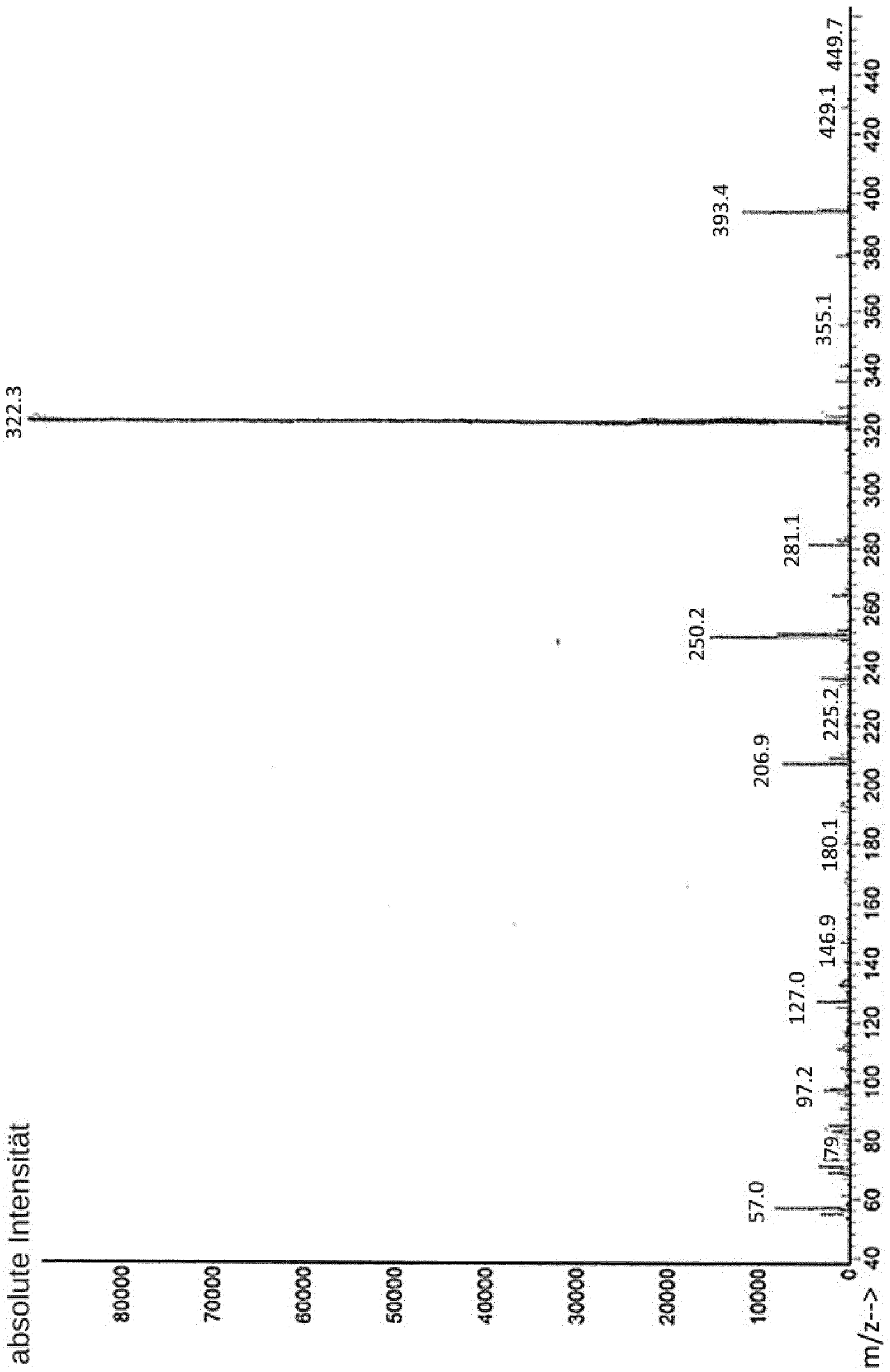
absolute Intensität
80000
70000
60000
50000
40000
30000
20000
10000
0
m/z-->
40
60
80
100
120
140
160
180
200
220
240
260
280
300
320
340
360
380
400
420
440
57.0
79
97.2
127.0
146.9
180.1
206.9
225.2
250.2
281.1
322.3
355.1
393.4
429.1
449.7

Fig. 2a

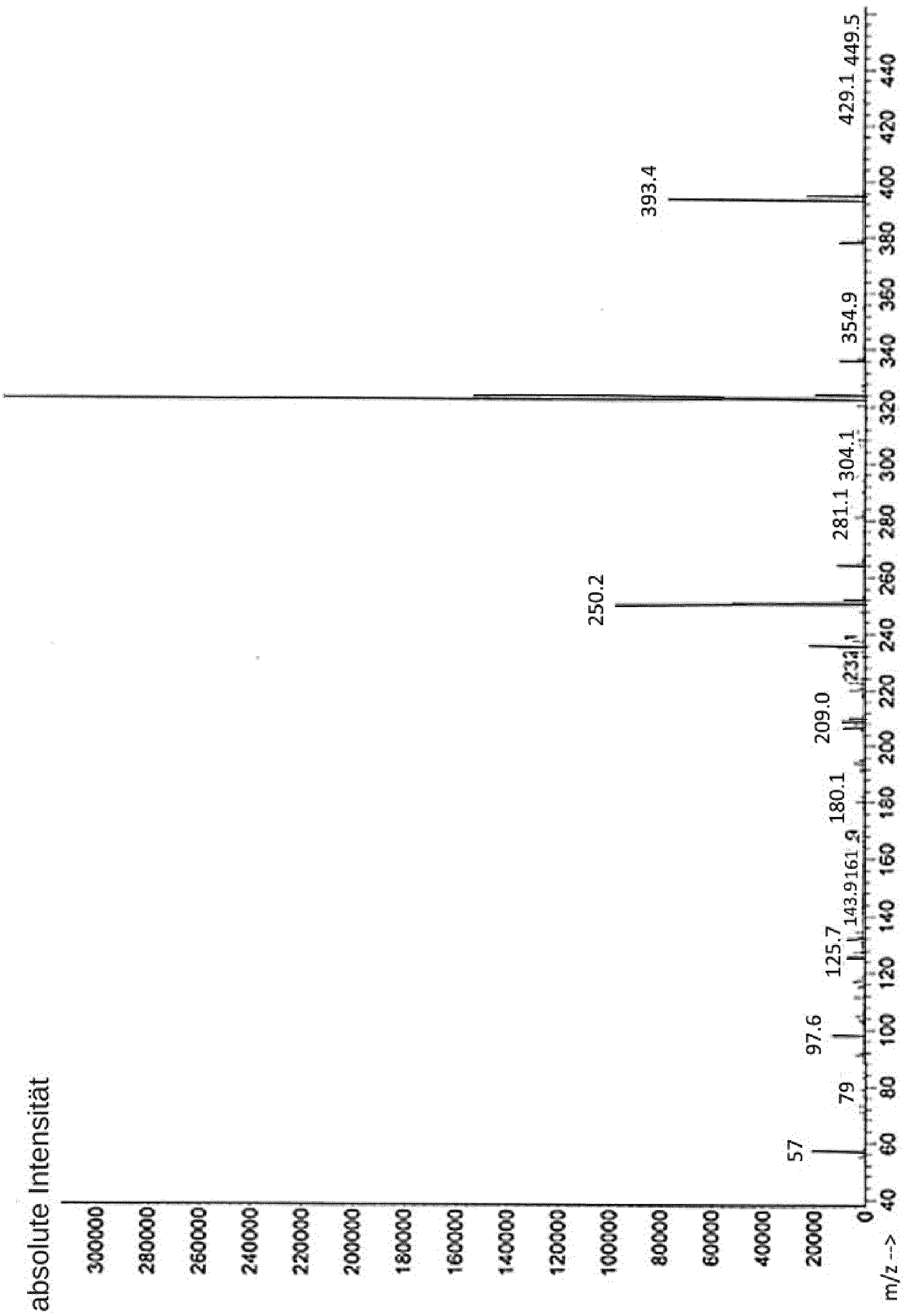
absolute Intensität
m/z -->
57
79
97.6
125.7
143.9
161.9
180.1
209.0
250.2
281.1
304.1
354.9
393.4
429.1
449.5

Fig. 2b

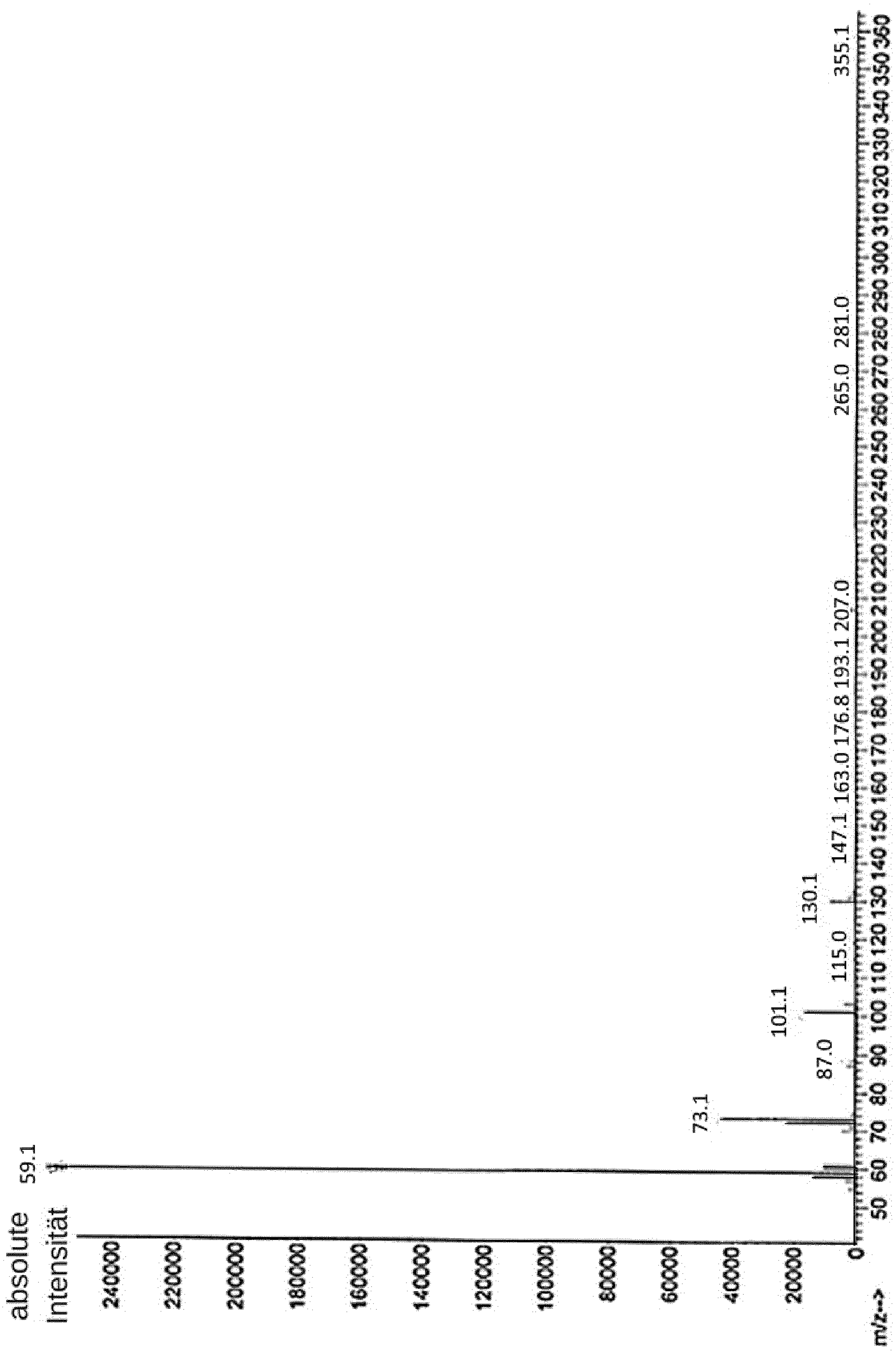
absolute Intensität
240000
220000
200000
180000
160000
140000
120000
100000
80000
60000
40000
20000
0
m/z-->
50 60 70 80 90 100 110 120 130 140 150 160 170 180 190 200 210 220 230 240 250 260 270 280 290 300 310 320 330 340 350 360
59.1
73.1
87.0
101.1
115.0
130.1
147.1
163.0
176.8
193.1
207.0
265.0
281.0
355.1

Fig. 3

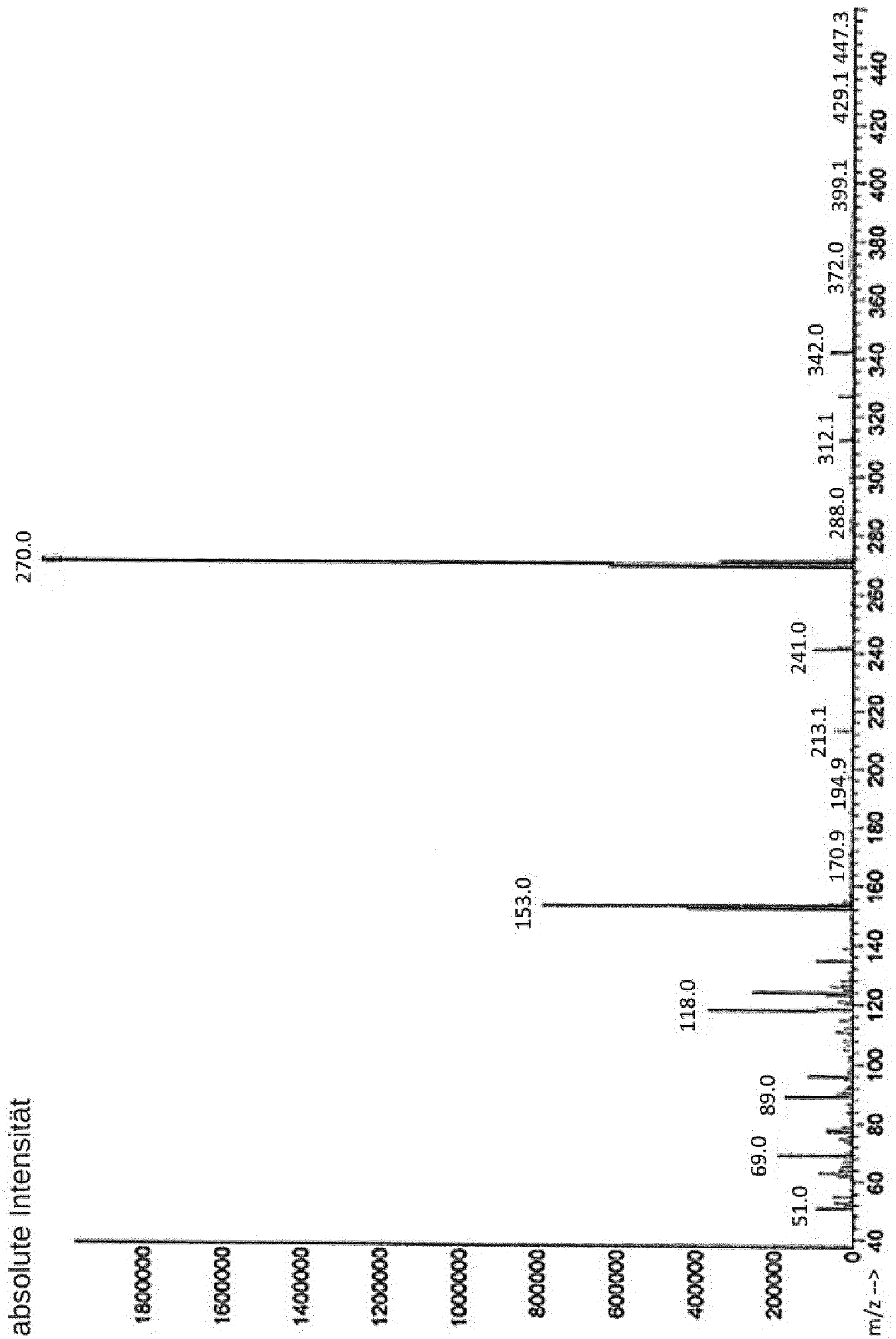
absolute Intensität
0
200000
400000
600000
800000
1000000
1200000
1400000
1600000
1800000
m/z -->
40
60
80
100
120
140
160
180
200
220
240
260
280
300
320
340
360
380
400
420
440
51.0
69.0
89.0
118.0
153.0
170.9
194.9
213.1
241.0
270.0
288.0
312.1
342.0
372.0
399.1
429.1
447.3

Fig. 4

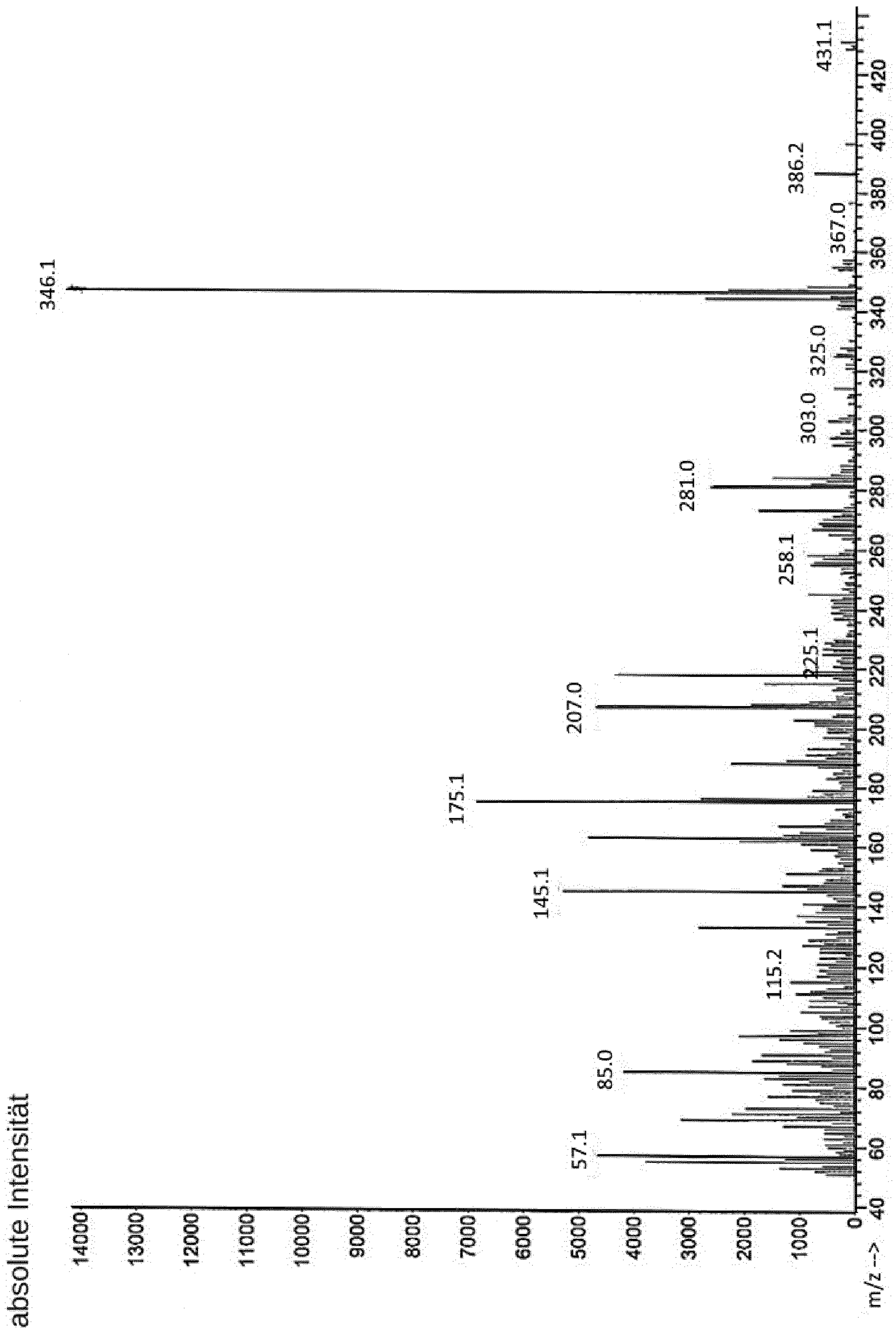
absolute Intensität
m/z -->
346.1
431.1
386.2
367.0
325.0
303.0
281.0
258.1
225.1
207.0
175.1
145.1
115.2
85.0
57.1

Fig. 5

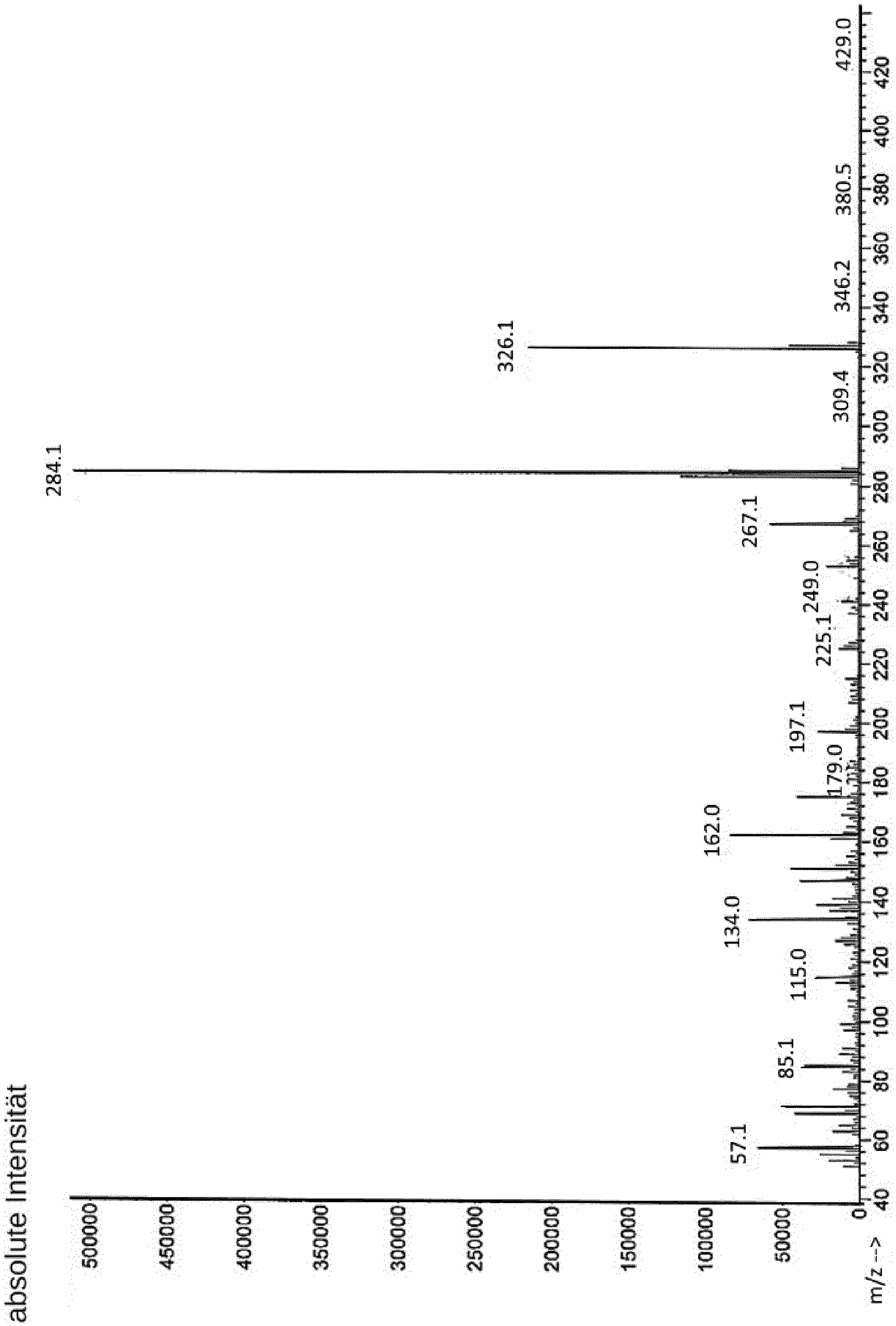
absolute Intensität
m/z -->
284.1
326.1
267.1
249.0
225.1
197.1
179.0
162.0
134.0
115.0
85.1
57.1
309.4
346.2
380.5
429.0
500000
450000
400000
350000
300000
250000
200000
150000
100000
50000
0
40
60
80
100
120
140
160
180
200
220
240
260
280
300
320
340
360
380
400
420

Fig.6

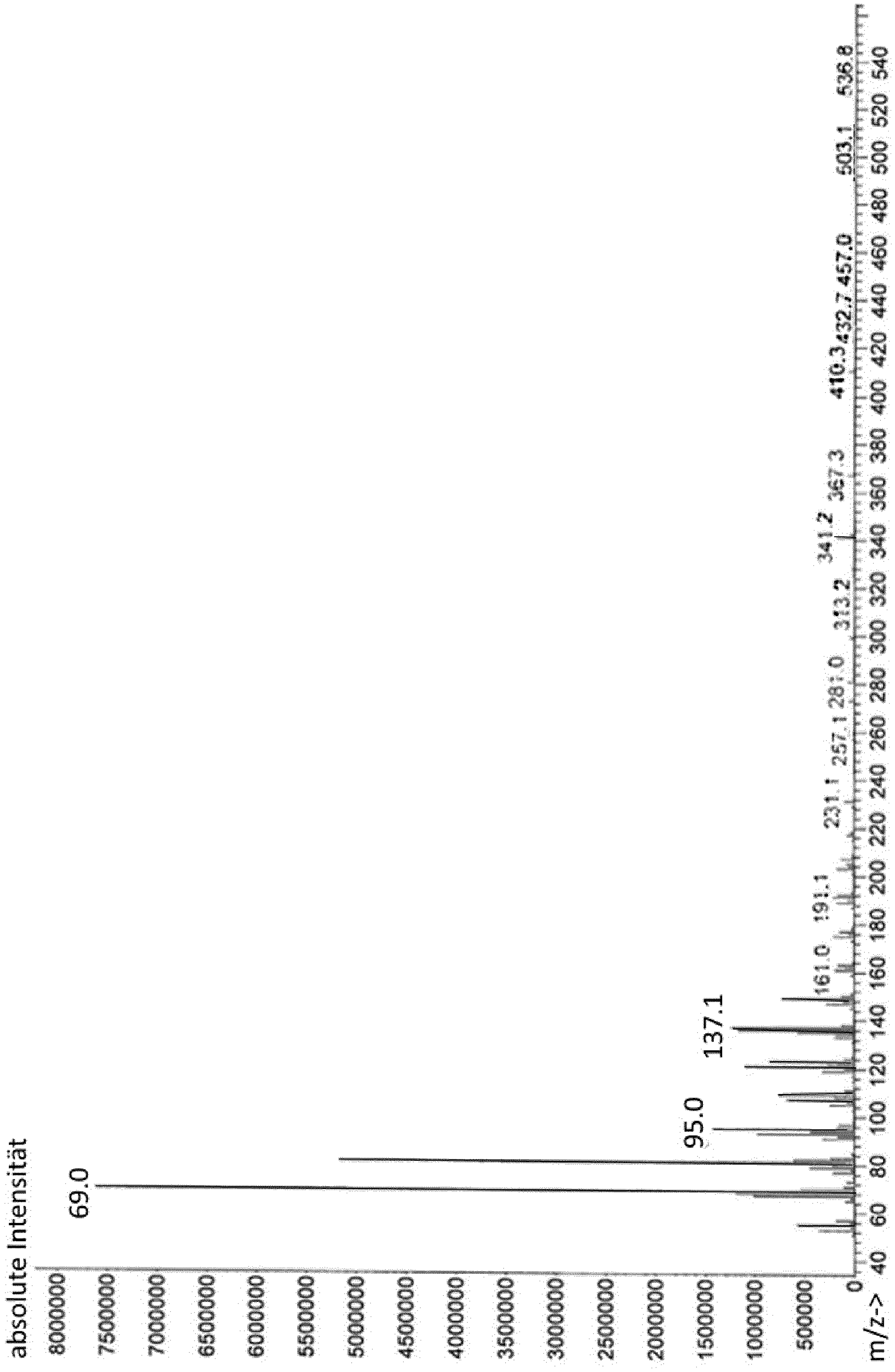
absolute Intensität
8000000
7500000
7000000
6500000
6000000
5500000
5000000
4500000
4000000
3500000
3000000
2500000
2000000
1500000
1000000
500000
0
m/z->
40 60 80 100 120 140 160 180 200 220 240 260 280 300 320 340 360 380 400 420 440 460 480 500 520 540
69.0
95.0
137.1
161.0
191.1
231.1
257.1
281.0
313.2
341.2
367.3
410.3
432.7
457.0
503.1
536.8

Fig. 7

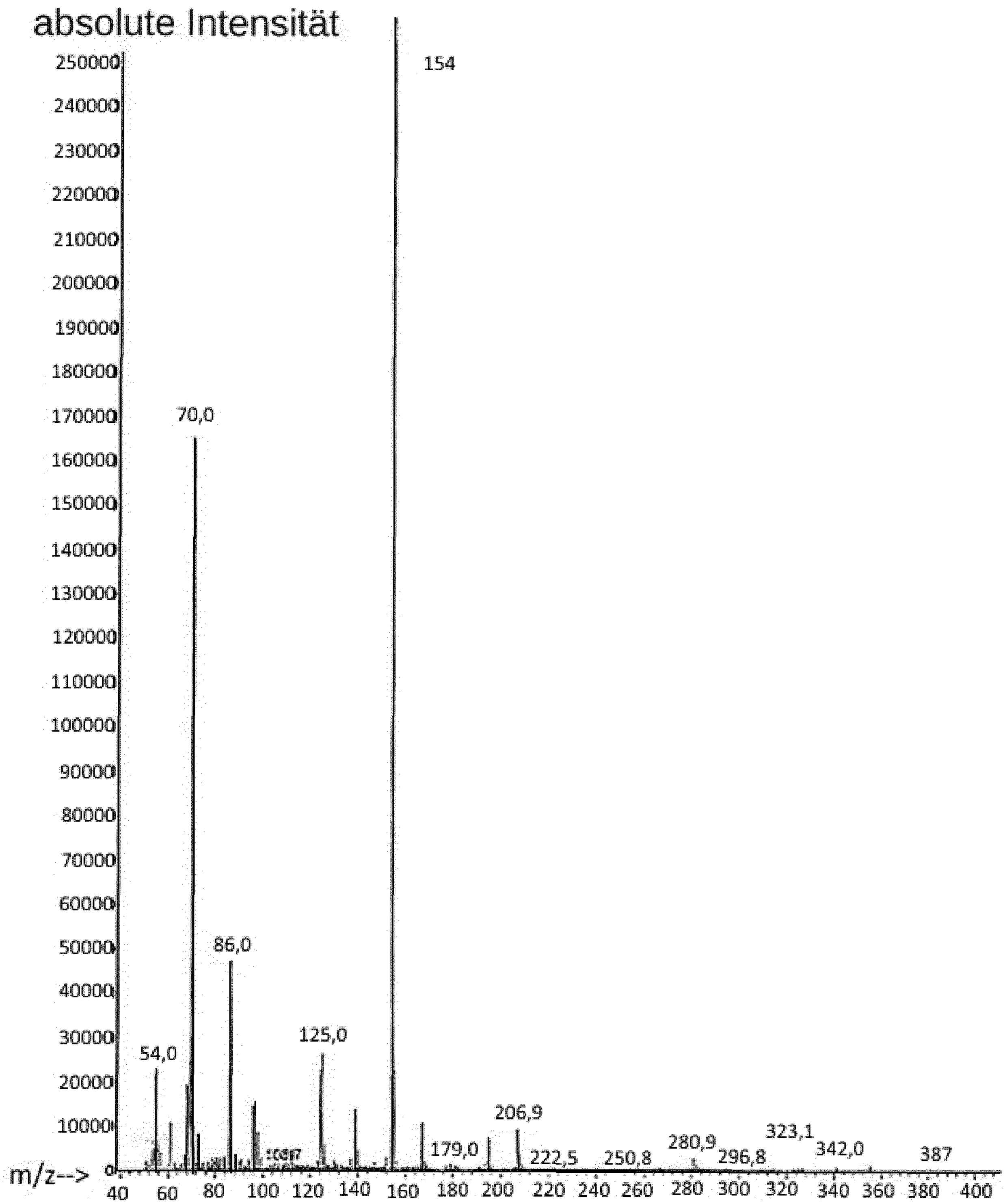
absolute Intensität
250000
240000
230000
220000
210000
200000
190000
180000
170000
160000
150000
140000
130000
120000
110000
100000
90000
80000
70000
60000
50000
40000
30000
20000
10000
0
m/z-->
40 60 80 100 120 140 160 180 200 220 240 260 280 300 320 340 360 380 400
154
70,0
86,0
125,0
54,0
206,9
179,0
222,5
250,8
280,9
296,8
323,1
342,0
387

Fig. 8

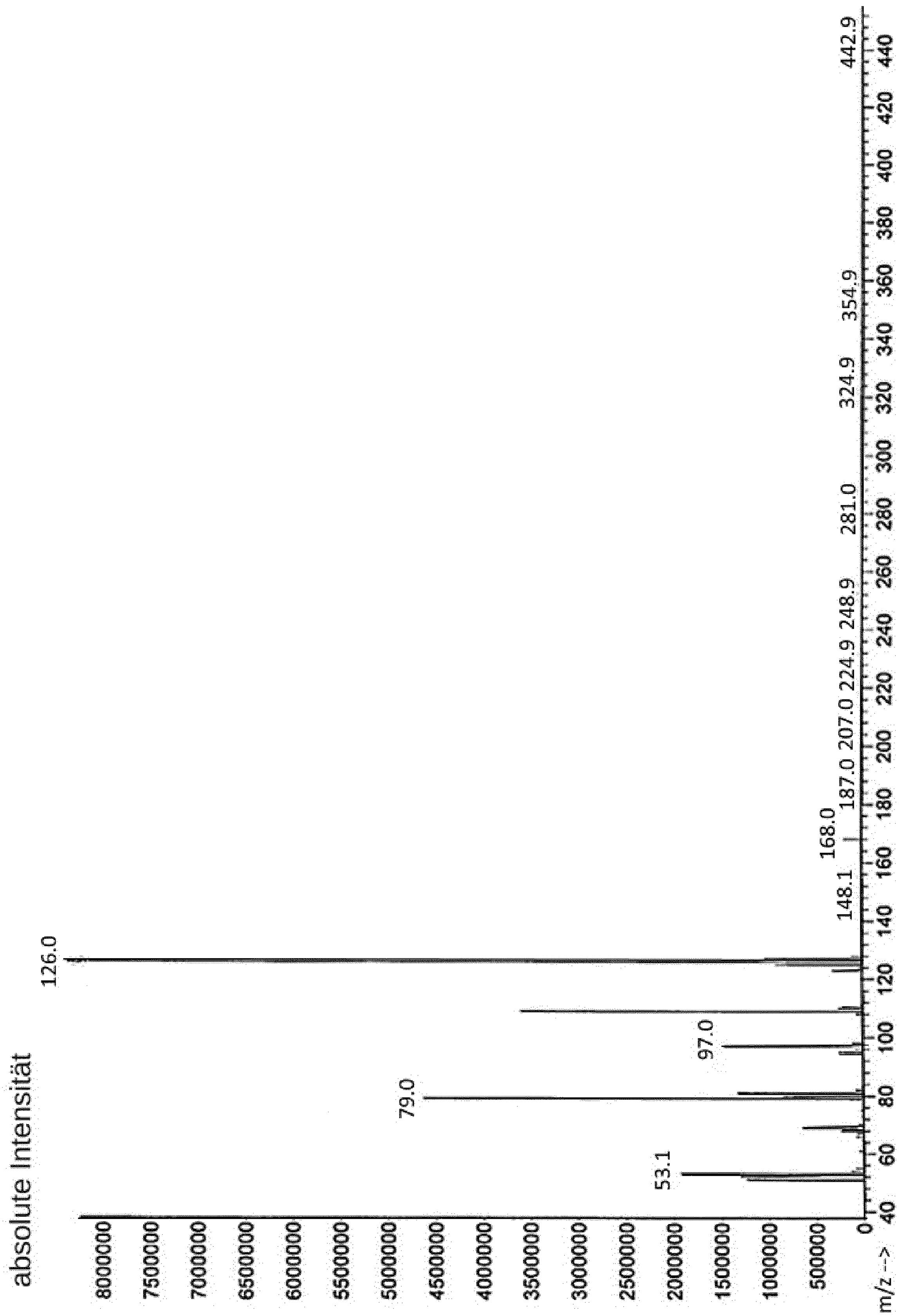
absolute Intensität
8000000
7500000
7000000
6500000
6000000
5500000
5000000
4500000
4000000
3500000
3000000
2500000
2000000
1500000
1000000
500000
0
m/z -->
40
60
80
100
120
140
160
180
200
220
240
260
280
300
320
340
360
380
400
420
440
53.1
79.0
97.0
126.0
148.1
168.0
187.0
207.0
224.9
248.9
281.0
324.9
354.9
442.9

Fig. 9

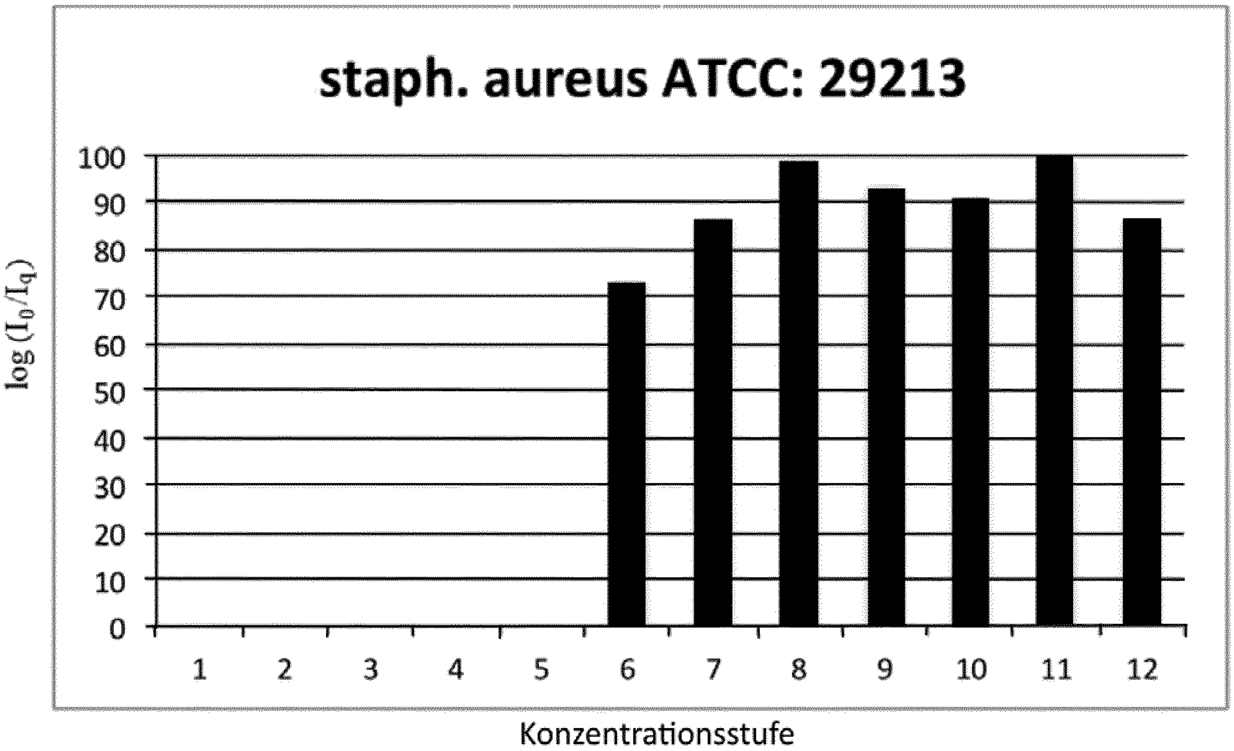


Fig.10a

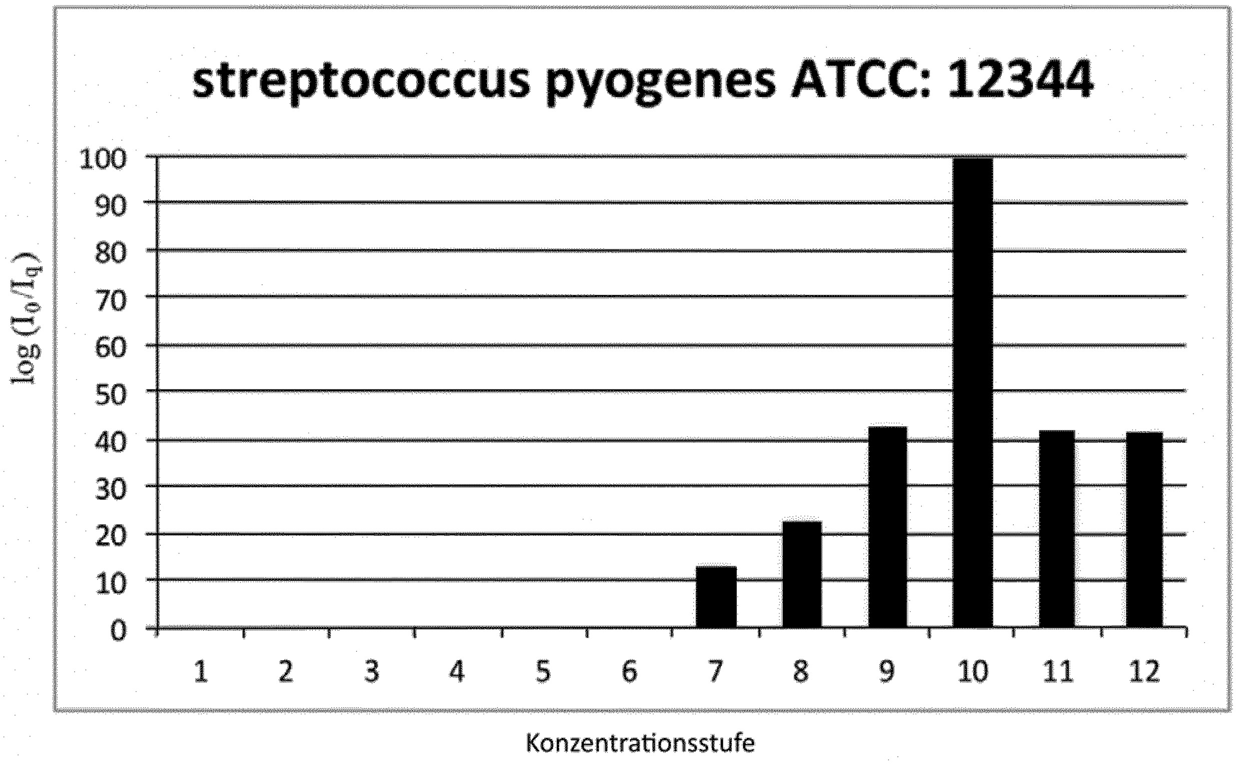


Fig. 10b

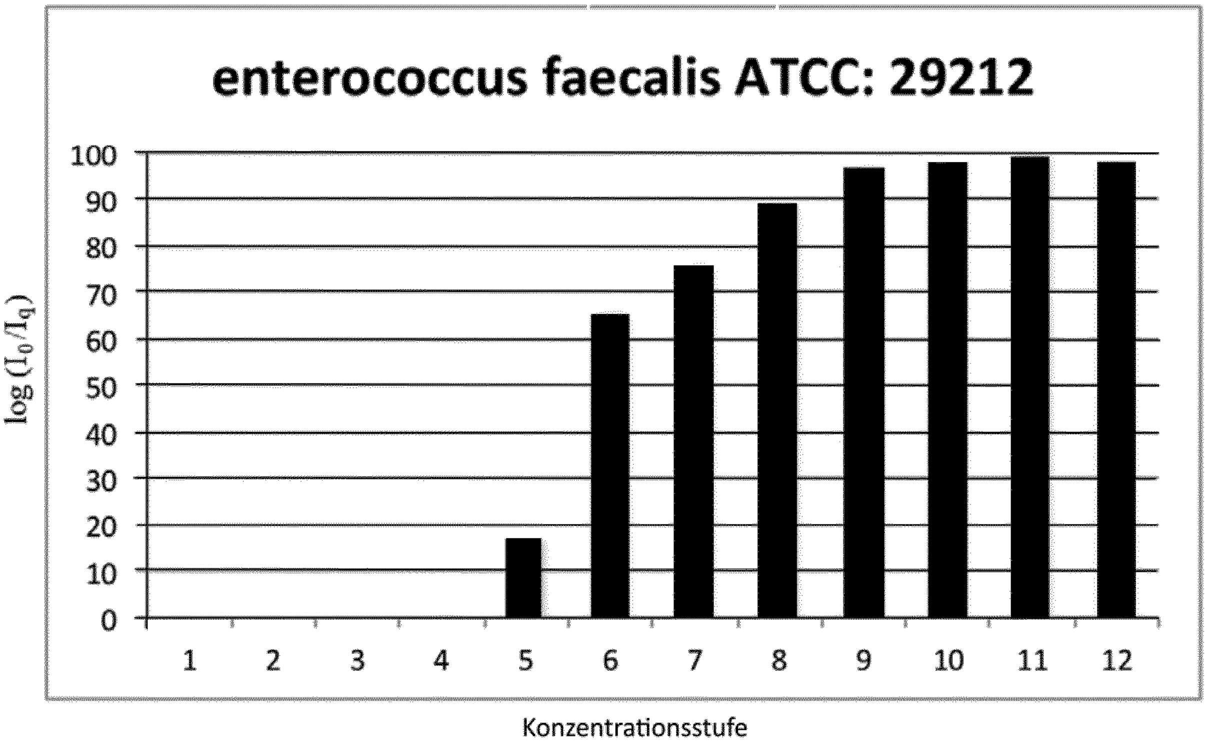


Fig. 10c

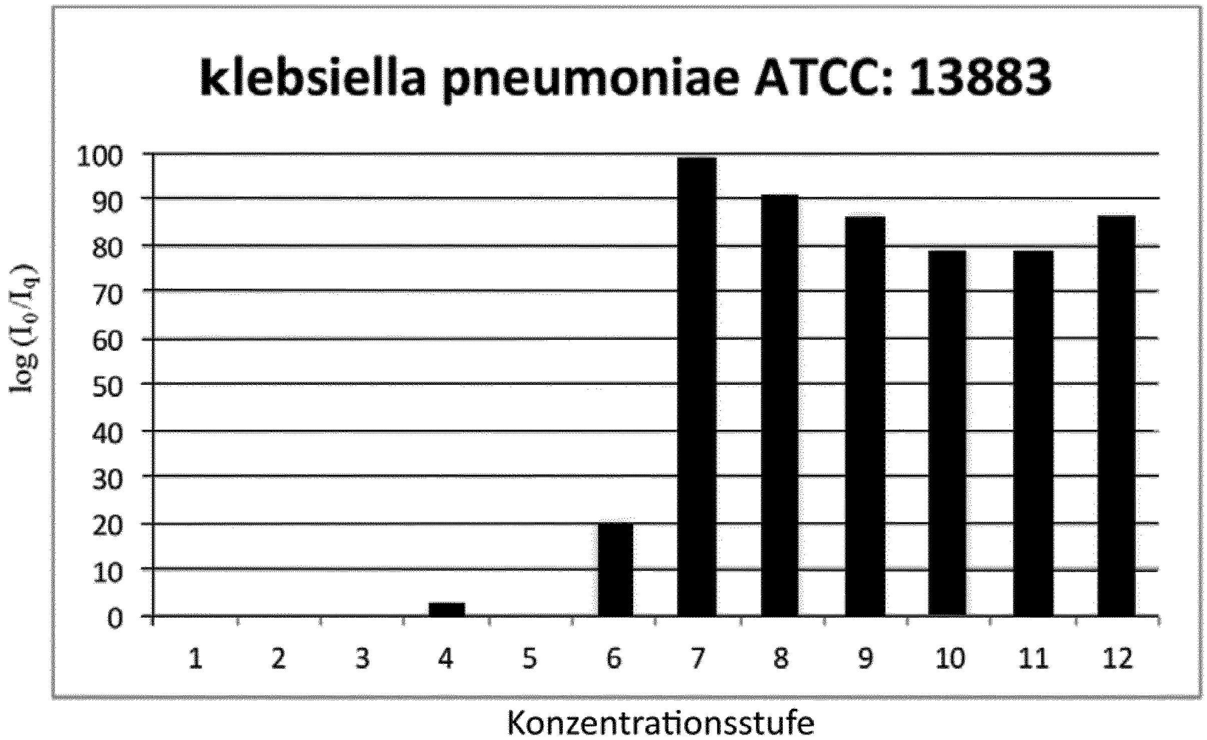


Fig. 10d

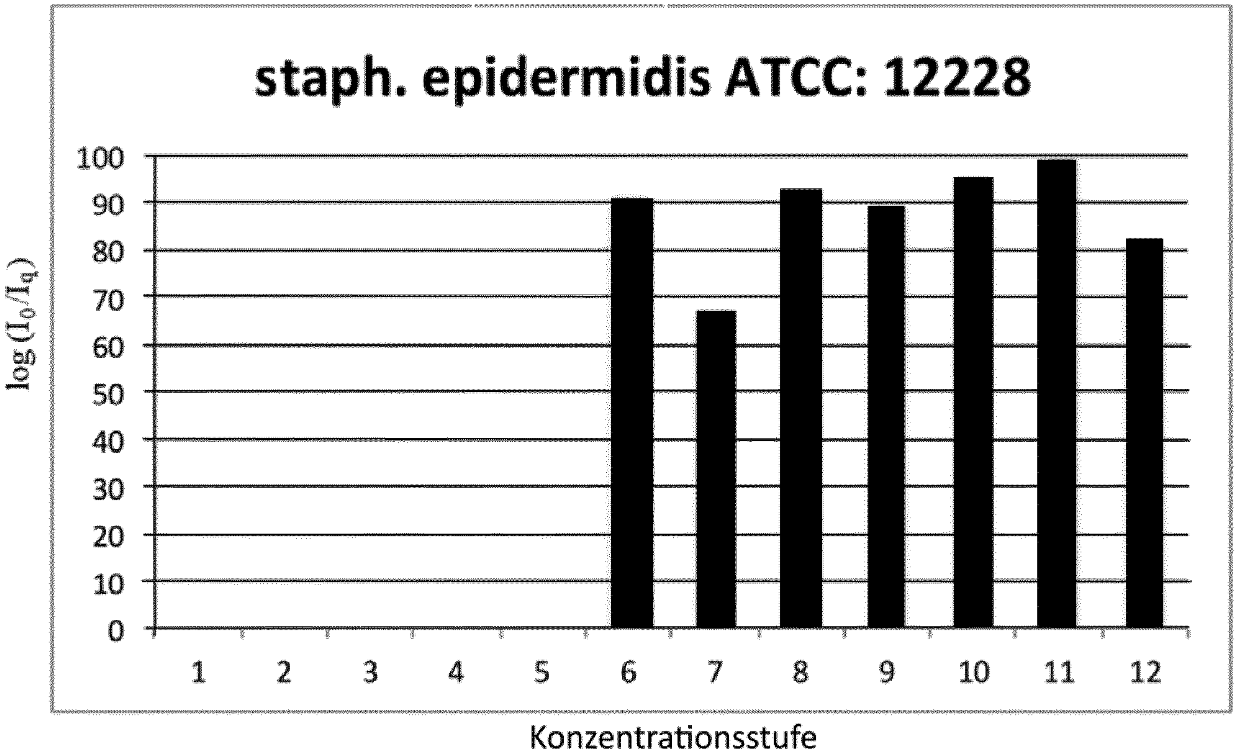


Fig. 10e

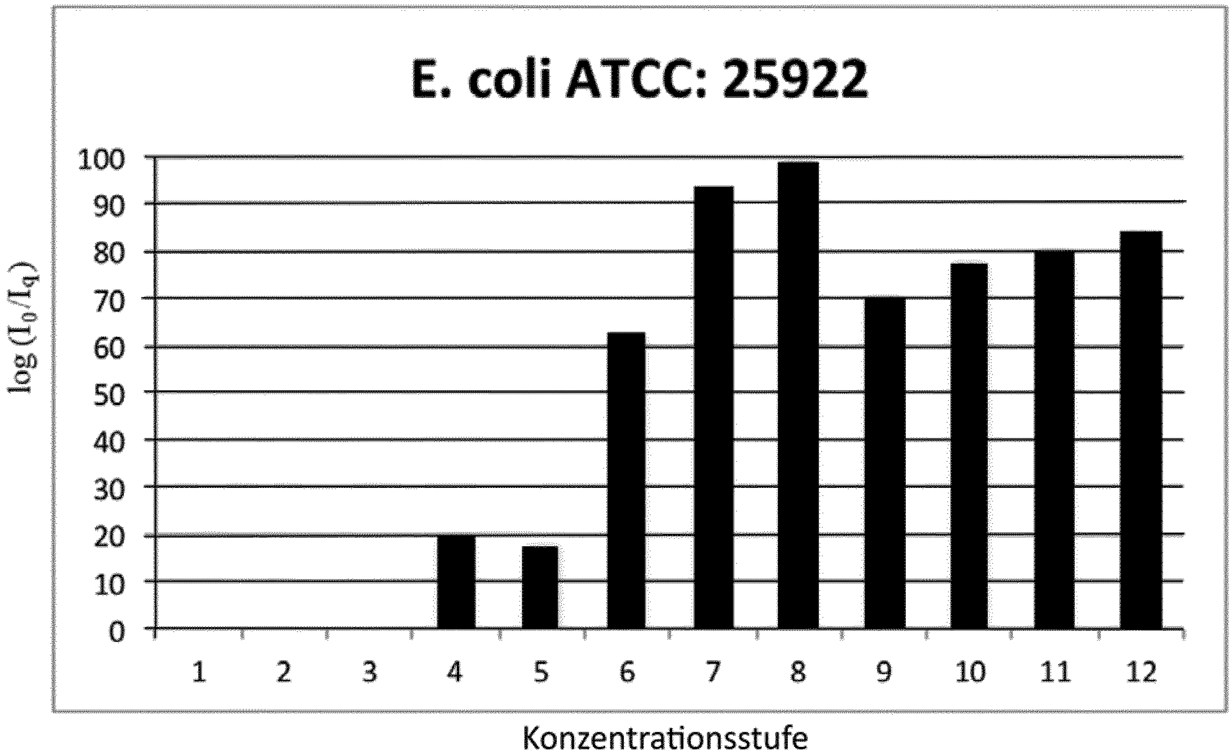


Fig. 10f

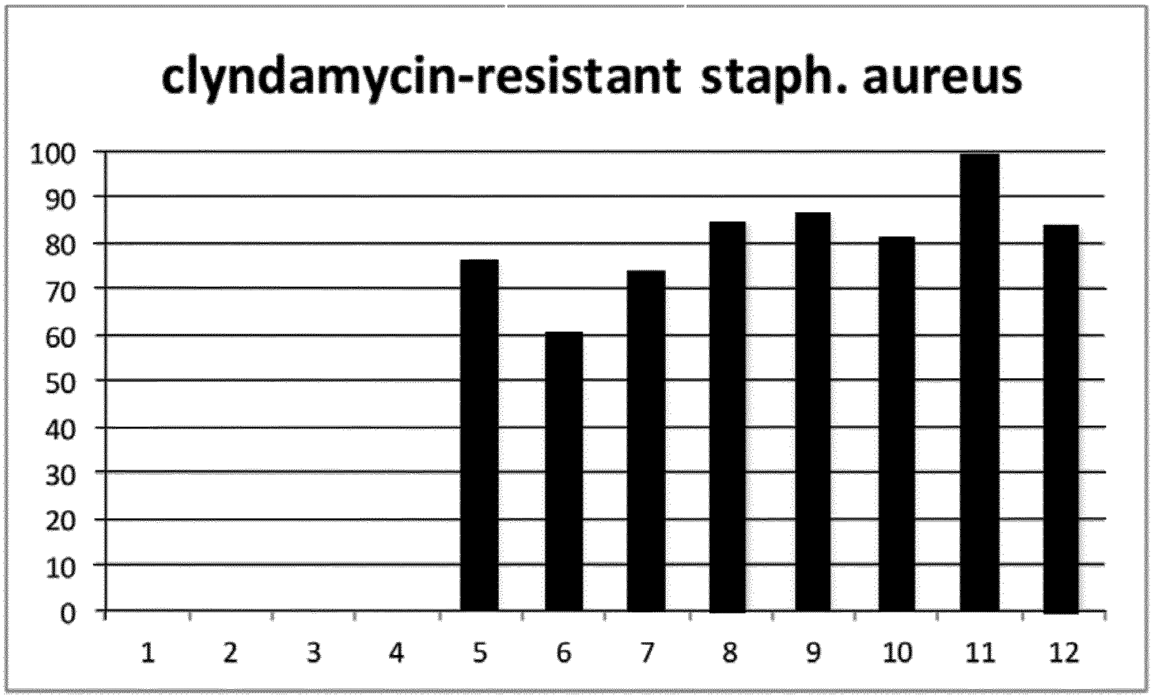


Konzentrationsstufe

Fig. 11a

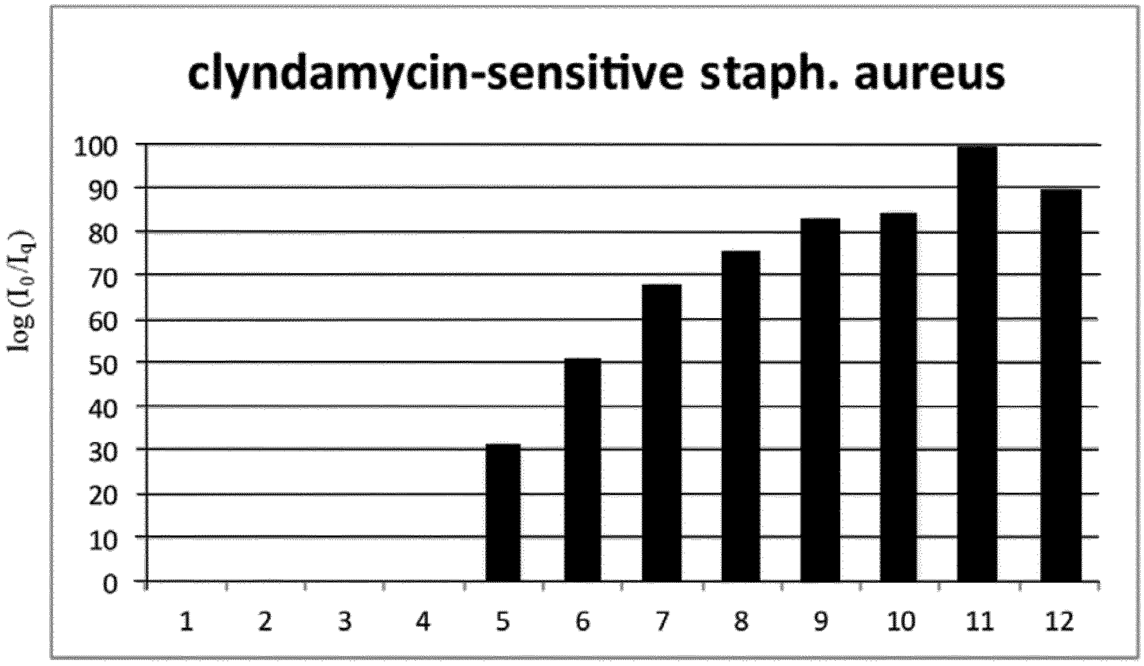


Konzentrationsstufe

Fig. 11b

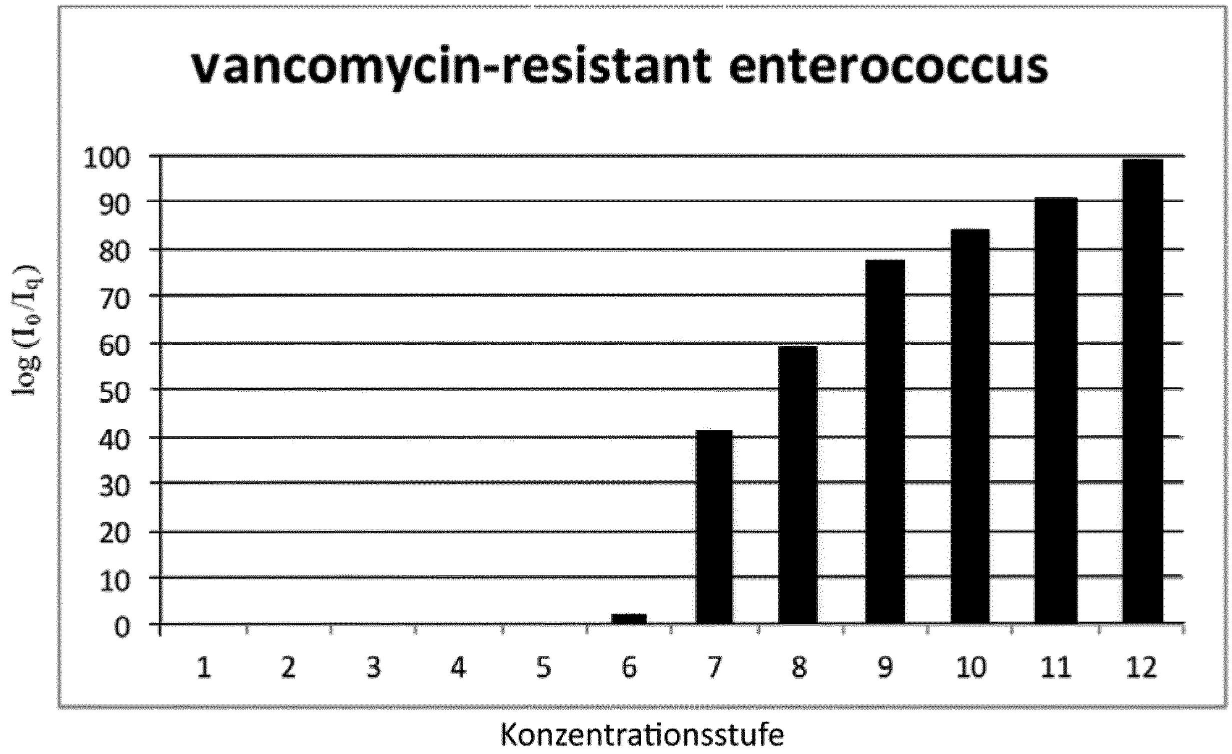


Fig. 11c

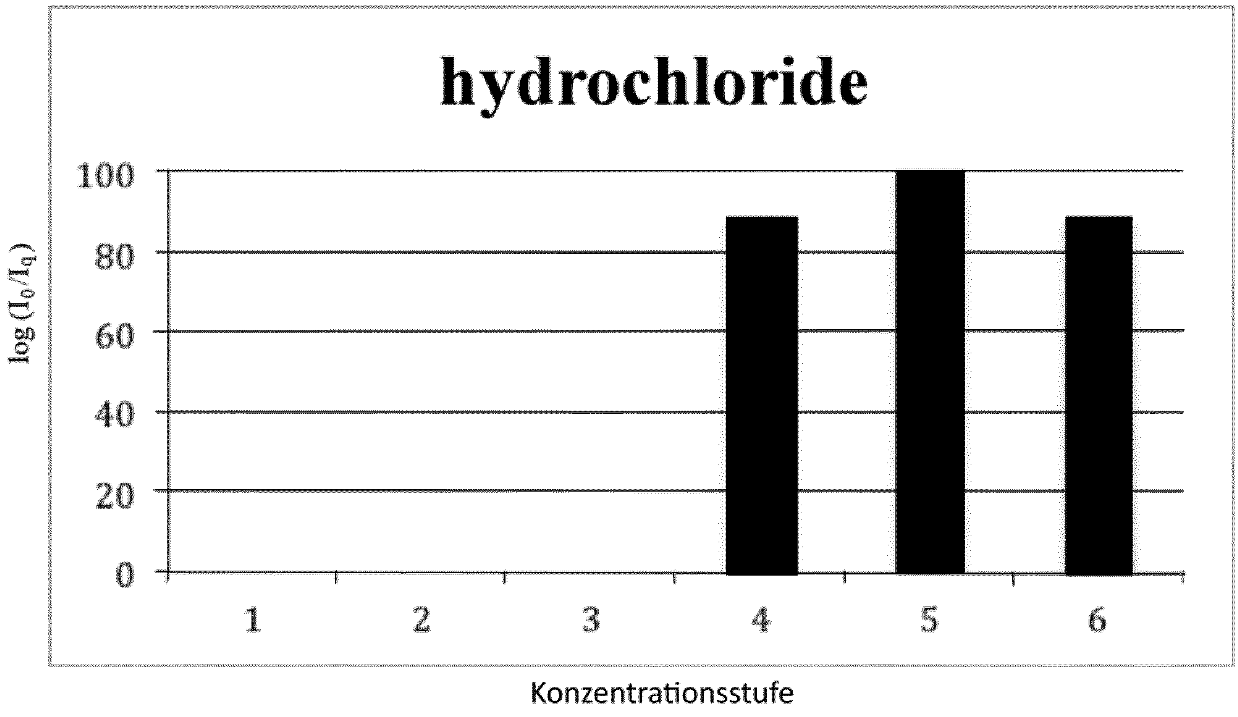


Fig. 12

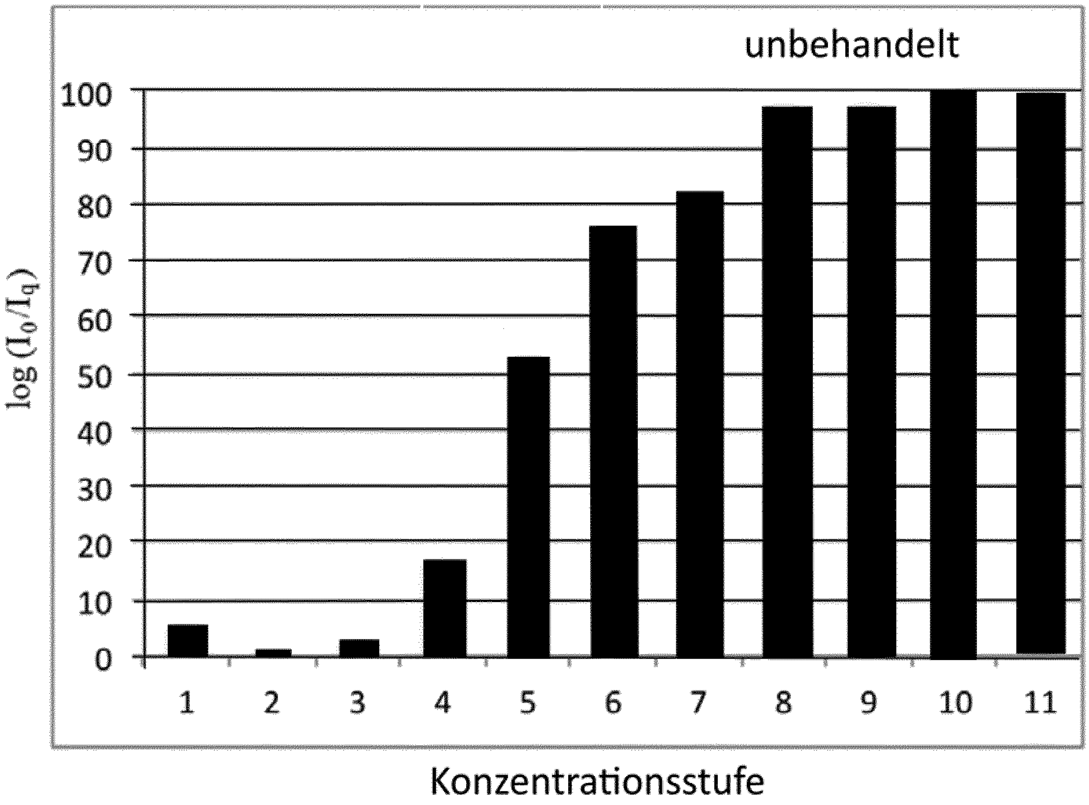


Fig. 13a

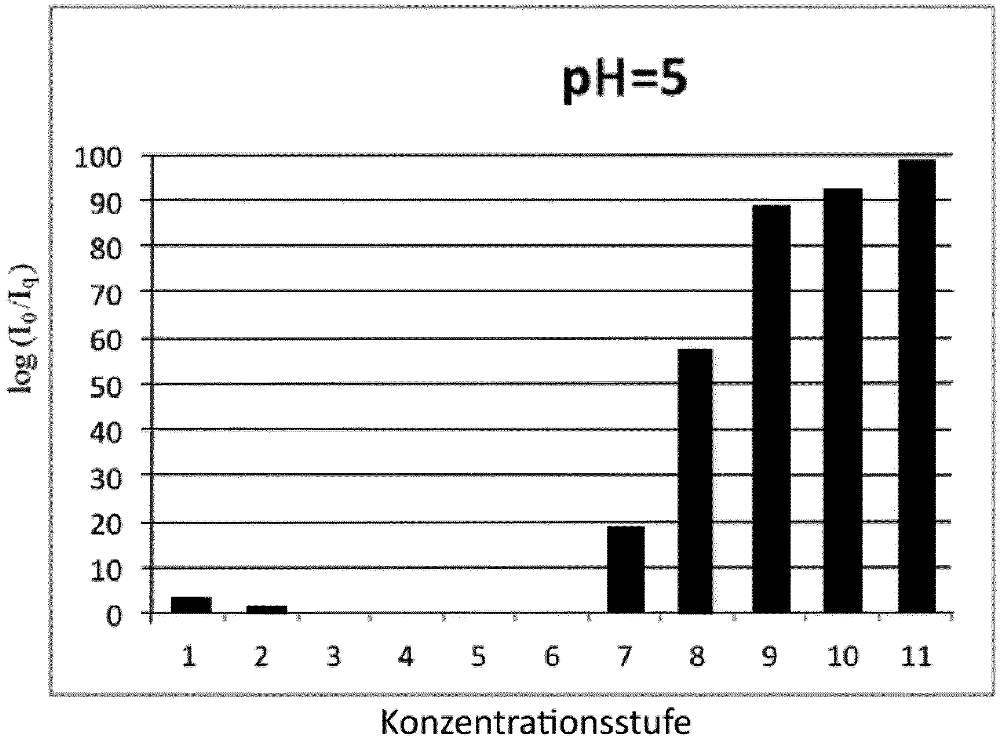


Fig. 13b

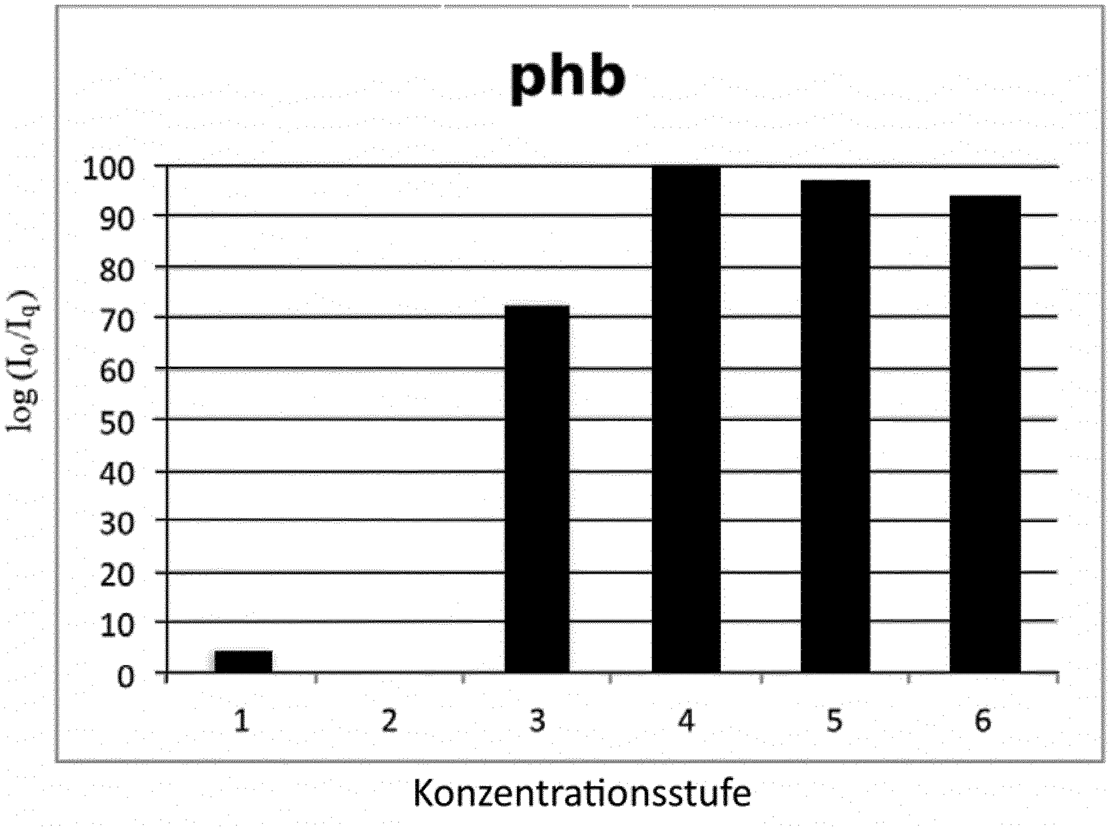


Fig. 14

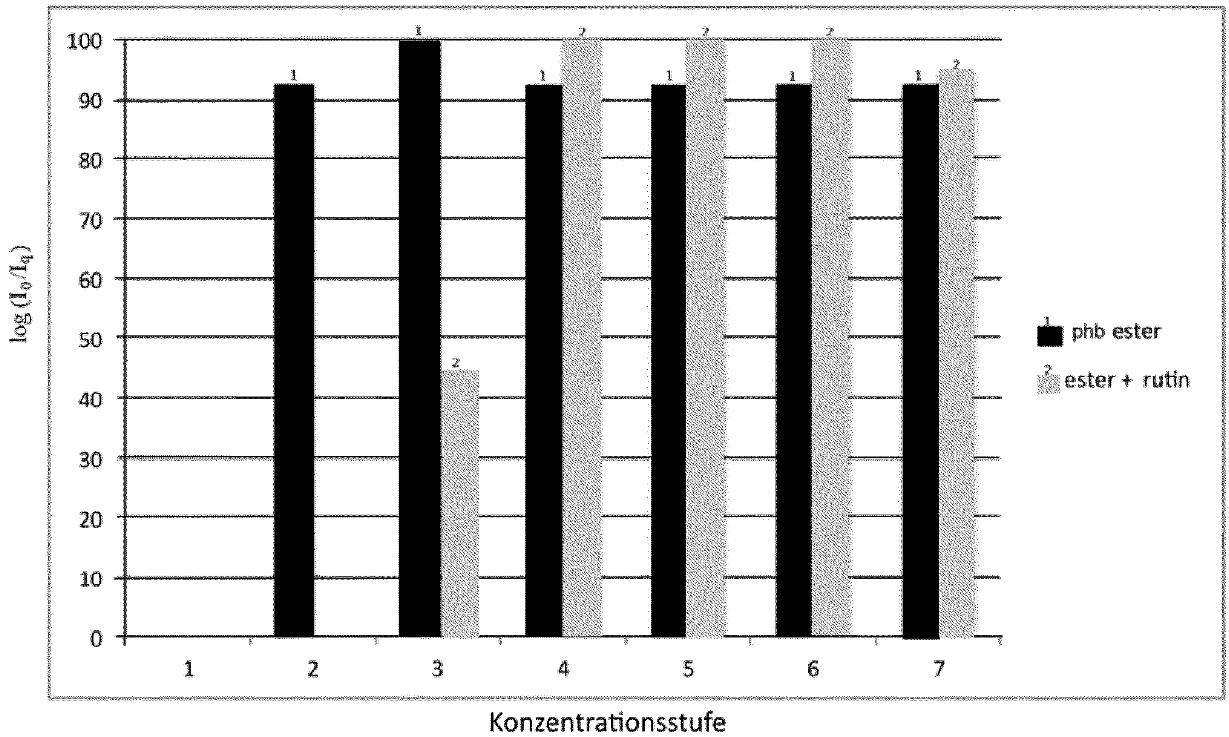


Fig. 15a

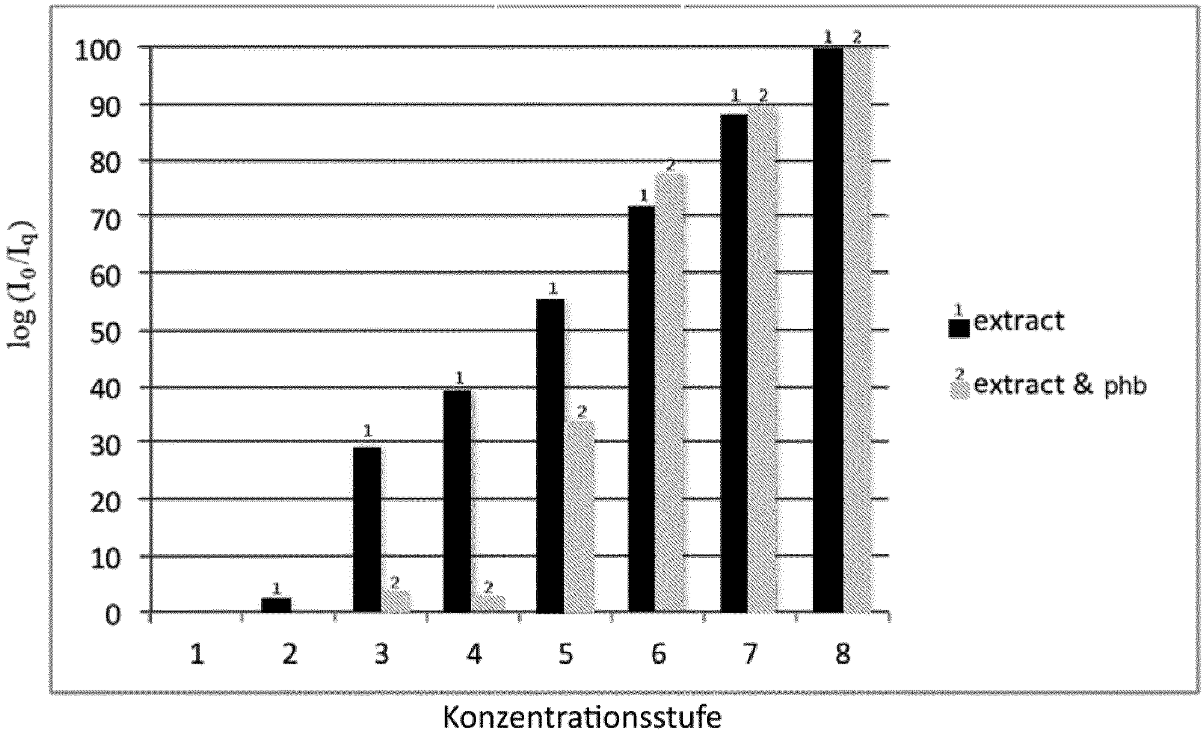


Fig. 15b

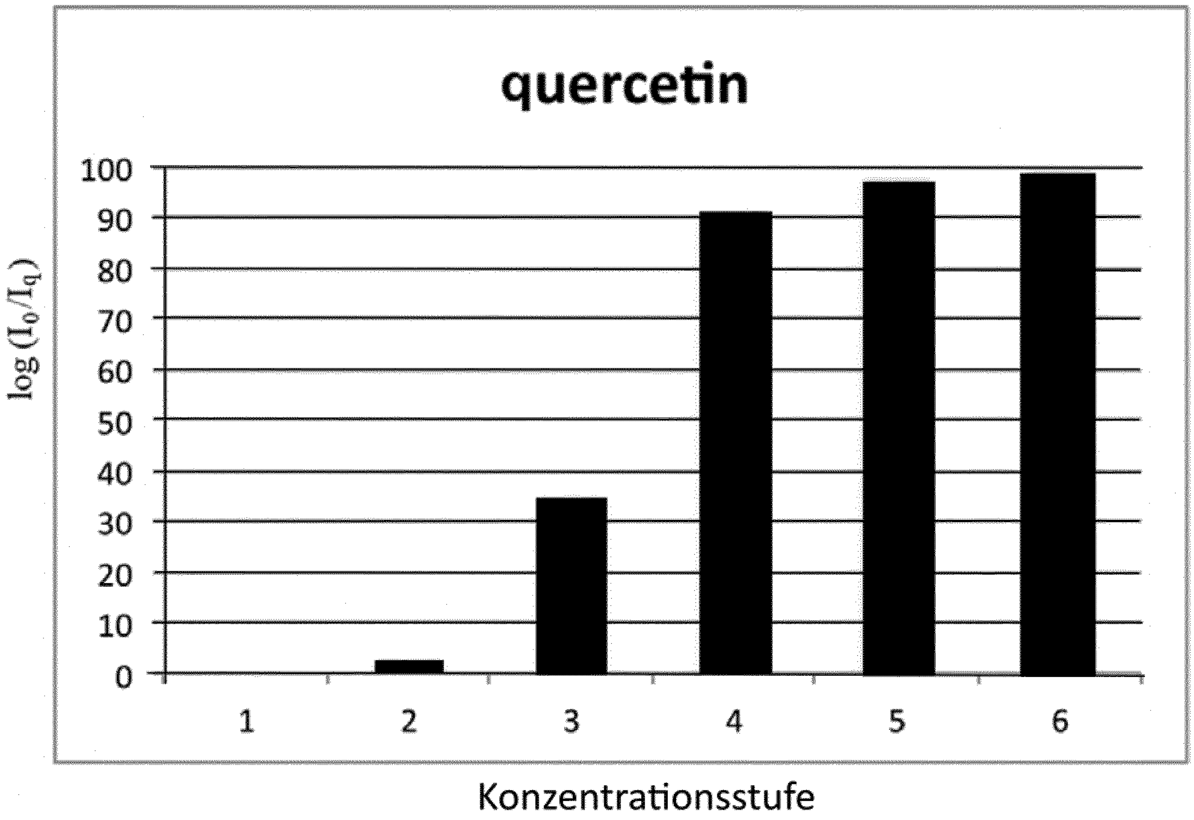


Fig. 16a

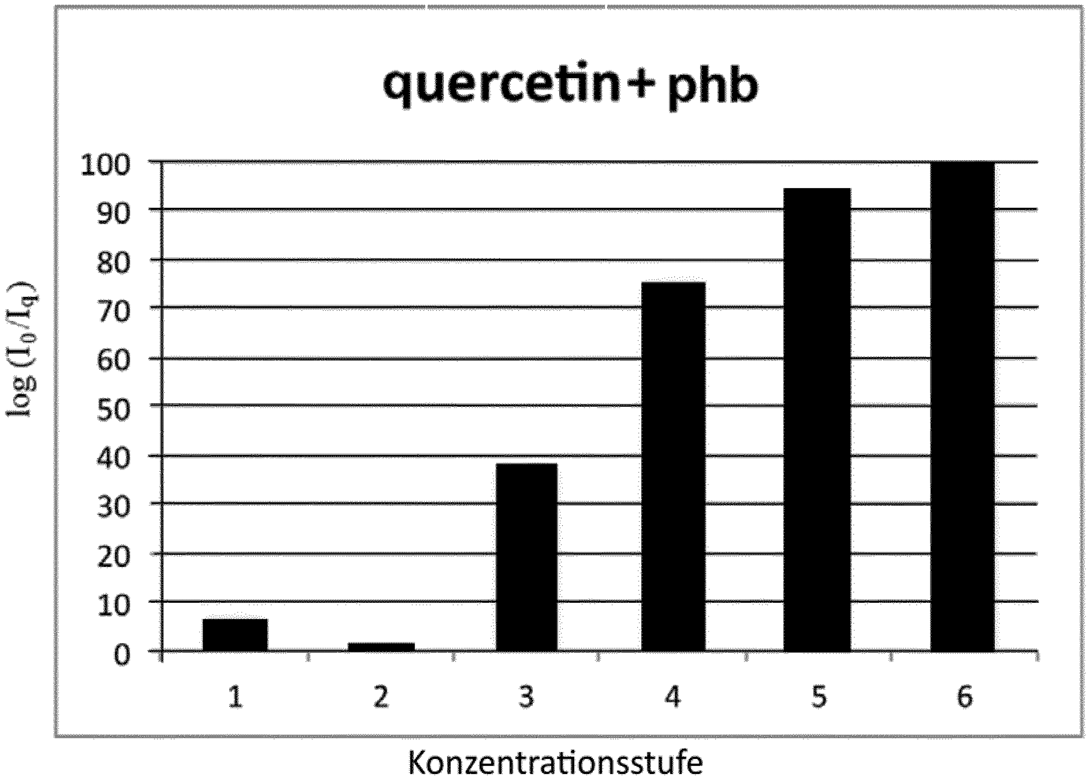


Fig. 16b

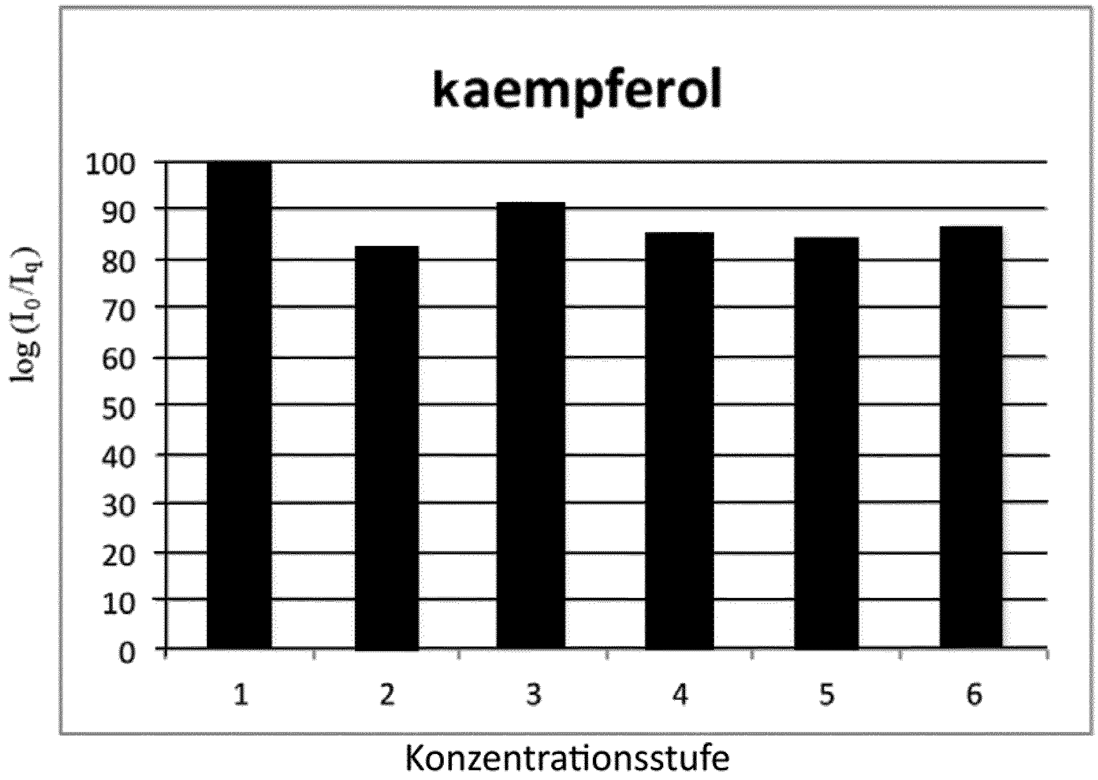


Fig. 17a

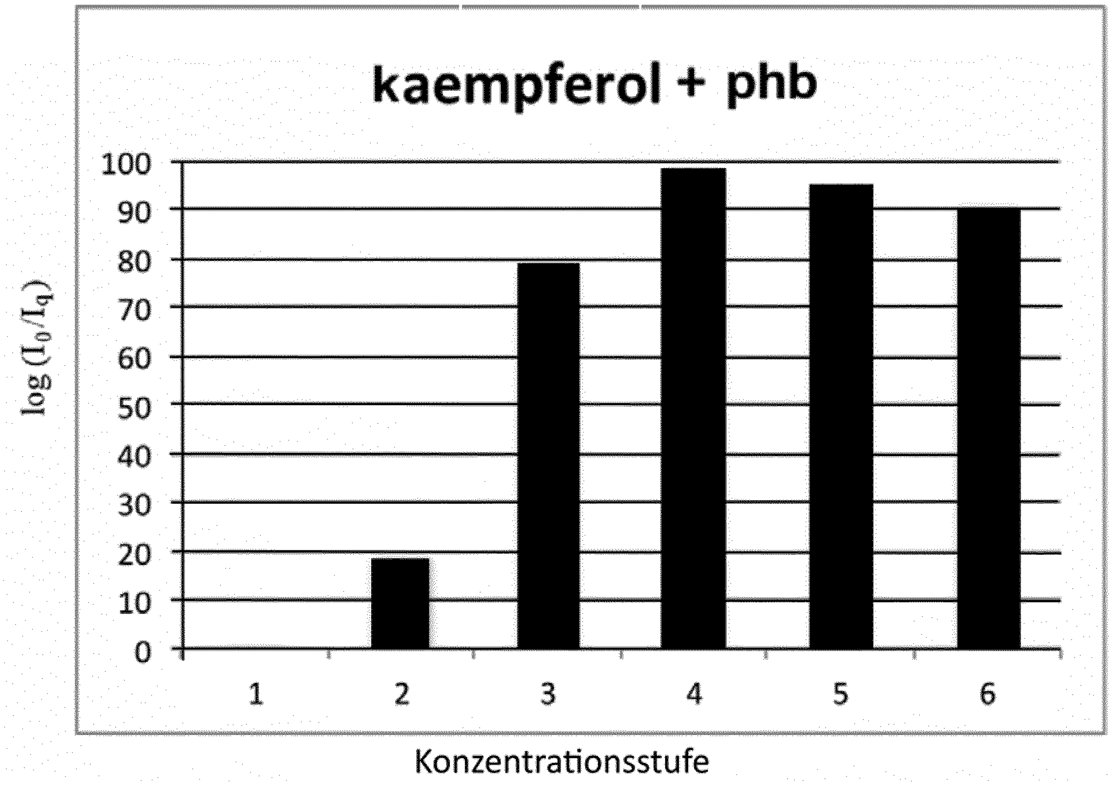


Fig. 17b

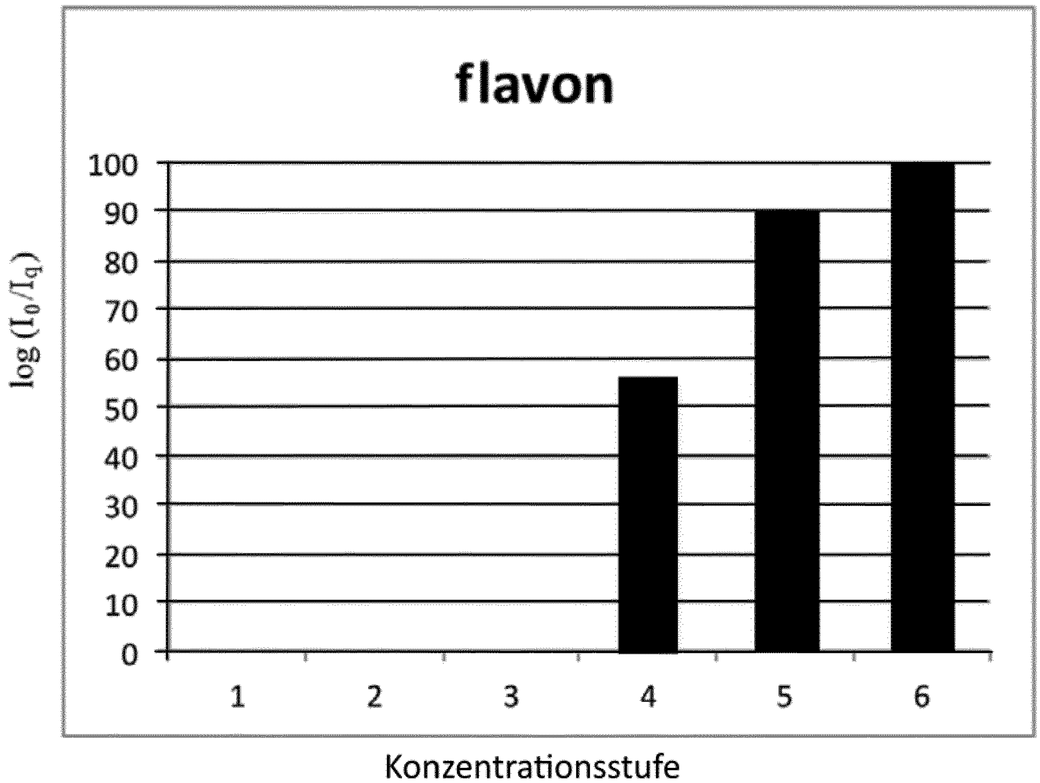


Fig. 18a

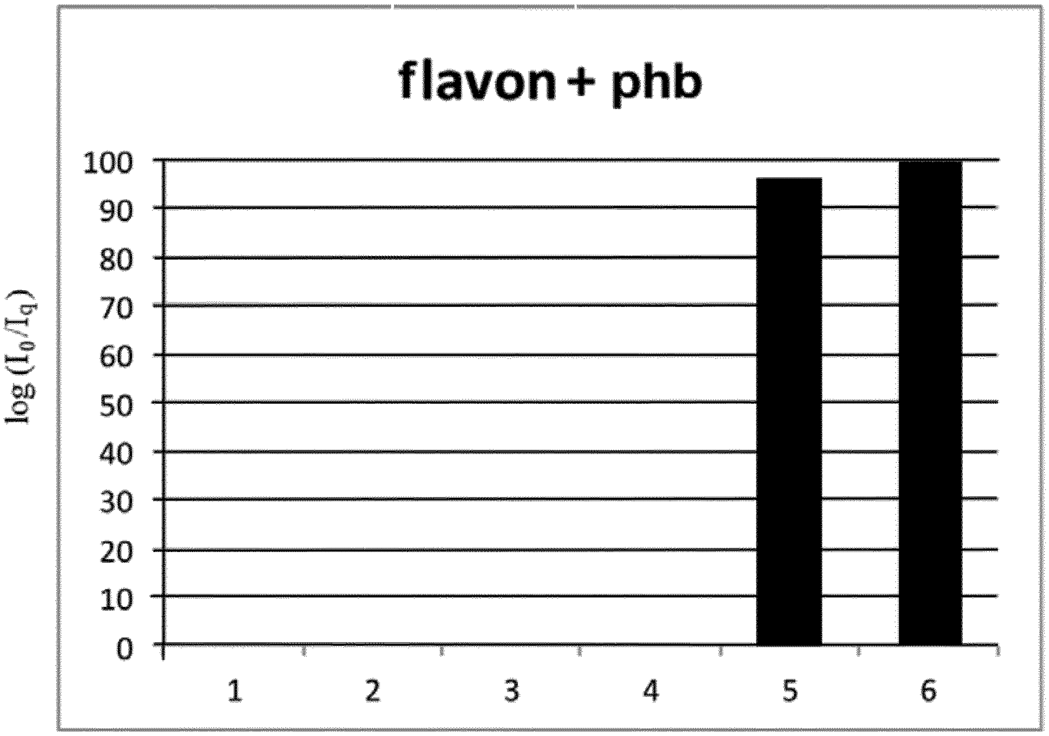


Konzentrationsstufe

Fig. 18b

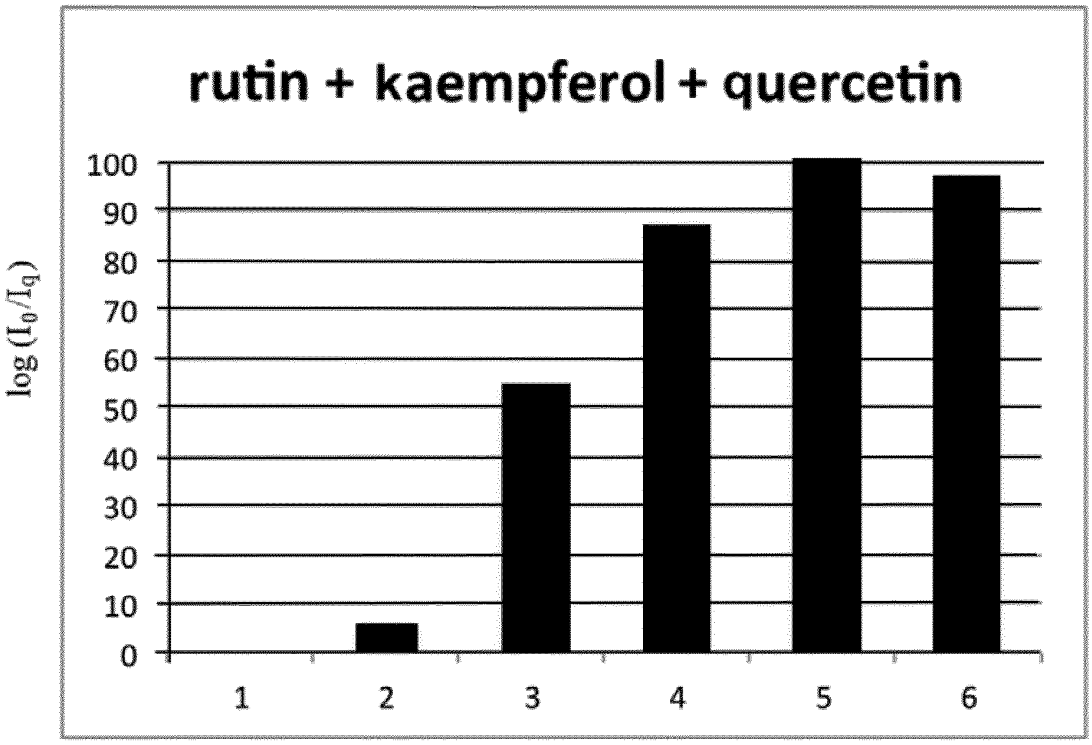


Konzentrationsstufe

Fig. 19a

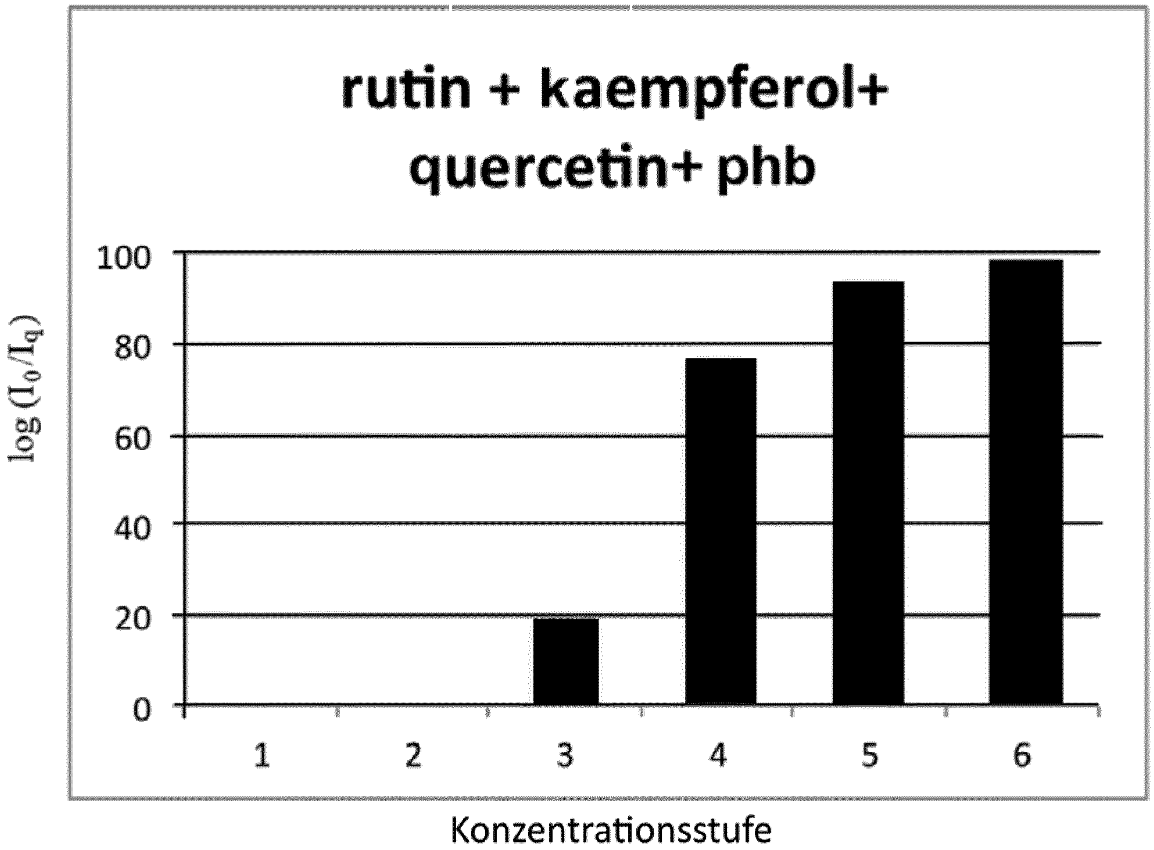


Fig. 19b

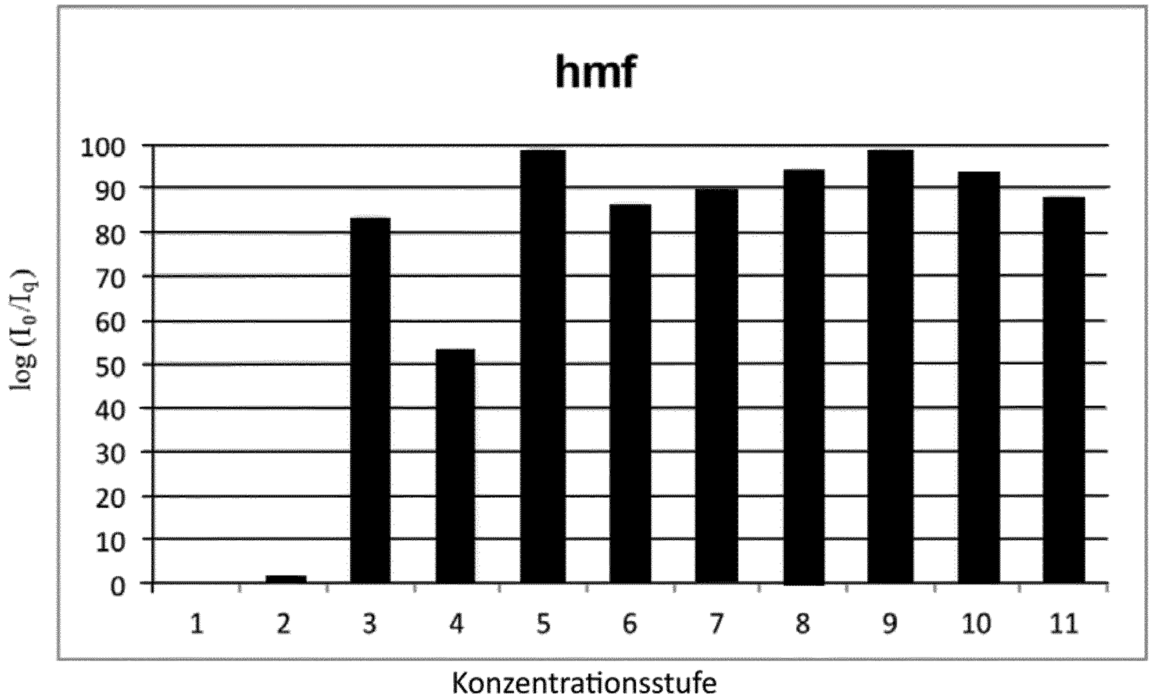


Fig. 20a

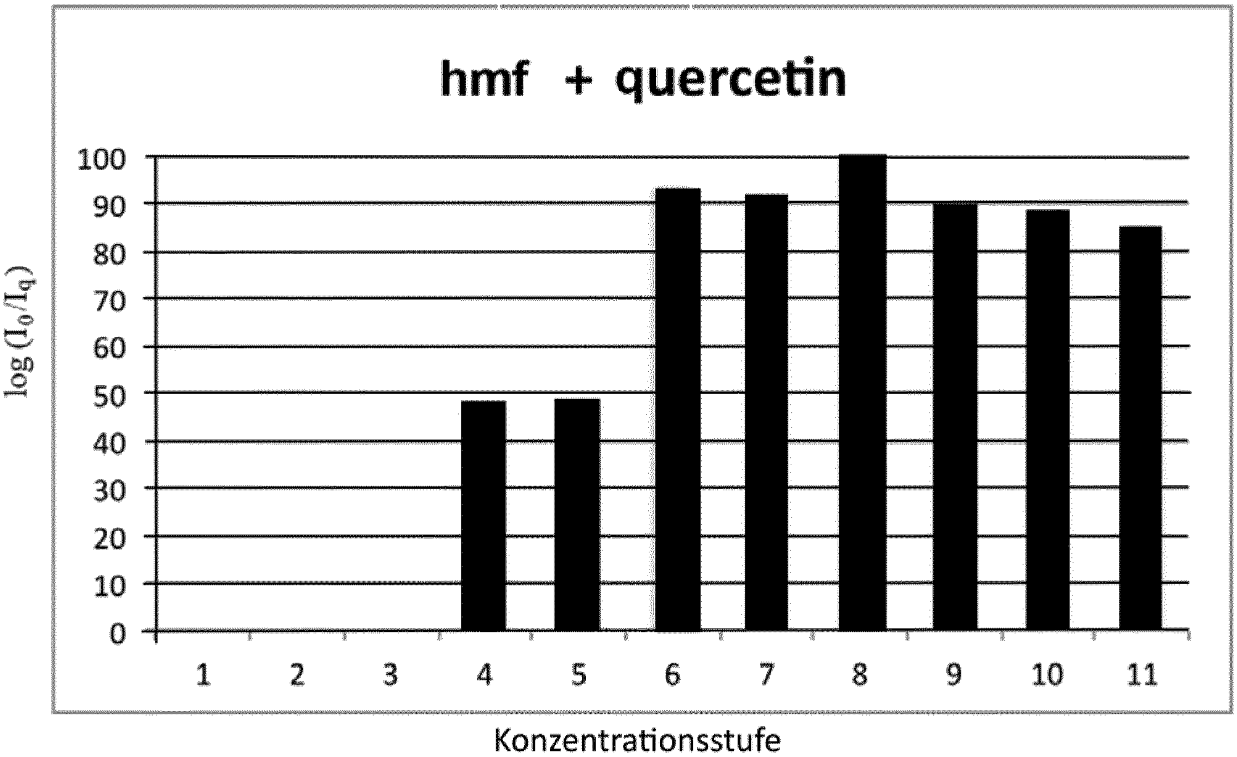
hmf + quercetin
log (I0/Iq)
100
90
80
70
60
50
40
30
20
10
0
1
2
3
4
5
6
7
8
9
10
11
Konzentrationsstufe

Fig. 20b

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- KR 1020020008349, von KIM, Jong
- RU 981000314
- KR 20130109084 A
- KR 20130078052 A
- CN 104288286 A
- CN 103877147 A
- JP S5944313 A
- DE 3144137 A1
- EP 2295031 A2
- GB 1054124 A


### In der Beschreibung aufgeführte Nicht-Patentliteratur


- **VON JOUNG DAE-KI ; SHIN DONG-WON.** *The Mechanism of Antimicrobial Activity of Sophoraflavanone B against Methicillin-Resistant Staphylococcus aureus*
- *Foodborne Pathogens and Disease,* Marz 2014, vol. 11 (3), 234
- **VON YAKUGAKU ZASSHI.** *Interaction in the antibacterial activity of flavonoids from Sophora japonica L. to Propioni bacterium,* April 1984, vol. 104 (4), 340
- Flavonol tetragycosides from fruits of Styphnolobium japonicum (Leguminosae) and the authentication of Fructus Sophorae and Flos Sophorae. **KITE G. C. et al.** Phytochemistry. Pergamon Press, 01. April 2009, vol. 70, 785-794
- **H.-F. GAO et al.** Hydroxymethyl furfural in Chinese herbal medicines: Its formation, presence, metabolism, bioactivities and implications. *African Journal of Traditional, complementary and alternative Medicines,* 13. April 2015, vol. 12 (2), 43
- **KIMURA M. et al.** Interaction in the Antibacterial Activity of Flavonoids from Sophora-Japonica to Propionibacterium. Yakugaku Zasshi, 1984, vol. 104, 340-346
- **YANG WOO-YOUNG et al.** Streptococcus mutanssortase A inhibitory metabolites from the flowers of Sophora japonica. *Bioorganic & Medicinal Chemistry Letters,* vol. 25 (7), 1394-1397
- Local and traditional uses, phytochemistry, and pharmacology of Sophora japonica L.: A review. **HE XIRUI et al.** Journal or Ethnopharmacology. Elsevier Ireland Ltd, 13. April 2016, vol. 187, 160-182